# EUROPEAN PATENT APPLICATION

(11) **EP 3 009 450 A1**
(43) Date of publication of application: **20.04.2016**
(21) Application number: 15197750.1
(22) Date of filing: 19.07.2012
(51) Int. Cl.: C07K 16/24

(54) **LIQUID FORMULATION COMPRISING ADALIMUMAB AND AN ACETATE BUFFER**

(30) Priority: 19.07.2011 GB 201112429
(62) Divisional of application: 12738101.0
(71) Applicant: Glaxo Group Limited, Brentford, Middlesex TW8 9GS (GB)
(72) Inventor: ELLIS, Jonathan Henry, Stevenage Hertfordshire SG1 2NY (GB); SHAH, Tejash, Stevenage Hertfordshire SG1 2NY (GB); TOMLINSON, Ian M., Stevenage Hertfordshire SG1 2NY (GB); MOLLOY, Michael J., Stevenage Hertfordshire SG1 2NY (GB); YASIN, Ahmed, Stevenage Hertfordshire SG1 2NY (GB)
(74) Representative: Lee, Alison Lesley

(57) **Abstract**

The present invention provides antigen binding proteins which bind specifically to TNF-alpha. For example novel variants of anti-TNF antibodies such as adalimumab which show increased binding to the FcRn receptor or increased half life compared to adalimumab. Also provided are compositions comprising the antigen binding proteins and uses of such compositions in treatment of disorders and disease.

SEQUENCE LISTING

<110> ELLIS, johnathan
      TOMLINSON, Ian
      SHAH, Tejash
      MOLLOY, Micheal
      YASIN, Ahmed

<120> Antigen-binding proteins with increased
  FcRn binding

<130> PB64705

<150> GB1112429.4
<151> 2011-07-19

<160> 180

<170> FastSEQ for Windows Version 4.0

<210> 1
<211> 642
<212> DNA
<213> Artificial Sequence

<220>
<223> Humanised sequence

<400> 1
gatatccaga tgacccagag ccccagcagc ctgagcgcct ctgtgggcga tagagtgacc 60
atcacctgcc gggccagcca gggcatcaga aactacctgg cctggtatca gcagaagcct 120
ggcaaggccc ctaagctgct gatctacgcc gccagcaccc tgcagagcgg cgtgcccagc 180
agattcagcg gcagcggctc cggcaccgac ttcaccctga ccatcagcag cctgcagccc 240
gaggacgtgg ccacctacta ctgccagcgg tacaacagag ccccttacac cttcggccag 300
ggcaccaagg tggagatcaa gcgtacggtg gccgccccca gcgtgttcat cttccccccc 360
agcgatgagc agctcaagag cggcaccgcc agcgtggtgt gtctgctgaa caacttctac 420
ccccgggagg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cggggcgagt gc                    642

<210> 2
<211> 214
<212> PRT
<213> Artificial Sequence

<220>
<223> Humanised sequence

<400> 2
Asp Ile Gln Met Thr Gln Ser Pro Ser Ser Leu Ser Ala Ser Val Gly
 1               5                  10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
                100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
                180                 185                 190
Ala Cys Glu Val Thr His Gln Gly Leu Ser Ser Pro Val Thr Lys Ser
        195                 200                 205
Phe Asn Arg Gly Glu Cys
    210

<210> 3
<211> 109
<212> PRT
<213> Artificial Sequence

<220>
<223> Humanised sequence

<400> 3
Asp Ile Gln Met Thr Gln Ser Pro Ser Ser Leu Ser Ala Ser Val Gly
 1               5                  10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Arg Ala Pro Tyr
                85                  90                  95
Thr Phe Gly Gln Gly Thr Lys Val Glu Ile Lys Arg Thr
                100                 105

<210> 4
<211> 1353
<212> DNA
<213> Artificial Sequence

<220>
<223> Humanised sequence

<400> 4
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660

cccaagagct gtgacaagac ccacacctgc cccccctgcc ctgcccccga gctgctggga 720
ggccccagcg tgttcctgtt cccccccaag cctaaggaca ccctgtacat caccagagag 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 5
<211> 451
<212> PRT
<213> Artificial Sequence

<220>
<223> Humanised sequence

<400> 5
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Tyr
                245                 250                 255
Ile Thr Arg Glu Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile

325                         330                         335
Glu Lys Thr Ile Ser Lys Ala Lys Gly Gln Pro Arg Glu Pro Gln Val
            340                         345                         350
Tyr Thr Leu Pro Pro Ser Arg Asp Glu Leu Thr Lys Asn Gln Val Ser
        355                         360                         365
Leu Thr Cys Leu Val Lys Gly Phe Tyr Pro Ser Asp Ile Ala Val Glu
    370                         375                         380
Trp Glu Ser Asn Gly Gln Pro Glu Asn Asn Tyr Lys Thr Thr Pro Pro
385                         390                         395                         400
Val Leu Asp Ser Asp Gly Ser Phe Phe Leu Tyr Ser Lys Leu Thr Val
                        405                         410                         415
Asp Lys Ser Arg Trp Gln Gln Gly Asn Val Phe Ser Cys Ser Val Met
                    420                         425                         430
His Glu Ala Leu His Asn His Tyr Thr Gln Lys Ser Leu Ser Leu Ser
            435                         440                         445
Pro Gly Lys
    450

<210> 6
<211> 121
<212> PRT
<213> Artificial Sequence

<220>
<223> Humanised sequence

<400> 6

<210> 7
<211> 330
<212> PRT
<213> Artificial Sequence

<220>
<223> Humanised sequence

<400> 7
Ala Ser Thr Lys Gly Pro Ser Val Phe Pro Leu Ala Pro Ser Ser Lys
 1               5                  10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Tyr Ile Thr Arg Glu Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330


<210> 8
<211> 1353
<212> DNA
<213> Artificial Sequence

<220>
<223> Humanised sequence

<400> 8
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080

gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgctgcacg aggccctgca cagccactac 1320
acccagaaga gcctgagcct gtcccctggc aag 1353

<210> 9
<211> 451
<212> PRT
<213> Artificial Sequence

<220>
<223> Humanised sequence

<400> 9
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Leu
            420                 425                 430
His Glu Ala Leu His Ser His Tyr Thr Gln Lys Ser Leu Ser Leu Ser
        435                 440                 445
Pro Gly Lys
    450

<210> 10
<211> 330
<212> PRT
<213> Artificial Sequence

<220>
<223> Humanised sequence

<400> 10
Ala Ser Thr Lys Gly Pro Ser Val Phe Pro Leu Ala Pro Ser Ser Lys
 1               5                  10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Leu His Glu Ala Leu His Ser His Tyr Thr
305                 310                 315                 320
Gln Lys Ser Leu Ser Leu Ser Pro Gly Lys
                325                 330

<210> 11
<211> 1353
<212> DNA
<213> Artificial Sequence

<220>
<223> Humanised sequence

<400> 11
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccctgccc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 12
<211> 451
<212> PRT
<213> Artificial Sequence

<220>
<223> Humanised sequence

<400> 12
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val

145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 13
<211> 330
<212> PRT
<213> Artificial Sequence

<220>
<223> Humanised sequence

<400> 13

<210> 14
<211> 1353
<212> DNA
<213> Artificial Sequence

<220>
<223> Humanised sequence

<400> 14
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggacc aactgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgttgcacg aggccctgca caatcactac 1320
acccagaaga gcctgagcct gtcccctggc aag                              1353

<210> 15
<211> 451
<212> PRT
<213> Artificial Sequence

<220>
<223> Humanised sequence

<400> 15
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Gln Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Leu

                    420                     425                     430
His Glu Ala Leu His Asn His Tyr Thr Gln Lys Ser Leu Ser Leu Ser
        435                     440                     445
Pro Gly Lys
    450



<210> 16
<211> 330
<212> PRT
<213> Artificial Sequence

<220>
<223> Humanised sequence

<400> 16
Ala Ser Thr Lys Gly Pro Ser Val Phe Pro Leu Ala Pro Ser Ser Lys
 1               5                  10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Gln Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Leu His Glu Ala Leu His Asn His Tyr Thr
305                 310                 315                 320
Gln Lys Ser Leu Ser Leu Ser Pro Gly Lys
                325                 330



<210> 17
<211> 1353
<212> DNA

<213> Artificial Sequence

<220>
<223> Humanised sequence

<400> 17
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accttcctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 18
<211> 451
<212> PRT
<213> Artificial Sequence

<220>
<223> Humanised sequence

<400> 18
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Phe Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 19
<211> 330
<212> PRT
<213> Artificial Sequence

<220>
<223> Humanised sequence

<400> 19
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Phe Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210> 20
<211> 1353
<212> DNA
<213> Artificial Sequence

<220>
<223> Humanised sequence

<400> 20
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgagatca cctgtgtggt ggtggatgtg agccacgagg accctgaagt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag                              1353

<210> 21
<211> 451
<212> PRT
<213> Artificial Sequence

<220>
<223> Humanised sequence

<400> 21
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Ile Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 22
<211> 330
<212> PRT
<213> Artificial Sequence

<220>
<223> Humanised sequence

<400> 22
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Ile Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330


<210> 23
<211> 1353
<212> DNA
<213> Artificial Sequence

<220>
<223> Humanised sequence

<400> 23
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
ctggaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca ctatcactac 1320
acccagaaga gcctgagcct gtcccctggc aag 1353

<210> 24
<211> 451
<212> PRT
<213> Artificial Sequence

<220>
<223> Humanised sequence

<400> 24
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly

225                 230                 235                 240
Gly Pro Ser Val Phe Leu Phe Pro Pro Lys Pro Lys Asp Thr Leu Met
                245                 250                 255
Ile Ser Arg Thr Leu Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Tyr His Tyr Thr Gln Lys Ser Leu Ser Leu Ser
        435                 440                 445
Pro Gly Lys
    450


<210> 25
<211> 330
<212> PRT
<213> Artificial Sequence

<220>
<223> Humanised sequence

<400> 25
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Leu Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu

                180                     185                     190
His Gln Asp Trp Leu Asn Gly Lys Glu Tyr Lys Cys Lys Val Ser Asn
        195                     200                     205
Lys Ala Leu Pro Ala Pro Ile Glu Lys Thr Ile Ser Lys Ala Lys Gly
    210                     215                     220
Gln Pro Arg Glu Pro Gln Val Tyr Thr Leu Pro Pro Ser Arg Asp Glu
225                     230                     235                     240
Leu Thr Lys Asn Gln Val Ser Leu Thr Cys Leu Val Lys Gly Phe Tyr
                245                     250                     255
Pro Ser Asp Ile Ala Val Glu Trp Glu Ser Asn Gly Gln Pro Glu Asn
            260                     265                     270
Asn Tyr Lys Thr Thr Pro Pro Val Leu Asp Ser Asp Gly Ser Phe Phe
        275                     280                     285
Leu Tyr Ser Lys Leu Thr Val Asp Lys Ser Arg Trp Gln Gln Gly Asn
    290                     295                     300
Val Phe Ser Cys Ser Val Met His Glu Ala Leu His Tyr His Tyr Thr
305                     310                     315                     320
Gln Lys Ser Leu Ser Leu Ser Pro Gly Lys
                325                     330


<210> 26
<211> 19
<212> PRT
<213> Artificial Sequence

<220>
<223> Humanised sequence

<400> 26
Met Gly Trp Ser Cys Ile Ile Leu Phe Leu Val Ala Thr Ala Thr Gly
 1               5                  10                  15
Val His Ser



<210> 27
<211> 5
<212> PRT
<213> Artificial Sequence

<220>
<223> Humanised sequence

<400> 27
Asp Tyr Ala Met His
 1               5


<210> 28
<211> 17
<212> PRT
<213> Artificial Sequence

<220>
<223> Humanised sequence

<400> 28
Ala Ile Thr Trp Asn Ser Gly His Ile Asp Tyr Ala Asp Ser Val Glu
 1               5                  10                  15
Gly

<210> 29
<211> 12
<212> PRT
<213> Artificial Sequence

<220>
<223> Humanised sequence

<400> 29
Val Ser Tyr Leu Ser Thr Ala Ser Ser Leu Asp Tyr
1               5                   10


<210> 30
<211> 11
<212> PRT
<213> Artificial Sequence

<220>
<223> Humanised sequence

<400> 30
Arg Ala Ser Gln Gly Ile Arg Asn Tyr Leu Ala
1               5                   10


<210> 31
<211> 7
<212> PRT
<213> Artificial Sequence

<220>
<223> Humanised sequence

<400> 31
Ala Ala Ser Thr Leu Gln Ser
1               5


<210> 32
<211> 9
<212> PRT
<213> Artificial Sequence

<220>
<223> Humanised sequence

<400> 32
Gln Arg Tyr Asn Arg Ala Pro Tyr Thr
1               5


<210> 33
<211> 5
<212> PRT
<213> Artificial Sequence

<220>
<223> Humanised sequence

<400> 33
Gln Tyr Ala Met His

1                   5


<210> 34
<211> 5
<212> PRT
<213> Artificial Sequence

<220>
<223> Humanised sequence

<400> 34
His Tyr Ala Leu His
1                   5


<210> 35
<211> 5
<212> PRT
<213> Artificial Sequence

<220>
<223> Humanised sequence

<400> 35
His Tyr Ala Met His
1                   5


<210> 36
<211> 5
<212> PRT
<213> Artificial Sequence

<220>
<223> Humanised sequence

<400> 36
Gln His Ala Leu His
1                   5


<210> 37
<211> 5
<212> PRT
<213> Artificial Sequence

<220>
<223> Humanised sequence

<400> 37
Gln His Ala Met His
1                   5


<210> 38
<211> 5
<212> PRT
<213> Artificial Sequence

<220>
<223> Humanised sequence

<400> 38
Asp His Ala Leu His
1               5

<210> 39
<211> 214
<212> PRT
<213> Artificial Sequence

<220>
<223> Humanised sequence

<400> 39
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asn Val Gly Thr Asn
            20                  25                  30
Val Ala Trp Tyr Gln Gln Lys Pro Gly Lys Ala Pro Lys Ala Leu Ile
        35                  40                  45
Tyr Ser Ala Ser Phe Leu Tyr Ser Gly Val Pro Tyr Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ile Tyr Pro Leu
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 40
<211> 12
<212> PRT
<213> Artificial Sequence

<220>
<223> Humanised sequence

<400> 40
Val His Tyr Leu Ser Thr Ala Ser Gln Leu His His
1               5                   10

<210> 41
<211> 12
<212> PRT
<213> Artificial Sequence

<220>

<223> Humanised sequence

<400> 41
Val Gln Tyr Leu Ser Thr Ala Ser Ser Leu Gln Ser
1               5                   10


<210> 42
<211> 12
<212> PRT
<213> Artificial Sequence

<220>
<223> Humanised sequence

<400> 42
Val Lys Tyr Leu Ser Thr Ala Ser Ser Leu His Tyr
1               5                   10


<210> 43
<211> 12
<212> PRT
<213> Artificial Sequence

<220>
<223> Humanised sequence

<400> 43
Val Lys Tyr Leu Ser Thr Ala Ser Asn Leu Glu Ser
1               5                   10


<210> 44
<211> 12
<212> PRT
<213> Artificial Sequence

<220>
<223> Humanised sequence

<400> 44
Val His Tyr Leu Ser Thr Ala Ser Ser Leu Asp Tyr
1               5                   10


<210> 45
<211> 12
<212> PRT
<213> Artificial Sequence

<220>
<223> Humanised sequence

<400> 45
Val Ser Tyr Leu Ser Thr Ala Ser Ser Leu Gln Ser
1               5                   10


<210> 46
<211> 12
<212> PRT
<213> Artificial Sequence

<220>
<223> Humanised sequence

<400> 46
Val Arg Tyr Leu Ser Thr Ala Ser Asn Leu Gln His
1               5                   10


<210> 47
<211> 12
<212> PRT
<213> Artificial Sequence

<220>
<223> Humanised sequence

<400> 47
Val Gln Tyr Leu Ser Thr Ala Ser Gln Leu His Ser
1               5                   10


<210> 48
<211> 12
<212> PRT
<213> Artificial Sequence

<220>
<223> Humanised sequence

<400> 48
Val Arg Tyr Leu Ser Thr Ala Ser Gln Leu Asp Tyr
1               5                   10


<210> 49
<211> 12
<212> PRT
<213> Artificial Sequence

<220>
<223> Humanised sequence

<400> 49
Val Arg Tyr Leu Ser Thr Ala Ser Ser Leu Asp Tyr
1               5                   10


<210> 50
<211> 11
<212> PRT
<213> Artificial Sequence

<220>
<223> Humanised sequence

<400> 50
His Ala Ser Lys Lys Ile Arg Asn Tyr Leu Ala
1               5                   10


<210> 51
<211> 11

<212> PRT
<213> Artificial Sequence

<220>
<223> Humanised sequence

<400> 51
His Ala Ser Arg Lys Leu Arg Asn Tyr Leu Ala
1               5                   10


<210> 52
<211> 11
<212> PRT
<213> Artificial Sequence

<220>
<223> Humanised sequence

<400> 52
His Ala Ser Arg Arg Leu Arg Asn Tyr Leu Ala
1               5                   10


<210> 53
<211> 11
<212> PRT
<213> Artificial Sequence

<220>
<223> Humanised sequence

<400> 53
His Ala Ser Lys Arg Ile Arg Asn Tyr Leu Ala
1               5                   10


<210> 54
<211> 11
<212> PRT
<213> Artificial Sequence

<220>
<223> Humanised sequence

<400> 54
His Ala Ser Arg Lys Ile Arg Asn Tyr Leu Ala
1               5                   10


<210> 55
<211> 11
<212> PRT
<213> Artificial Sequence

<220>
<223> Humanised sequence

<400> 55
His Ala Ser Arg Arg Ile Arg Asn Tyr Leu Ala
1               5                   10

<210> 56
<211> 11
<212> PRT
<213> Artificial Sequence

<220>
<223> Humanised sequence

<400> 56
His Ala Ser Arg Glu Ile Arg Asn Tyr Leu Ala
1               5                   10


<210> 57
<211> 11
<212> PRT
<213> Artificial Sequence

<220>
<223> Humanised sequence

<400> 57
His Ala Ser Gln Gly Ile Arg Asn Tyr Leu Ala
1               5                   10


<210> 58
<211> 11
<212> PRT
<213> Artificial Sequence

<220>
<223> Humanised sequence

<400> 58
His Ala Ser Gln Lys Ile Arg Asn Tyr Leu Ala
1               5                   10


<210> 59
<211> 11
<212> PRT
<213> Artificial Sequence

<220>
<223> Humanised sequence

<400> 59
Arg Ala Ser Arg Gly Leu Arg Asn Tyr Leu Ala
1               5                   10


<210> 60
<211> 11
<212> PRT
<213> Artificial Sequence

<220>
<223> Humanised sequence

<400> 60
His Ala Ser Gln Arg Ile Arg Asn Tyr Leu Ala
1               5                   10

<210> 61
<211> 11
<212> PRT
<213> Artificial Sequence

<220>
<223> Humanised sequence

<400> 61
Arg Ala Ser Arg Arg Ile Arg Asn Tyr Leu Ala
1               5                   10


<210> 62
<211> 7
<212> PRT
<213> Artificial Sequence

<220>
<223> Humanised sequence

<400> 62
Ala Ala Ser Ser Leu Leu Arg
1               5


<210> 63
<211> 7
<212> PRT
<213> Artificial Sequence

<220>
<223> Humanised sequence

<400> 63
Ala Ala Ser Ser Leu Leu Lys
1               5


<210> 64
<211> 7
<212> PRT
<213> Artificial Sequence

<220>
<223> Humanised sequence

<400> 64
Ala Ala Ser Ser Leu Leu Pro
1               5


<210> 65
<211> 7
<212> PRT
<213> Artificial Sequence

<220>
<223> Humanised sequence

<400> 65

Ala Ala Ser Ser Leu Gln Pro
1               5

<210> 66
<211> 7
<212> PRT
<213> Artificial Sequence

<220>
<223> Humanised sequence

<400> 66
Ala Ala Ser Ser Leu Leu His
1               5

<210> 67
<211> 7
<212> PRT
<213> Artificial Sequence

<220>
<223> Humanised sequence

<400> 67
Ala Ala Ser Ser Phe Leu Pro
1               5

<210> 68
<211> 7
<212> PRT
<213> Artificial Sequence

<220>
<223> Humanised sequence

<400> 68
Ala Ala Ser Ser Leu Leu Gln
1               5

<210> 69
<211> 7
<212> PRT
<213> Artificial Sequence

<220>
<223> Humanised sequence

<400> 69
Ala Ala Ser Ser Leu Gln Gln
1               5

<210> 70
<211> 7
<212> PRT
<213> Artificial Sequence

<220>
<223> Humanised sequence

<400> 70
Ala Ala Ser Thr Leu Leu Lys
1               5

<210> 71
<211> 7
<212> PRT
<213> Artificial Sequence

<220>
<223> Humanised sequence

<400> 71
Ala Ala Ser Ser Leu Gln Asn
1               5

<210> 72
<211> 7
<212> PRT
<213> Artificial Sequence

<220>
<223> Humanised sequence

<400> 72
Ala Ala Ser Ser Leu Gln Lys
1               5

<210> 73
<211> 9
<212> PRT
<213> Artificial Sequence

<220>
<223> Humanised sequence

<400> 73
Gln Arg Tyr Asp Arg Pro Pro Tyr Thr
1               5

<210> 74
<211> 9
<212> PRT
<213> Artificial Sequence

<220>
<223> Humanised sequence

<400> 74
Gln Arg Tyr Asp Lys Pro Pro Tyr Thr
1               5

<210> 75
<211> 9
<212> PRT
<213> Artificial Sequence

<220>
<223> Humanised sequence

<400> 75
Gln Arg Tyr Asn Arg Pro Pro Tyr Thr
1               5

<210> 76
<211> 9
<212> PRT
<213> Artificial Sequence

<220>
<223> Humanised sequence

<400> 76
Gln Arg Tyr Asn Lys Pro Pro Tyr Thr
1               5

<210> 77
<211> 363
<212> DNA
<213> Artificial Sequence

<220>
<223> Humanised sequence

<400> 77
gaggtgcagc tggtggagtc tggcggcgga ctggtgcagc ccggcagaag cctgagactg 60
agctgtgccg ccagcggctt caccttcgac cagtacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agc                                                               363

<210> 78
<211> 121
<212> PRT
<213> Artificial Sequence

<220>
<223> Humanised sequence

<400> 78
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser
        115                 120

<210> 79
<211> 363
<212> DNA
<213> Artificial Sequence

<220>
<223> Humanised sequence

<400> 79
gaggtgcagc tggtggagtc tggcggcgga ctggtgcagc ccggcagaag cctgagactg 60
agctgtgccg ccagcggctt caccttcgac gaccacgccc tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgagg 300
tacctgagca ccgccagcag cctggactac tggggccagg gcacactagt gaccgtgtcc 360
agc 363

<210> 80
<211> 121
<212> PRT
<213> Artificial Sequence

<220>
<223> Humanised sequence

<400> 80
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp His
            20                  25                  30
Ala Leu His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Arg Tyr Leu Ser Thr Ala Ser Ser Leu Asp Tyr Trp Gly
            100                 105                 110
Gln Gly Thr Leu Val Thr Val Ser Ser
        115                 120

<210> 81
<211> 363
<212> DNA
<213> Artificial Sequence

<220>
<223> Humanised sequence

<400> 81
gaggtgcagc tggtggagtc tggcggcgga ctggtgcagc ccggcagaag cctgagactg 60
agctgtgccg ccagcggctt caccttcgac cactacgccc tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agc 363

<210> 82
<211> 121
<212> PRT
<213> Artificial Sequence

<220>
<223> Humanised sequence

<400> 82
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp His Tyr
            20                  25                  30
Ala Leu His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser
        115                 120


<210> 83
<211> 363
<212> DNA
<213> Artificial Sequence

<220>
<223> Humanised sequence

<400> 83
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgcac 300
tacctgagca ccgccagcca actgcaccac tggggccagg gcacactagt gaccgtgtcc 360
agc                                                               363

<210> 84
<211> 121
<212> PRT
<213> Artificial Sequence

<220>
<223> Humanised sequence

<400> 84
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95
Ala Lys Val His Tyr Leu Ser Thr Ala Ser Gln Leu His His Trp Gly
            100                 105                 110
Gln Gly Thr Leu Val Thr Val Ser Ser
        115                 120

<210> 85
<211> 363
<212> DNA
<213> Artificial Sequence

<220>
<223> Humanised sequence

<400> 85
gaggtgcagc tggtggagtc tggcggcgga ctggtgcagc ccggcagaag cctgagactg 60
agctgtgccg ccagcggctt caccttcgac cactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgcag 300
tacctgagca ccgccagcag cctgcagagc tggggccagg gcacactagt gaccgtgtcc 360
agc                                                               363

<210> 86
<211> 121
<212> PRT
<213> Artificial Sequence

<220>
<223> Humanised sequence

<400> 86
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp His Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Gln Tyr Leu Ser Thr Ala Ser Ser Leu Gln Ser Trp Gly
            100                 105                 110
Gln Gly Thr Leu Val Thr Val Ser Ser
        115                 120

<210> 87
<211> 363
<212> DNA
<213> Artificial Sequence

<220>
<223> Humanised sequence

<400> 87
gaggtgcagc tggtggagtc tggcggcgga ctggtgcagc ccggcagaag cctgagactg 60
agctgtgccg ccagcggctt caccttcgac cagtacgcca tgcactgggt gaggcaggcc 120

cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgaag 300
tacctgagca ccgccagcag cctgcactac tggggccagg gcacactagt gaccgtgtcc 360
agc 363

<210> 88
<211> 121
<212> PRT
<213> Artificial Sequence

<220>
<223> Humanised sequence

<400> 88
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Lys Tyr Leu Ser Thr Ala Ser Ser Leu His Tyr Trp Gly
            100                 105                 110
Gln Gly Thr Leu Val Thr Val Ser Ser
        115                 120

<210> 89
<211> 363
<212> DNA
<213> Artificial Sequence

<220>
<223> Humanised sequence

<400> 89
gaggtgcagc tggtggagtc tggcggcgga ctggtgcagc ccggcagaag cctgagactg 60
agctgtgccg ccagcggctt caccttcgac cagcacgccc tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgcac 300
tacctgagca ccgccagcag cctggactac tggggccagg gcacactagt gaccgtgtcc 360
agc 363

<210> 90
<211> 121
<212> PRT
<213> Artificial Sequence

<220>
<223> Humanised sequence

<400> 90
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln His
            20                  25                  30

Ala Leu His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val His Tyr Leu Ser Thr Ala Ser Ser Leu Asp Tyr Trp Gly
            100                 105                 110
Gln Gly Thr Leu Val Thr Val Ser Ser
        115                 120

<210> 91
<211> 363
<212> DNA
<213> Artificial Sequence

<220>
<223> Humanised sequence

<400> 91
gaggtgcagc tggtggagtc tggcggcgga ctggtgcagc ccggcagaag cctgagactg 60
agctgtgccg ccagcggctt caccttcgac cagtacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgcac 300
tacctgagca ccgccagcca gctgcaccac tggggccagg gcacactagt gaccgtgtcc 360
agc                                                               363

<210> 92
<211> 121
<212> PRT
<213> Artificial Sequence

<220>
<223> Humanised sequence

<400> 92
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val His Tyr Leu Ser Thr Ala Ser Gln Leu His His Trp Gly
            100                 105                 110
Gln Gly Thr Leu Val Thr Val Ser Ser
        115                 120

<210> 93
<211> 363
<212> DNA
<213> Artificial Sequence

<220>
<223> Humanised sequence

<400> 93
gaggtgcagc tggtggagtc tggcggcgga ctggtgcagc ccggcagaag cctgagactg 60
agctgtgccg ccagcggctt caccttcgac cagcacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agc 363

<210> 94
<211> 121
<212> PRT
<213> Artificial Sequence

<220>
<223> Humanised sequence

<400> 94
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln His
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser
        115                 120

<210> 95
<211> 363
<212> DNA
<213> Artificial Sequence

<220>
<223> Humanised sequence

<400> 95
gaggtgcagc tggtggagtc tggcggcgga ctggtgcagc ccggcagaag cctgagactg 60
agctgtgccg ccagcggctt caccttcgac cagtacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgaag 300
tacctgagca ccgccagcaa cctggagagc tggggccagg gcacactagt gaccgtgtcc 360
agc 363

<210> 96
<211> 121
<212> PRT
<213> Artificial Sequence

<220>
<223> Humanised sequence

<400> 96
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Lys Tyr Leu Ser Thr Ala Ser Asn Leu Glu Ser Trp Gly
            100                 105                 110
Gln Gly Thr Leu Val Thr Val Ser Ser
        115                 120

<210> 97
<211> 327
<212> DNA
<213> Artificial Sequence

<220>
<223> Humanised sequence

<400> 97
gatatccaga tgacccagag ccccagcagc ctgagcgcct ctgtgggcga tagagtgacc 60
atcacctgcc acgccagcaa gaagatcaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 98
<211> 109
<212> PRT
<213> Artificial Sequence

<220>
<223> Humanised sequence

<400> 98
Asp Ile Gln Met Thr Gln Ser Pro Ser Ser Leu Ser Ala Ser Val Gly
1               5                   10                  15
Asp Arg Val Thr Ile Thr Cys His Ala Ser Lys Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 99
<211> 327

<212> DNA
<213> Artificial Sequence

<220>
<223> Humanised sequence

<400> 99
gatatccaga tgacccagag ccccagcagc ctgagcgcct ctgtgggcga tagagtgacc 60
atcacctgcc acgccagcag gaagctgaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 100
<211> 109
<212> PRT
<213> Artificial Sequence

<220>
<223> Humanised sequence

<400> 100
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 101
<211> 327
<212> DNA
<213> Artificial Sequence

<220>
<223> Humanised sequence

<400> 101
gatatccaga tgacccagag ccccagcagc ctgagcgcct ctgtgggcga tagagtgacc 60
atcacctgcc acgccagcag gaggctgaga aactacctgg cctggtatca gcagaagcct 120
ggcaaggccc ctaagctgct gatctacgcc gccagcagcc tgctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 102
<211> 109
<212> PRT
<213> Artificial Sequence

<220>
<223> Humanised sequence

<400> 102
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 103
<211> 327
<212> DNA
<213> Artificial Sequence

<220>
<223> Humanised sequence

<400> 103
gatatccaga tgacccagag ccccagcagc ctgagcgcct ctgtgggcga tagagtgacc 60
atcacctgcc acgccagcag gaagctgaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 104
<211> 109
<212> PRT
<213> Artificial Sequence

<220>
<223> Humanised sequence

<400> 104
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 105
<211> 327
<212> DNA
<213> Artificial Sequence

<220>
<223> Humanised sequence

<400> 105
gatatccaga tgacccagag ccccagcagc ctgagcgcct ctgtgggcga tagagtgacc 60
atcacctgcc acgccagcaa gaggatcaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacgacaagc ccccttacac cttcggccag 300
ggcaccaagg tggagatcaa gcgtacg 327

<210> 106
<211> 109
<212> PRT
<213> Artificial Sequence

<220>
<223> Humanised sequence

<400> 106
Asp Ile Gln Met Thr Gln Ser Pro Ser Ser Leu Ser Ala Ser Val Gly
1               5                   10                  15
Asp Arg Val Thr Ile Thr Cys His Ala Ser Lys Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Lys Pro Pro Tyr
                85                  90                  95
Thr Phe Gly Gln Gly Thr Lys Val Glu Ile Lys Arg Thr
            100                 105

<210> 107
<211> 327
<212> DNA
<213> Artificial Sequence

<220>
<223> Humanised sequence

<400> 107
gatatccaga tgacccagag ccccagcagc ctgagcgcct ctgtgggcga tagagtgacc 60
atcacctgcc acgccagcag gaagctgaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacaacagac ccccttacac cttcggccag 300
ggcaccaagg tggagatcaa gcgtacg 327

<210> 108
<211> 109
<212> PRT
<213> Artificial Sequence

<220>
<223> Humanised sequence

<400> 108
Asp Ile Gln Met Thr Gln Ser Pro Ser Ser Leu Ser Ala Ser Val Gly
1               5                   10                  15

Asp Arg Val Thr Ile Thr Cys His Ala Ser Arg Lys Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Arg Pro Pro Tyr
                85                  90                  95
Thr Phe Gly Gln Gly Thr Lys Val Glu Ile Lys Arg Thr
            100                 105

<210> 109
<211> 327
<212> DNA
<213> Artificial Sequence

<220>
<223> Humanised sequence

<400> 109
gatatccaga tgacccagag ccccagcagc ctgagcgcct ctgtgggcga tagagtgacc 60
atcacctgcc acgccagcag gaagatcaga aactacctgg cctggtatca gcagaagcct 120
ggcaaggccc ctaagctgct gatctacgcc gccagcagcc tgcagcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 110
<211> 109
<212> PRT
<213> Artificial Sequence

<220>
<223> Humanised sequence

<400> 110
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 111
<211> 327
<212> DNA
<213> Artificial Sequence

<220>
<223> Humanised sequence

<400> 111
gatatccaga tgacccagag ccccagcagc ctgagcgcct ctgtgggcga tagagtgacc 60
atcacctgcc acgccagcag gaggatcaga aactacctgg cctggtatca gcagaagcct 120
ggcaaggccc ctaagctgct gatctacgcc gccagcagcc tgctgcacgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 112
<211> 109
<212> PRT
<213> Artificial Sequence

<220>
<223> Humanised sequence

<400> 112
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu His Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 113
<211> 327
<212> DNA
<213> Artificial Sequence

<220>
<223> Humanised sequence

<400> 113
gatatccaga tgacccagag ccccagcagc ctgagcgcct ctgtgggcga tagagtgacc 60
atcacctgcc acgccagcag gaggctgaga aactacctgg cctggtatca gcagaagcct 120
ggcaaggccc ctaagctgct gatctacgcc gccagcagcc tgcagcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 114
<211> 109
<212> PRT
<213> Artificial Sequence

<220>
<223> Humanised sequence

<400> 114
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Leu Arg Asn Tyr
            20                  25                  30
Leu Ala Trp Tyr Gln Gln Lys Pro Gly Lys Ala Pro Lys Leu Leu Ile

35                  40                  45
Tyr Ala Ala Ser Ser Leu Gln Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 115
<211> 327
<212> DNA
<213> Artificial Sequence

<220>
<223> Humanised sequence

<400> 115
gatatccaga tgacccagag ccccagcagc ctgagcgcct ctgtgggcga tagagtgacc 60
atcacctgcc acgccagcag gaggctgaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacaacaagc ccccttacac cttcggccag 300
ggcaccaagg tggagatcaa gcgtacg                                     327

<210> 116
<211> 109
<212> PRT
<213> Artificial Sequence

<220>
<223> Humanised sequence

<400> 116
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Lys Pro Pro Tyr
                85                  90                  95
Thr Phe Gly Gln Gly Thr Lys Val Glu Ile Lys Arg Thr
            100                 105

<210> 117
<211> 327
<212> DNA
<213> Artificial Sequence

<220>
<223> Humanised sequence

<400> 117
gatatccaga tgacccagag ccccagcagc ctgagcgcct ctgtgggcga tagagtgacc 60
atcacctgcc acgccagcag gaagatcaga aactacctgg cctggtatca gcagaagcct 120

ggcaaggccc ctaagctgct gatctacgcc gccagcagct tcctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 118
<211> 109
<212> PRT
<213> Artificial Sequence

<220>
<223> Humanised sequence

<400> 118
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Phe Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 119
<211> 327
<212> DNA
<213> Artificial Sequence

<220>
<223> Humanised sequence

<400> 119
gatatccaga tgacccagag ccccagcagc ctgagcgcct ctgtgggcga tagagtgacc 60
atcacctgcc acgccagcaa gaagatcaga aactacctgg cctggtatca gcagaagcct 120
ggcaaggccc ctaagctgct gatctacgcc gccagcagcc tgcagcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 120
<211> 109
<212> PRT
<213> Artificial Sequence

<220>
<223> Humanised sequence

<400> 120
Asp Ile Gln Met Thr Gln Ser Pro Ser Ser Leu Ser Ala Ser Val Gly
1               5                   10                  15
Asp Arg Val Thr Ile Thr Cys His Ala Ser Lys Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 121
<211> 327
<212> DNA
<213> Artificial Sequence

<220>
<223> Humanised sequence

<400> 121
gatatccaga tgacccagag ccccagcagc ctgagcgcct ctgtgggcga tagagtgacc 60
atcacctgcc acgccagcag gaggatcaga aactacctgg cctggtatca gcagaagcct 120
ggcaaggccc ctaagctgct gatctacgcc gccagcagcc tgctgcaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 122
<211> 109
<212> PRT
<213> Artificial Sequence

<220>
<223> Humanised sequence

<400> 122
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Gln Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 123
<211> 327
<212> DNA
<213> Artificial Sequence

<220>
<223> Humanised sequence

<400> 123
gatatccaga tgacccagag ccccagcagc ctgagcgcct ctgtgggcga tagagtgacc 60
atcacctgcc acgccagcag gaagctgaga aactacctgg cctggtatca gcagaagcct 120
ggcaaggccc ctaagctgct gatctacgcc gccagcagcc tgcagcaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300

ggcaccaagg tggagatcaa gcgtacg                                          327

<210> 124
<211> 109
<212> PRT
<213> Artificial Sequence

<220>
<223> Humanised sequence

<400> 124
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Gln Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105


<210> 125
<211> 327
<212> DNA
<213> Artificial Sequence

<220>
<223> Humanised sequence

<400> 125
gatatccaga tgacccagag ccccagcagc ctgagcgcct ctgtgggcga tagagtgacc 60
atcacctgcc acgccagcag gaagctgaga aactacctgg cctggtatca gcagaagcct 120
ggcaaggccc ctaagctgct gatctacgcc gccagcagcc tgctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                          327

<210> 126
<211> 109
<212> PRT
<213> Artificial Sequence

<220>
<223> Humanised sequence

<400> 126
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr

                85                  90                  95
Thr Phe Gly Gln Gly Thr Lys Val Glu Ile Lys Arg Thr
            100                 105

<210> 127
<211> 327
<212> DNA
<213> Artificial Sequence

<220>
<223> Humanised sequence

<400> 127
gatatccaga tgacccagag ccccagcagc ctgagcgcct ctgtgggcga tagagtgacc 60
atcacctgcc acgccagcag ggagatcaga aactacctgg cctggtatca gcagaagcct 120
ggcaaggccc ctaagctgct gatctacgcc gccagcagcc tgctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 128
<211> 109
<212> PRT
<213> Artificial Sequence

<220>
<223> Humanised sequence

<400> 128
Asp Ile Gln Met Thr Gln Ser Pro Ser Ser Leu Ser Ala Ser Val Gly
1               5                   10                  15
Asp Arg Val Thr Ile Thr Cys His Ala Ser Arg Glu Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 129
<211> 327
<212> DNA
<213> Artificial Sequence

<220>
<223> Humanised sequence

<400> 129
gatatccaga tgacccagag ccccagcagc ctgagcgcct ctgtgggcga tagagtgacc 60
atcacctgcc acgccagcca gggcatcaga aactacctgg cctggtatca gcagaagcct 120
ggcaaggccc ctaagctgct gatctacgcc gccagcaccc tgctgaaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 130

<211> 109
<212> PRT
<213> Artificial Sequence

<220>
<223> Humanised sequence

<400> 130
Asp Ile Gln Met Thr Gln Ser Pro Ser Ser Leu Ser Ala Ser Val Gly
1               5                   10                  15
Asp Arg Val Thr Ile Thr Cys His Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 131
<211> 327
<212> DNA
<213> Artificial Sequence

<220>
<223> Humanised sequence

<400> 131
gatatccaga tgacccagag ccccagcagc ctgagcgcct ctgtgggcga tagagtgacc 60
atcacctgcc acgccagcca gaagatcaga aactacctgg cctggtatca gcagaagcct 120
ggcaaggccc ctaagctgct gatctacgcc gccagcagcc tgcagcaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 132
<211> 109
<212> PRT
<213> Artificial Sequence

<220>
<223> Humanised sequence

<400> 132
Asp Ile Gln Met Thr Gln Ser Pro Ser Ser Leu Ser Ala Ser Val Gly
1               5                   10                  15
Asp Arg Val Thr Ile Thr Cys His Ala Ser Gln Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Gln Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 133
<211> 327
<212> DNA
<213> Artificial Sequence

<220>
<223> Humanised sequence

<400> 133
gatatccaga tgacccagag ccccagcagc ctgagcgcct ctgtgggcga tagagtgacc 60
atcacctgcc acgccagcag gaggctgaga aactacctgg cctggtatca gcagaagcct 120
ggcaaggccc ctaagctgct gatctacgcc gccagcagcc tgctgcacgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 134
<211> 109
<212> PRT
<213> Artificial Sequence

<220>
<223> Humanised sequence

<400> 134
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu His Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 135
<211> 327
<212> DNA
<213> Artificial Sequence

<220>
<223> Humanised sequence

<400> 135
gatatccaga tgacccagag ccccagcagc ctgagcgcct ctgtgggcga tagagtgacc 60
atcacctgcc acgccagcag gaggctgaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg 327

<210> 136
<211> 109
<212> PRT
<213> Artificial Sequence

<220>
<223> Humanised sequence

<400> 136
Asp Ile Gln Met Thr Gln Ser Pro Ser Ser Leu Ser Ala Ser Val Gly
1               5                   10                  15
Asp Arg Val Thr Ile Thr Cys His Ala Ser Arg Arg Leu Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 137
<211> 327
<212> DNA
<213> Artificial Sequence

<220>
<223> Humanised sequence

<400> 137
gatatccaga tgacccagag ccccagcagc ctgagcgcct ctgtgggcga tagagtgacc 60
atcacctgcc acgccagcaa gaggatcaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacaacaagc ccccttacac cttcggccag 300
ggcaccaagg tggagatcaa gcgtacg                                     327

<210> 138
<211> 109
<212> PRT
<213> Artificial Sequence

<220>
<223> Humanised sequence

<400> 138
Asp Ile Gln Met Thr Gln Ser Pro Ser Ser Leu Ser Ala Ser Val Gly
1               5                   10                  15
Asp Arg Val Thr Ile Thr Cys His Ala Ser Lys Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Lys Pro Pro Tyr
                85                  90                  95
Thr Phe Gly Gln Gly Thr Lys Val Glu Ile Lys Arg Thr
            100                 105

<210> 139

<211> 327
<212> DNA
<213> Artificial Sequence

<220>
<223> Humanised sequence

<400> 139
gatatccaga tgacccagag ccccagcagc ctgagcgcct ctgtgggcga tagagtgacc 60
atcacctgcc acgccagcag gaagatcaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacgacaagc ccccttacac cttcggccag 300
ggcaccaagg tggagatcaa gcgtacg 327

<210> 140
<211> 109
<212> PRT
<213> Artificial Sequence

<220>
<223> Humanised sequence

<400> 140
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Lys Pro Pro Tyr
                85                  90                  95
Thr Phe Gly Gln Gly Thr Lys Val Glu Ile Lys Arg Thr
            100                 105


<210> 141
<211> 327
<212> DNA
<213> Artificial Sequence

<220>
<223> Humanised sequence

<400> 141
gatatccaga tgacccagag ccccagcagc ctgagcgcct ctgtgggcga tagagtgacc 60
atcacctgcc acgccagcaa gaggatcaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacaacaagc ccccttacac cttcggccag 300
ggcaccaagg tggagatcaa gcgtacg 327

<210> 142
<211> 109
<212> PRT
<213> Artificial Sequence

<220>
<223> Humanised sequence

<400> 142
Asp Ile Gln Met Thr Gln Ser Pro Ser Ser Leu Ser Ala Ser Val Gly
1               5                   10                  15
Asp Arg Val Thr Ile Thr Cys His Ala Ser Lys Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Lys Pro Pro Tyr
                85                  90                  95
Thr Phe Gly Gln Gly Thr Lys Val Glu Ile Lys Arg Thr
            100                 105

<210> 143
<211> 327
<212> DNA
<213> Artificial Sequence

<220>
<223> Humanised sequence

<400> 143
gatatccaga tgacccagag ccccagcagc ctgagcgcct ctgtgggcga tagagtgacc 60
atcacctgcc acgccagcag gaagatcaga aactacctgg cctggtatca gcagaagcct 120
ggcaaggccc ctaagctgct gatctacgcc gccagcagcc tgctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacg                                     327

<210> 144
<211> 109
<212> PRT
<213> Artificial Sequence

<220>
<223> Humanised sequence

<400> 144
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr
            100                 105

<210> 145
<211> 451
<212> PRT
<213> Artificial Sequence

<220>
<223> Humanised sequence

<400> 145
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Tyr
                245                 250                 255
Ile Thr Arg Glu Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 146
<211> 451
<212> PRT
<213> Artificial Sequence

<220>
<223> Humanised sequence

<400> 146
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp His
            20                  25                  30
Ala Leu His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Arg Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Tyr
                245                 250                 255
Ile Thr Arg Glu Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 147
<211> 642
<212> DNA
<213> Artificial Sequence

<220>
<223> Humanised sequence

<400> 147
gatatccaga tgacccagag ccccagcagc ctgagcgcct ctgtgggcga tagagtgacc 60
atcacctgcc acgccagcag gaagatcaga aactacctgg cctggtatca gcagaagcct 120
ggcaaggccc ctaagctgct gatctacgcc gccagcagcc tgctgagggg cgtgcccagc 180
agattcagcg gcagcggctc cggcaccgac ttcaccctga ccatcagcag cctgcagccc 240
gaggacgtgg ccacctacta ctgccagcgg tacgacaagc ccccttacac cttcggccag 300
ggcaccaagg tggagatcaa gcgtacggtg gccgccccca gcgtgttcat cttccccccc 360
agcgatgagc agctgaagag cggcaccgcc agcgtggtgt gtctgctgaa caacttctac 420
ccccgggagg ccaaggtgca gtggaaggtg gacaatgccc tgcagagcgg caacagccag 480
gagagcgtga ccgagcagga cagcaaggac tccacctaca gcctgagcag caccctgacc 540
ctgagcaagg ccgactacga gaagcacaag gtgtacgcct gtgaggtgac ccaccagggc 600
ctgtccagcc ccgtgaccaa gagcttcaac cggggcgagt gc 642

<210> 148
<211> 214
<212> PRT
<213> Artificial Sequence

<220>
<223> Humanised sequence

<400> 148
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Lys Pro Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser

195                 200                 205
Phe Asn Arg Gly Glu Cys
    210



<210> 149
<211> 642
<212> DNA
<213> Artificial Sequence

<220>
<223> Humanised sequence

<400> 149
gatatccaga tgacccagag ccccagcagc ctgagcgcct ctgtgggcga tagagtgacc 60
atcacctgcc acgccagcaa gaggatcaga aactacctgg cctggtatca gcagaagcct 120
ggcaaggccc ctaagctgct gatctacgcc gccagcagcc tgctgaaggg cgtgcccagc 180
agattcagcg gcagcggctc cggcaccgac ttcaccctga ccatcagcag cctgcagccc 240
gaggacgtgg ccacctacta ctgccagcgg tacaacaagc ccccttacac cttcggccag 300
ggcaccaagg tggagatcaa gcgtacggtg gccgccccca gcgtgttcat cttccccccc 360
agcgatgagc agctgaagag cggcaccgcc agcgtggtgt gtctgctgaa caacttctac 420
ccccgggagg ccaaggtgca gtggaaggtg gacaatgccc tgcagagcgg caacagccag 480
gagagcgtga ccgagcagga cagcaaggac tccacctaca gcctgagcag caccctgacc 540
ctgagcaagg ccgactacga gaagcacaag gtgtacgcct gtgaggtgac ccaccagggc 600
ctgtccagcc ccgtgaccaa gagcttcaac cggggcgagt gc                    642

<210> 150
<211> 214
<212> PRT
<213> Artificial Sequence

<220>
<223> Humanised sequence

<400> 150
Asp Ile Gln Met Thr Gln Ser Pro Ser Ser Leu Ser Ala Ser Val Gly
1               5                   10                  15
Asp Arg Val Thr Ile Thr Cys His Ala Ser Lys Arg Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Lys Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Lys Pro Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 151
<211> 642
<212> DNA
<213> Artificial Sequence

<220>
<223> Humanised sequence

<400> 151
gatatccaga tgacccagag ccccagcagc ctgagcgcct ctgtgggcga tagagtgacc 60
atcacctgcc acgccagcag gaagatcaga aactacctgg cctggtatca gcagaagcct 120
ggcaaggccc ctaagctgct gatctacgcc gccagcagcc tgctgcccgg cgtgcccagc 180
agattcagcg gcagcggctc cggcaccgac ttcaccctga ccatcagcag cctgcagccc 240
gaggacgtgg ccacctacta ctgccagcgg tacgacagac ccccttacac cttcggccag 300
ggcaccaagg tggagatcaa gcgtacggtg gccgccccca gcgtgttcat cttccccccc 360
agcgatgagc agctgaagag cggcaccgcc agcgtggtgt gtctgctgaa caacttctac 420
ccccgggagg ccaaggtgca gtggaaggtg gacaatgccc tgcagagcgg caacagccag 480
gagagcgtga ccgagcagga cagcaaggac tccacctaca gcctgagcag caccctgacc 540
ctgagcaagg ccgactacga gaagcacaag gtgtacgcct gtgaggtgac ccaccagggc 600
ctgtccagcc ccgtgaccaa gagcttcaac cggggcgagt gc 642

<210> 152
<211> 214
<212> PRT
<213> Artificial Sequence

<220>
<223> Humanised sequence

<400> 152
Asp Ile Gln Met Thr Gln Ser Pro Ser Ser Leu Ser Ala Ser Val Gly
1               5                   10                  15
Asp Arg Val Thr Ile Thr Cys His Ala Ser Arg Lys Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Leu Pro Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asp Arg Pro Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 153

<211> 1353
<212> DNA
<213> Artificial Sequence

<220>
<223> Humanised sequence

<400> 153
gaggtgcagc tggtggagtc tggcggcgga ctggtgcagc ccggcagaag cctgagactg 60
agctgtgccg ccagcggctt caccttcgac cagtacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccctgccc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 154
<211> 451
<212> PRT
<213> Artificial Sequence

<220>
<223> Humanised sequence

<400> 154
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Gln Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 155
<211> 1353
<212> DNA
<213> Artificial Sequence

<220>
<223> Humanised sequence

<400> 155
gaggtgcagc tggtggagtc tggcggcgga ctggtgcagc ccggcagaag cctgagactg 60
agctgtgccg ccagcggctt caccttcgac gaccacgccc tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgagg 300
tacctgagca ccgccagcag cctggactac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960

aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 156
<211> 451
<212> PRT
<213> Artificial Sequence

<220>
<223> Humanised sequence

<400> 156
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp His
            20                  25                  30
Ala Leu His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Arg Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 157
<211> 1353
<212> DNA
<213> Artificial Sequence

<220>
<223> Humanised sequence

<400> 157
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgtacat caccagagag 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag                              1353

<210> 158
<211> 451
<212> PRT
<213> Artificial Sequence

<220>
<223> Humanised sequence

<400> 158
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser

50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Tyr
                245                 250                 255
Ile Thr Arg Glu Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 159
<211> 654
<212> DNA
<213> Artificial Sequence

<220>
<223> Humanised sequence

<400> 159
gacatcgtgc tgacccagag cccctcttcc ctgagcgcaa gcgtgggcga tagggtgacc 60

atcacctgca aggccagcca gagcgtggac tacgacggcg acagctacat gaactggtac 120
cagcagaagc ccggcaaggc ccccaaactg ctgatctacg ccgccagcaa cctcgagtca 180
ggcattccca gcaggtttag cggcagcggc agcggcaccg acttcacctt cacaatcagc 240
agcctgcagc ccgaggacat cgccacctac tactgccagc agagcaacga ggaccctccc 300
accttcggac agggcaccaa ggtcgagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgc 654

<210> 160
<211> 218
<212> PRT
<213> Artificial Sequence

<220>
<223> Humanised sequence

<400> 160
Asp Ile Val Leu Thr Gln Ser Pro Ser Ser Leu Ser Ala Ser Val Gly
1               5                   10                  15
Asp Arg Val Thr Ile Thr Cys Lys Ala Ser Gln Ser Val Asp Tyr Asp
            20                  25                  30
Gly Asp Ser Tyr Met Asn Trp Tyr Gln Gln Lys Pro Gly Lys Ala Pro
        35                  40                  45
Lys Leu Leu Ile Tyr Ala Ala Ser Asn Leu Glu Ser Gly Ile Pro Ser
    50                  55                  60
Arg Phe Ser Gly Ser Gly Ser Gly Thr Asp Phe Thr Phe Thr Ile Ser
65                  70                  75                  80
Ser Leu Gln Pro Glu Asp Ile Ala Thr Tyr Tyr Cys Gln Gln Ser Asn
                85                  90                  95
Glu Asp Pro Pro Thr Phe Gly Gln Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210> 161
<211> 451
<212> PRT
<213> Artificial Sequence

<220>
<223> Humanised sequence

<400> 161
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30

Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 162
<211> 451
<212> PRT
<213> Artificial Sequence

<220>
<223> Humanised sequence

<400> 162
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Leu
            420                 425                 430
His Glu Ala Leu His Ser His Tyr Thr Gln Lys Ser Leu Ser Leu Ser
        435                 440                 445
Pro Gly Lys
    450

<210> 163

<211> 330
<212> PRT
<213> Artificial Sequence

<220>
<223> Humanised sequence

<400> 163
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Glu Glu
225                 230                 235                 240
Met Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Leu His Glu Ala Leu His Ser His Tyr Thr
305                 310                 315                 320
Gln Lys Ser Leu Ser Leu Ser Pro Gly Lys
                325                 330


<210> 164
<211> 451
<212> PRT
<213> Artificial Sequence

<220>
<223> Humanised sequence

<400> 164
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu Lys Phe His Tyr Thr Gln Lys Ser Leu Ser Leu Ser
        435                 440                 445
Pro Gly Lys
    450

<210> 165
<211> 330
<212> PRT
<213> Artificial Sequence

<220>
<223> Humanised sequence

<400> 165
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu Lys Phe His Tyr Thr
305                 310                 315                 320
Gln Lys Ser Leu Ser Leu Ser Pro Gly Lys
                325                 330


<210> 166
<211> 451
<212> PRT
<213> Artificial Sequence

<220>
<223> Humanised sequence

<400> 166
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45

Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Glu Glu Met Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu Lys Phe His Tyr Thr Gln Lys Ser Leu Ser Leu Ser
        435                 440                 445
Pro Gly Lys
    450

<210> 167
<211> 330
<212> PRT
<213> Artificial Sequence

<220>
<223> Humanised sequence

<400> 167

Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Glu Glu
225                 230                 235                 240
Met Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu Lys Phe His Tyr Thr
305                 310                 315                 320
Gln Lys Ser Leu Ser Leu Ser Pro Gly Lys
                325                 330


<210> 168
<211> 450
<212> PRT
<213> Artificial Sequence

<220>
<223> Humanised sequence

<400> 168
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Pro Val Ala Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Gln Phe Asn Trp Tyr Val Asp Gly Val Glu Val His
        275                 280                 285
Asn Ala Lys Thr Lys Pro Arg Glu Glu Gln Phe Asn Ser Thr Phe Arg
    290                 295                 300
Val Val Ser Val Leu Thr Val Val His Gln Asp Trp Leu Asn Gly Lys
305                 310                 315                 320
Glu Tyr Lys Cys Lys Val Ser Asn Lys Gly Leu Pro Ala Pro Ile Glu
                325                 330                 335
Lys Thr Ile Ser Lys Thr Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Met
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Leu His
            420                 425                 430
Glu Ala Leu His Ser His Tyr Thr Gln Lys Ser Leu Ser Leu Ser Pro
        435                 440                 445
Gly Lys
    450

<210> 169
<211> 329
<212> PRT
<213> Artificial Sequence

<220>
<223> Humanised sequence

<400> 169
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Pro Val Ala Gly Pro Ser Val Phe Leu Phe Pro Pro Lys
        115                 120                 125
Pro Lys Asp Thr Leu Met Ile Ser Arg Thr Pro Glu Val Thr Cys Val
    130                 135                 140
Val Val Asp Val Ser His Glu Asp Pro Glu Val Gln Phe Asn Trp Tyr
145                 150                 155                 160
Val Asp Gly Val Glu Val His Asn Ala Lys Thr Lys Pro Arg Glu Glu
                165                 170                 175
Gln Phe Asn Ser Thr Phe Arg Val Val Ser Val Leu Thr Val Val His
            180                 185                 190
Gln Asp Trp Leu Asn Gly Lys Glu Tyr Lys Cys Lys Val Ser Asn Lys
        195                 200                 205
Gly Leu Pro Ala Pro Ile Glu Lys Thr Ile Ser Lys Thr Lys Gly Gln
    210                 215                 220
Pro Arg Glu Pro Gln Val Tyr Thr Leu Pro Pro Ser Arg Glu Glu Met
225                 230                 235                 240
Thr Lys Asn Gln Val Ser Leu Thr Cys Leu Val Lys Gly Phe Tyr Pro
                245                 250                 255
Ser Asp Ile Ala Val Glu Trp Glu Ser Asn Gly Gln Pro Glu Asn Asn
            260                 265                 270
Tyr Lys Thr Thr Pro Pro Met Leu Asp Ser Asp Gly Ser Phe Phe Leu
        275                 280                 285
Tyr Ser Lys Leu Thr Val Asp Lys Ser Arg Trp Gln Gln Gly Asn Val
    290                 295                 300
Phe Ser Cys Ser Val Leu His Glu Ala Leu His Ser His Tyr Thr Gln
305                 310                 315                 320
Lys Ser Leu Ser Leu Ser Pro Gly Lys
                325

<210> 170
<211> 126
<212> PRT
<213> Artificial Sequence

<220>
<223> Humanised sequence

<400> 170
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Ile Phe Ser Ser Tyr
            20                  25                  30
Ala Met His Trp Val Arg Gln Ala Pro Gly Asn Gly Leu Glu Trp Val
        35                  40                  45
Ala Phe Met Ser Tyr Asp Gly Ser Asn Lys Lys Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Arg Gly Ile Ala Ala Gly Gly Asn Tyr Tyr Tyr Tyr Gly
            100                 105                 110

Met Asp Val Trp Gly Gln Gly Thr Thr Val Thr Val Ser Ser
        115                 120                 125

<210> 171
<211> 110
<212> PRT
<213> Artificial Sequence

<220>
<223> Humanised sequence

<400> 171
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Tyr Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro Pro
                85                  90                  95
Phe Thr Phe Gly Pro Gly Thr Lys Val Asp Ile Lys Arg Thr
            100                 105                 110

<210> 172
<211> 456
<212> PRT
<213> Artificial Sequence

<220>
<223> Humanised sequence

<400> 172
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Ile Phe Ser Ser Tyr
            20                  25                  30
Ala Met His Trp Val Arg Gln Ala Pro Gly Asn Gly Leu Glu Trp Val
        35                  40                  45
Ala Phe Met Ser Tyr Asp Gly Ser Asn Lys Lys Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Arg Gly Ile Ala Ala Gly Gly Asn Tyr Tyr Tyr Tyr Gly
            100                 105                 110
Met Asp Val Trp Gly Gln Gly Thr Thr Val Thr Val Ser Ser Ala Ser
        115                 120                 125
Thr Lys Gly Pro Ser Val Phe Pro Leu Ala Pro Ser Ser Lys Ser Thr
    130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
                165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile

195                 200                 205
Cys Asn Val Asn His Lys Pro Ser Asn Thr Lys Val Asp Lys Lys Val
    210                 215                 220
Glu Pro Lys Ser Cys Asp Lys Thr His Thr Cys Pro Pro Cys Pro Ala
225                 230                 235                 240
Pro Glu Leu Leu Gly Gly Pro Ser Val Phe Leu Phe Pro Pro Lys Pro
                245                 250                 255
Lys Asp Thr Leu Tyr Ile Thr Arg Glu Pro Glu Val Thr Cys Val Val
            260                 265                 270
Val Asp Val Ser His Glu Asp Pro Glu Val Lys Phe Asn Trp Tyr Val
        275                 280                 285
Asp Gly Val Glu Val His Asn Ala Lys Thr Lys Pro Arg Glu Glu Gln
    290                 295                 300
Tyr Asn Ser Thr Tyr Arg Val Val Ser Val Leu Thr Val Leu His Gln
305                 310                 315                 320
Asp Trp Leu Asn Gly Lys Glu Tyr Lys Cys Lys Val Ser Asn Lys Ala
                325                 330                 335
Leu Pro Ala Pro Ile Glu Lys Thr Ile Ser Lys Ala Lys Gly Gln Pro
            340                 345                 350
Arg Glu Pro Gln Val Tyr Thr Leu Pro Pro Ser Arg Asp Glu Leu Thr
        355                 360                 365
Lys Asn Gln Val Ser Leu Thr Cys Leu Val Lys Gly Phe Tyr Pro Ser
    370                 375                 380
Asp Ile Ala Val Glu Trp Glu Ser Asn Gly Gln Pro Glu Asn Asn Tyr
385                 390                 395                 400
Lys Thr Thr Pro Pro Val Leu Asp Ser Asp Gly Ser Phe Phe Leu Tyr
                405                 410                 415
Ser Lys Leu Thr Val Asp Lys Ser Arg Trp Gln Gln Gly Asn Val Phe
            420                 425                 430
Ser Cys Ser Val Met His Glu Ala Leu His Asn His Tyr Thr Gln Lys
        435                 440                 445
Ser Leu Ser Leu Ser Pro Gly Lys
    450                 455

<210> 173
<211> 215
<212> PRT
<213> Artificial Sequence

<220>
<223> Humanised sequence

<400> 173
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Tyr Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro Pro
                85                  90                  95
Phe Thr Phe Gly Pro Gly Thr Lys Val Asp Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser

145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210> 174
<211> 120
<212> PRT
<213> Artificial Sequence

<220>
<223> Humanised sequence

<400> 174
Glu Val Lys Leu Glu Glu Ser Gly Gly Gly Leu Val Gln Pro Gly Gly
1               5                   10                  15
Ser Met Lys Leu Ser Cys Val Ala Ser Gly Phe Ile Phe Ser Asn His
            20                  25                  30
Trp Met Asn Trp Val Arg Gln Ser Pro Glu Lys Gly Leu Glu Trp Val
        35                  40                  45
Ala Glu Ile Arg Ser Lys Ser Ile Asn Ser Ala Thr His Tyr Ala Glu
    50                  55                  60
Ser Val Lys Gly Arg Phe Thr Ile Ser Arg Asp Asp Ser Lys Ser Ala
65                  70                  75                  80
Val Tyr Leu Gln Met Thr Asp Leu Arg Thr Glu Asp Thr Gly Val Tyr
                85                  90                  95
Tyr Cys Ser Arg Asn Tyr Tyr Gly Ser Thr Tyr Asp Tyr Trp Gly Gln
            100                 105                 110
Gly Thr Thr Leu Thr Val Ser Ser
        115                 120

<210> 175
<211> 109
<212> PRT
<213> Artificial Sequence

<220>
<223> Humanised sequence

<400> 175
Asp Ile Leu Leu Thr Gln Ser Pro Ala Ile Leu Ser Val Ser Pro Gly
1               5                   10                  15
Glu Arg Val Ser Phe Ser Cys Arg Ala Ser Gln Phe Val Gly Ser Ser
            20                  25                  30
Ile His Trp Tyr Gln Gln Arg Thr Asn Gly Ser Pro Arg Leu Leu Ile
        35                  40                  45
Lys Tyr Ala Ser Glu Ser Met Ser Gly Ile Pro Ser Arg Phe Ser Gly
    50                  55                  60
Ser Gly Ser Gly Thr Asp Phe Thr Leu Ser Ile Asn Thr Val Glu Ser
65                  70                  75                  80
Glu Asp Ile Ala Asp Tyr Tyr Cys Gln Gln Ser His Ser Trp Pro Phe
                85                  90                  95
Thr Phe Gly Ser Gly Thr Asn Leu Glu Val Lys Arg Thr
            100                 105

<210> 176
<211> 450
<212> PRT
<213> Artificial Sequence

<220>
<223> Humanised sequence

<400> 176
Glu Val Lys Leu Glu Glu Ser Gly Gly Gly Leu Val Gln Pro Gly Gly
1               5                   10                  15
Ser Met Lys Leu Ser Cys Val Ala Ser Gly Phe Ile Phe Ser Asn His
            20                  25                  30
Trp Met Asn Trp Val Arg Gln Ser Pro Glu Lys Gly Leu Glu Trp Val
        35                  40                  45
Ala Glu Ile Arg Ser Lys Ser Ile Asn Ser Ala Thr His Tyr Ala Glu
    50                  55                  60
Ser Val Lys Gly Arg Phe Thr Ile Ser Arg Asp Asp Ser Lys Ser Ala
65                  70                  75                  80
Val Tyr Leu Gln Met Thr Asp Leu Arg Thr Glu Asp Thr Gly Val Tyr
                85                  90                  95
Tyr Cys Ser Arg Asn Tyr Tyr Gly Ser Thr Tyr Asp Tyr Trp Gly Gln
            100                 105                 110
Gly Thr Thr Leu Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Tyr Ile
                245                 250                 255
Thr Arg Glu Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445
Gly Lys
    450

<210> 177
<211> 214
<212> PRT
<213> Artificial Sequence

<220>
<223> Humanised sequence

<400> 177
Asp Ile Leu Leu Thr Gln Ser Pro Ala Ile Leu Ser Val Ser Pro Gly
1               5                   10                  15
Glu Arg Val Ser Phe Ser Cys Arg Ala Ser Gln Phe Val Gly Ser Ser
            20                  25                  30
Ile His Trp Tyr Gln Gln Arg Thr Asn Gly Ser Pro Arg Leu Leu Ile
        35                  40                  45
Lys Tyr Ala Ser Glu Ser Met Ser Gly Ile Pro Ser Arg Phe Ser Gly
    50                  55                  60
Ser Gly Ser Gly Thr Asp Phe Thr Leu Ser Ile Asn Thr Val Glu Ser
65                  70                  75                  80
Glu Asp Ile Ala Asp Tyr Tyr Cys Gln Gln Ser His Ser Trp Pro Phe
                85                  90                  95
Thr Phe Gly Ser Gly Thr Asn Leu Glu Val Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 178
<211> 118
<212> PRT
<213> Artificial Sequence

<220>
<223> Humanised sequence

<400> 178
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Val Phe Thr Asp Tyr
            20                  25                  30
Gly Met Asn Trp Val Arg Gln Ala Pro Gly Lys Gly Leu Glu Trp Met
        35                  40                  45
Gly Trp Ile Asn Thr Tyr Ile Gly Glu Pro Ile Tyr Ala Asp Ser Val
    50                  55                  60
Lys Gly Arg Phe Thr Phe Ser Leu Asp Thr Ser Lys Ser Thr Ala Tyr

65                  70                  75                  80
Leu Gln Met Asn Ser Leu Arg Ala Glu Asp Thr Ala Val Tyr Tyr Cys
                85                  90                  95
Ala Arg Gly Tyr Arg Ser Tyr Ala Met Asp Tyr Trp Gly Gln Gly Thr
            100                 105                 110
Leu Val Thr Val Ser Ser
        115



<210> 179
<211> 109
<212> PRT
<213> Artificial Sequence

<220>
<223> Humanised sequence

<400> 179
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asn Val Gly Thr Asn
            20                  25                  30
Val Ala Trp Tyr Gln Gln Lys Pro Gly Lys Ala Pro Lys Ala Leu Ile
        35                  40                  45
Tyr Ser Ala Ser Phe Leu Tyr Ser Gly Val Pro Tyr Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ile Tyr Pro Leu
                85                  90                  95
Thr Phe Gly Gln Gly Thr Lys Val Glu Ile Lys Arg Thr
            100                 105



<210> 180
<211> 229
<212> PRT
<213> Artificial Sequence

<220>
<223> Humanised sequence

<400> 180
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Val Phe Thr Asp Tyr
            20                  25                  30
Gly Met Asn Trp Val Arg Gln Ala Pro Gly Lys Gly Leu Glu Trp Met
        35                  40                  45
Gly Trp Ile Asn Thr Tyr Ile Gly Glu Pro Ile Tyr Ala Asp Ser Val
    50                  55                  60
Lys Gly Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Arg Gly Tyr Arg Ser Tyr Ala Met Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Ala Ala
225

## Description

### Field

The invention relates to novel variants of anti-TNF antibodies.

### Background

Antibodies are heteromultimeric glycoproteins comprising at least two heavy and two light chains. Aside from IgM, intact antibodies are usually heterotetrameric glycoproteins of approximately 150Kda, composed of two identical light (L) chains and two identical heavy (H) chains. Each heavy chain has at one end a variable domain (VH) followed by a number of constant regions. Each light chain has a variable domain (VL) and a constant region at its other end; the constant region of the light chain is aligned with the first constant region of the heavy chain and the light chain variable domain is aligned with the variable domain of the heavy chain. Depending on the amino acid sequence of the constant region of their heavy chains, human antibodies can be assigned to five different classes, IgA, IgD, IgE, IgG and IgM. IgG and IgA can be further subdivided into subclasses, IgG1, IgG2, IgG3 and IgG4; and IgA1 and IgA2. The variable domain of the antibody confers binding specificity upon the antibody with certain regions displaying particular variability called complementarity determining regions (CDRs). The more conserved portions of the variable region are called Framework regions (FR). The variable domains of intact heavy and light chains each comprise four FR connected by three CDRs. The constant regions are not directly involved in the binding of the antibody to the antigen but exhibit various effector functions such as participation in antibody dependent cell-mediated cytotoxicity (ADCC), phagocytosis via binding to Fcγ receptor, half-life/clearance rate via neonatal Fc receptor (FcRn) and complement dependent cytotoxicity via the C1q component of the complement cascade. The nature of the structure of an IgG antibody is such that there are two antigen-binding sites, both of which are specific for the same epitope. They are therefore, monospecific.

In adult mammals, FcRn, also known as the neonatal Fc receptor, plays a key role in maintaining serum antibody levels by acting as a protective receptor that binds and salvages antibodies of the IgG isotype from degradation. IgG molecules are endocytosed by endothelial cells, and if they bind to FcRn, are recycled out into circulation. In contrast, IgG molecules that do not bind to FcRn enter the cells and are targeted to the lysosomal pathway where they are degraded.

The neonatal FcRn receptor is believed to be involved in both antibody clearance and the transcytosis across tissues (see Junghans R.P (1997) Immunol.Res 16. 29-57 and Ghetie et al (2000) Annu.Rev.lmmunol. 18, 739-766).

WO 9734631 discloses a composition comprising a mutant IgG molecule having increased serum half-life and at least one amino acid substitution in the Fc-hinge region. Amino acid substitution at one or more of the amino acids selected from number 252, 254, 256, 309, 311 or 315 in the CH2 domain or 433 or 434 in the CH3 domain is disclosed.

WO 00/42072 discloses a polypeptide comprising a variant Fc region with altered FcRn binding affinity, which polypeptide comprises an amino acid modification at any one or more of amino acid positions 238, 252, 253, 254, 255, 256, 265, 272, 286, 288, 303, 305, 307, 309, 311, 312, 317, 340, 356, 360, 362, 376, 378, 380, 386,388, 400, 413, 415, 424,433, 434,435, 436, 439, and 447 of the Fc region.

WO 02/060919 discloses a modified IgG comprising an IgG constant domain comprising amino acid modifications at one or more of positions 251, 253, 255, 285-290, 308-314, 385-389, and 428-435. WO 2004035752 discloses a modified antibody of class IgG wherein at least one amino acid residue from the heavy chain constant region selected from the group consisting of amino acid residues 250, 314, and 428 is different from that present in an unmodified class IgG antibody.

Shields et al. (2001, J Biol Chem ; 276:6591-604) used alanine scanning mutagenesis to alter residues in the Fc region of a human IgG1 antibody and then assessed the binding to human FcRn. Positions that effectively abrogated binding to FcRn when changed to alanine include I253, S254, H435, and Y436. Other positions showed a less pronounced reduction in binding as follows: E233-G236, R255, K288, L309, S415, and H433. Several amino acid positions exhibited an improvement in FcRn binding when changed to alanine.

Dall'Acqua et al. (2002, J Immunol.;169:5171-80) described random mutagenesis and screening of human IgG1 hinge-Fc fragment phage display libraries against mouse FcRn. They disclosed random mutagenesis of positions 251, 252, 254-256, 308, 309, 311, 312, 314, 385-387, 389, 428, 433, 434, and 436.

WO2006130834 discloses modified IgG comprising an IgG comprising an IgG constant domain comprising amino acid modifications at one or more positions of 252, 254, 256, 433, 434 and 436. Therefore, modification of Fc domains of IgG antibodies has been discussed as a means of increasing the serum half- life of therapeutic antibodies. However, numerous such modifications have been suggested with varying and sometimes contradictory results in different antibodies.

The administration of antigen binding proteins as therapeutics requires injections with a prescribed frequency relating to the clearance and half-life characteristics of the protein.

Adalimumab is a monoclonal antibody against TNF-alpha which is used for treatment of rheumatoid arthritis, psoriatic arthritis, ankylosing spondylitis, and Crohn's disease. It is produced by recombinant DNA technology using a mammalian cell expression system. It consists of 330 amino acids and has a molecular weight of approximately 148 kilodaltons. See United States Patent 6090382. At doses of 0.5 mg/kg (-40 mg), clearance for adalimumab is said to range from 11 to 15 ml/hour, the distribution volume (Vₛₛ) ranges from 5 to 6 litres and the mean terminal phase half-life was approximately two weeks (Summary of Product Characteristics available from www.medicines.org.uk). These half life and clearance properties mean that currently adalimumab needs to be administered once every two weeks. In some patients depending on disease it may be necessary to administer a loading dose such as for example in psoriasis patients. This dosage may differ from the maintenance dose.

### Summary of invention

In one aspect, the invention relates to an antigen binding protein which specifically binds to TNF-alpha comprising CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32) or variants thereof wherein said variants may contain 1, 2, 3 or 4 amino acid substitutions, insertions or deletions as compared to CDRH1, CDRH2, CDRH3, CDRL1, CDRL2, or CDRL3; and a neonatal Fc receptor (FcRn) binding portion of a human IgG1 constant domain comprising one of more amino acid substitutions relative to the human IgG1 constant domain, wherein the antigen binding protein has an increased FcRn binding affinity at pH 6 and/ or increased half-life as compared to an IgG comprising the light chain sequence of SEQ ID No. 2 and the heavy chain sequence of SEQ ID No.12.

Throughout the specification the term "human IgG1 constant domain" encompasses all allotypes and variants thereof known to a person skilled in the art.

In one aspect, the invention relates to an antigen binding protein which specifically binds to TNF-alpha comprising CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); or variants thereof wherein said variants may contain 1, 2, 3 or 4 amino acid substitutions, insertions or deletions as compared to CDRH1, CDRH2, CDRH3, CDRL1, CDRL2, or CDRL3; and a neonatal Fc receptor (FcRn) binding portion of a human IgG1 constant domain comprising one of more amino acid substitutions relative to the human IgG1 constant domain, wherein the antigen binding protein has an increased half life as compared to an IgG comprising the light chain sequence of SEQ ID No. 2 and heavy chain sequence of SEQ ID No.12 and the antigen binding protein can be administered no more than once every four weeks to achieve comparable mean steady-state trough concentration as that achieved by the same dose of IgG comprising the light chain sequence of SEQ ID No. 2 and the heavy chain sequence of SEQ ID No. 12 administered once every two weeks.

In one aspect, the invention relates to an antigen binding protein which specifically binds to TNF-alpha comprising CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32) or variants thereof wherein said variants may contain 1, 2, 3 or 4 amino acid substitutions, insertions or deletions as compared to CDRH1, CDRH2, CDRH3, CDRL1, CDRL2, or CDRL3; and an FcRn binding portion of a human IgG1 constant domain comprising one of more amino acid substitutions relative to the human IgG1 constant domain, wherein the antigen binding protein has an affinity for FcRn of 2 fold, or 3 fold, or 4 fold or 5 fold, or 6 fold or 8 fold or greater than an anti-TNF antigen binding protein with the same CDR's without such modifications at pH 6 as assessed by ProteOn XPR36 protein interaction array system at 25°C, the array system having antigen binding proteins immobilised on the chip.

In one aspect, the invention relates to an antigen binding protein which is a variant of an IgG comprising the light chain sequence of SEQ ID No. 2 and the heavy chain sequence of SEQ ID No.12, wherein the antigen binding protein variant comprises one or more substitutions in the neonatal Fc receptor (FcRn) binding portion of the IgG constant domain to increase the half-life of the antigen binding protein variant compared with the IgG without such substitutions , wherein when the variant is administered to patients at a single dose of 40 mg at a four to eight weekly interval, the mean steady-state trough concentration in the patient population does not fall below 4µg/ml or does not fall below 5 µg /ml between dosing intervals. Preferably, the mean serum trough antibody concentration in the patient population does not fall below 6 µg /ml between dosing intervals. Preferably, the mean serum trough antibody concentration in the patient population does not fall below 5 µg /ml between dosing intervals when the variant is administered to patients at a single dose of 40 mg at an eight weekly interval..Preferably, the mean serum trough antibody concentration in the patient population does not fall below 4 µg /ml between dosing intervals whilst still providing the optimal efficacy when the variant is administered to patients at a single dose of 40 mg at an eight weekly interval. Preferably, the mean serum trough antibody concentration in the patient population does not fall below 3 µg /ml between dosing intervals whilst still providing the optimal efficacy when the variant is administered to patients at a single dose of 40 mg at an eight weekly interval.

In one aspect, the invention relates to an antigen binding protein as disclosed herein for treatment of a disease wherein the antigen binding protein can be administered to patients no more than once every four weeks to achieve comparable mean steady-state trough concentration as that achieved by the same dose of an IgG comprising light chain sequence of SEQ ID No. 2 and heavy chain sequence of SEQ ID No. 12 administered once every two weeks.

In one aspect, the invention relates to a method of treating a patient with a disease, the method comprising administering an antigen binding protein according to the invention.

In one aspect, the invention relates to a nucleic acid sequence encoding the antigen binding protein according to the invention, or a part thereof such as a heavy or light chain. In one aspect, the invention relates to an expression vector encoding the antigen binding protein according to the invention, or a part thereof such as a heavy or light chain.

In one aspect, the invention relates to a host cell comprising the nucleic acid sequence encoding the antigen binding protein according to the invention. In one aspect, the invention relates to an antigen binding protein according to the invention for use in the treatment of Psoriasis or rheumatoid arthritis.

In one aspect, the invention relates to a kit comprising the antigen binding protein according to the invention, and optionally comprising methotrexate for concomitant delivery of antigen binding protein according to the invention and methotrexate.

In one aspect, the invention relates to an antigen binding protein as disclosed herein for treatment of Rheumatoid arthritis in an individual who is already being treated with methotrexate, and to an antigen binding protein in combination with methotrexate for treatment of Rheumatoid arthritis, wherein the combination is delivered simultaneously, substantially simultaneously, or sequentially.

In one aspect, the invention relates to an antigen binding protein as disclosed herein for treatment of Psoriasis in an individual who is already being treated with methotrexate, and to an antigen binding protein in combination with methotrexate for treatment of Psoriasis, wherein the combination is delivered simultaneously, substantially simultaneously, or sequentially.

### Brief Description of Figures

**Figure 1** **-** Binding of anti-TNFα antibodies to human TNFα
**Figure** 2 - Analysis of binding activity of anti-TNFα antibodies to human TNFα following an accelerated stressor study
**Figure 3** **-** Binding of anti-TNFα antibodies to human TNFα following incubation in 25% human serum for 2 weeks
**Figure 4** **-** Binding of anti-TNFα antibodies to human TNFα following freeze-thaw
**Figure 5** **-** Analysis of anti-TNFα antibodies to FcγRIIIa receptors **(a)** Binding to human FcγRIIIa (valine 158 variant) **(b)** Binding to human FcγRIIIA (phenylalanine 158 variant)
**Figure 6** **-** Average dose normalised plasma concentrations of BPC2604 in female cynomolgus monkeys and pascolizumab in male cynomolgus monkeys following a single intravenous (1 hr infusion)

### Detailed Description of Invention

The invention relates to novel antigen binding proteins binding specifically to TNF-alpha. In particular, the invention relates to novel variants of anti-TNF antibodies such as adalimumab which show increased binding to the FcRn receptor and/ or increased half life as compared to adalimumab. Adalimumab is an IgG monoclonal antibody comprising the light chain sequence of SEQ ID No. 2 and heavy chain sequence of SEQ ID No.12.

The inventors have found that specific modifications to adalimumab as described herein show particular improvements in FcRn binding as shown in the examples below. Affinity matured variants of adalimumab also show improvement in anti-TNF-alpha binding and/or neutralisation activity.

The novel antigen binding proteins of the invention have an increased binding to the FcRn receptor and/ or increased half life and/ or increased Mean Residence Time and/ or decreased Clearance. It is considered that binding to FcRn results in longer serum retention *in vivo.* In order to increase the retention of the Fc proteins *in vivo,* the increase in binding affinity is observed around pH 6. In one aspect, the present invention therefore provides an antigen binding protein with optimised binding to FcRn.

In one embodiment, the half-life of the antigen binding protein of the present invention is increased 2 to 6 fold, such as 2 fold, 3 fold, 4 fold, 5 fold or 6 fold as compared to an IgG comprising the light chain sequence of SEQ ID No. 2 and heavy chain sequence of SEQ ID No.12. Preferably, the half-life of the antigen binding protein of the invention is increased 3 fold, 4 fold, or more compared to an IgG comprising the light chain sequence of SEQ ID No. 2 and heavy chain sequence of SEQ ID No.12. For example, if the IgG is adalimumab having a half life of 10 days or in the range of 10 to 20 days then in one embodiment an antigen binding protein of the present invention shows a half life of about 40 to 80 days. For example an antigen binding protein comprising a heavy chain sequence selected from SEQ ID NO:5 or SEQ ID NO:9 or SEQ ID NO:15 or SEQ ID NO:18. or SEQ ID NO:21. or SEQ ID NO:24 or SEQ ID NO:163, or SEQ ID NO:165, or SEQ ID NO:167, or SEQ ID NO:169.

In one embodiment, the antigen binding protein of the invention administered no more than once every four weeks in patients, achieves mean steady-state trough concentrations between about 2 µg/ml to about 7 µg/ml. Preferably, the mean steady-state trough concentrations are between about 4 µg/ml to about 7 µg/ml and more preferably between about 5 µg/ml to about 6 µg/ml.

In one embodiment, the antigen binding protein of the invention administered no more than once every 28 days in patients, achieves mean steady-state trough concentrations between about 2 µg/ml to about 7 µg/ml. Preferably, the mean steady-state trough concentrations are between about 4 µg/ml to about 7 µg/ml and more preferably between about 5 µg/ml to about 6 µg/ml.

In one embodiment of the invention, the antigen binding protein of the invention can be administered once every 4, 5, 6, 7 or 8 weeks to achieve comparable mean steady-state trough concentrations as those achieved by adalimumab, when administered once every two weeks at the same dose.

In a preferred embodiment of all aspects of the invention, the antigen binding protein of the invention can be administered once every 7 or 8 weeks.

In one embodiment of the invention, the antigen binding protein of the invention can be administered once every 25-80 days for example once every 40-60 days, or for example once every 28, 35, 42, 49 or 56 days to achieve comparable mean steady-state trough concentrations as those achieved by adalimumab, when administered once every 14 days at the same dose.

In one embodiment of the invention, the antigen binding protein can be administered once every 49 to 60 day, for example every 56 days.

In an embodiment of all aspects of the invention, the antigen binding protein has a 2 fold, or 4 fold, or 6 fold, or 8 fold or greater affinity for human FcRn at pH 6 as assessed by ProteOn XPR36 protein interaction array system at 25°C wherein the antibodies are immobilised on the chip. Preferably, the antigen binding protein has an affinity for human FcRn between about 100 to about 500 KD(nM), such as between about 130 to about 360 KD(nM) or between about 140 to about 250KD(nM) or between about 140 to about 210KD(nM).

In one embodiment, the clearance of the antigen binding protein is about 2 to about 10 ml/hr, preferably about 2 to about 5ml/hr or 2 to 4ml/hr or 2 to 3ml/hr, such as about 2, about 2.5, 3, 4 or 5 ml/hr. In one embodiment the antigen binding protein of the invention shows a clearance rate which is 2 fold, 3 fold, 4 fold or 5 fold lower than adalimumab. In one embodiment, clearance for an antigen binding protein according to the invention is in the ranges specified above or 2 fold, 3 fold, 4 fold or 5 fold lower than adalimumab at a human dose of about 40 mg.

In one aspect, the antigen binding protein of the invention is a variant of adalimumab (IgG comprising the light chain sequence of SEQ ID No. 2 and the heavy chain sequence of SEQ ID No.12), the variant comprising one or more substitutions in the FcRn binding portion of the IgG constant domain to increase the half-life of the variant compared with adalimumab, wherein when the variant is administered to patients at a single dose of 40 mg at a four to eight weekly interval, preferably eight weekly interval, the mean steady-state trough antibody concentration in the patient population does not fall below 5 µg /ml. In one embodiment the mean steady-state trough antibody concentration in the patient population does not fall below 6 µg /ml, between dosing intervals.

In a further embodiment, the antigen binding protein comprises at least one amino acid modification in the Fc region of said antigen binding protein, wherein said modification is at one or more of positions 250, 252, 254, 256, 257, 259, 308, 428 or 434 of the Fc region as compared to same position in the adalimumab sequence, wherein the numbering of the amino acids in the Fc region is that of the EU index in Kabat.

The wild type human IgG1 has amino acid residues Val-Leu-His-Gln-Asp-Trp-Leu at positions 308-314, amino acid residues Leu-Met- Ile-Ser-Arg-Thr at positions 251-256, amino acid residues Met-His-Glu-Ala-Leu-His-Asn-HisTyr at positions 428-436, and amino acid residues Gly-Gln-Pro-Glu-Asn at positions 385-389. Residue numbering may differ for IgG2-4.

In one embodiment, the antigen binding protein of the invention comprises one or more amino acid substitution relative to the human IgG1 constant domain comprising the sequence of SEQ ID No. 13.

In one embodiment, the one or more amino acid substitution in the FcRn binding portion of the human IgG1 heavy chain constant domain is at amino acid residues 252, 254 and 256 numbered according to EU index of Kabat and the aa substitution at residue 252 is a substitution of met with tyr, phe, tryp or thr; the aa substitution at residue 254 is a substitution of ser with thr; and the aa substitution at residue 256 is a substitution of thr with ser, arg, glu, asp or thr. Preferably, the aa substitution at residue 252 is a substitution with tyr; the aa substitution at residue 254 is a substitution with thr and the substitution at residue 256 is a substitution with glu. Preferably, the IgG1 constant domain is as shown in SEQ ID No: 7.

In one embodiment, the one or more amino acid substitutions in the FcRn binding portion of the human IgG1 constant domain is at amino acid residues 250 and 428 numbered according to EU index of Kabat and the aa substitution at residue 250 is a substitution of thr with glu or gin; the aa substitution at residue 428 is a substitution of met with leu or phe. Preferably, the aa substitution at residue 250 is a substitution with glu and the aa substitution at residue 428 is a substitution with leu. Preferably, the IgG1 constant domain is as shown in SEQ ID No: 16.

In one embodiment, the one or more amino acid substitution in the FcRn binding portion of the human IgG1 constant domain is at amino acid residues 428 and/ or 434 numbered according to EU index of Kabat. Preferably, the aa substitution at residue 428 is a substitution of met with leu and the aa substitution at residue 434 is a substitution of asn with ser. Preferably, the IgG1 constant domain is as shown in SEQ ID No: 10.

In one embodiment, the one or more amino acid substitution in the FcRn binding portion of the human IgG1 constant domain is at amino acid residues 259 or 308 numbered according to EU index of Kabat. Preferably, the substitution at residue 259 is a substitution of val with ile and the aa substitution at residue 308 is a substitution of val with phe. Preferably, the IgG1 constant domain is as shown in SEQ ID No: 19 or SEQ ID No: 22.

In one embodiment, the one or more amino acid substitution in the FcRn binding portion of the human IgG1 heavy chain constant domain is at amino acid residues 257 and 434 numbered according to EU index of Kabat as shown in SEQ ID No: 25.

In one embodiment, the one or more amino acid substitution in the FcRn binding portion of the human IgG1 heavy chain constant domain is at amino acid residues 433 and 434 numbered according to EU index of Kabat for example the residues are H433K and N434F Preferably, the IgG1 constant domain is as shown in SEQ ID No: 165 or SEQ ID No: 167.

In one embodiment, the antigen binding protein comprises any of the IgG1 constant domain modifications listed in Table A.

In one embodiment, the antigen binding protein is an antibody.

In one embodiment, the antigen binding protein comprises a variable domain of SEQ ID NO: 6 and/or SEQ ID NO: 3 or a variant thereof which contains 1, 2, 3, 4, 5, 6, 7, 8, 9 or 10 amino acid substitutions, insertions or deletions and/or shares at least 90% identity across the length of SEQ ID NO: 6 or SEQ ID NO: 3.

In one embodiment, the antigen binding protein comprises the heavy chain sequence as shown in SEQ ID No 5, 9 or 15 optionally with a light chain sequence as shown in SEQ ID No: 2.

In one embodiment, the antigen binding protein comprises a variable heavy domain sequence as shown in SEQ ID NO: 78 or 80.

In one embodiment, the antigen binding protein comprises a heavy chain sequence as shown in SEQ ID NO: 145 or SEQ ID NO: 146 optionally with a light chain variant as shown in SEQ ID Nos. 148, 150 or 152.

In one embodiment, the antigen binding protein comprises the heavy chain sequence as as shown in SEQ ID No 18 or 21 optionally with a light chain sequence as shown in SEQ ID No: 2.

In one embodiment the antigen binding protein according to the invention comprises any of the variable domains specified in Table A. In one embodiment, the antigen binding protein according to the invention comprises the variable heavy domain having the sequence of cb1-3-VH, cb2-44-VH, cb1-39-VH, cb1-31-VH, cb2-11-VH, cb2-40-VH, cb2-35-VH, cb2-28-VH, cb2-38-VH, cb2-20-VH, cb1-8-VL or cb1-43-VL as shown in Table A.

In one embodiment, the antigen binding protein according to the invention comprises the variable light domain having the sequence of cb1-45-VL, cb1-4-VL, cb1-41-VL, cb1-37-VL, cb1-39-VL, cb1-33-VL, cb1-35-VL, cb1-31-VL, cb1-29-VL, cb1-22-VL, cb1-23-VL, cb1-12-VL, cb1-10-VL, cb2-1-VL, cb2-11-VL, cb2-40-VL, cb2-35-VL, cb2-28-VL, cb2-20-VL, cb1-3-VL, cb2-6-VL or cb2-44-VL as shown in Table A.

For example, the antigen binding protein according to the invention comprises a variable domain having the sequence of cb1-3VH, cb2-44VH or cb2-6VL as shown in Table A.

In one embodiment the antigen binding protein according to the invention comprises any of the variable domains specified in Table A. In one embodiment, the antigen binding protein according to the invention comprises the variable heavy domain having a sequence selected from SEQ ID NO: 170 or SEQ ID NO: 174 or SEQ ID NO:178

In one embodiment, the antigen binding protein according to the invention comprises the variable light domain having a sequence selected from SEQ ID NO: 171 or SEQ ID NO: 175 or SEQ ID NO:179

In a further embodiment the antigen binding protein comprises any of the IgG1 constant domain modifications listed in Table A.

Variants of all the above mentioned variable domains or heavy chain sequences or light chain sequences which contain 1, 2, 3, 4, 5, 6, 7, 8, 9 or 10 amino acid substitutions, insertions or deletions and/or share at least 90% identity across the length of any of these sequences are also within the scope of the invention.

In one embodiment, the antigen binding protein of the invention comprises a variant of CDRH3 (SEQ ID No: 29) which variant has 1, 2, 3 or 4 amino acid substitutions as compared to SEQ ID No: 29. In one embodiment, the variant CDRH3 may have the sequence as shown in any one of SEQ ID Nos. 40 to 49.

In one embodiment, the antigen binding protein of the invention comprises a variant of CDRH1 (SEQ ID No: 27) which variant has 1 or 2 amino acid substitutions as compared to SEQ ID No: 27. In one embodiment, the variant CDRH1 may have the sequence as shown in any one of SEQ ID Nos. 33 to 38.

In one embodiment, the antigen binding protein of the invention comprises a variant of CDRL1 (SEQ ID No: 30) which variant has 1, 2 or 3 amino acid substitutions as compared to SEQ ID No: 30. In one embodiment, the variant CDRL1 may have the sequence as shown in any one of SEQ ID Nos. 50 to 61.

In one embodiment, the antigen binding protein of the invention comprises a variant of CDRL2 (SEQ ID No: 31) which variant has 1, 2 or 3 amino acid substitutions as compared to SEQ ID No: 31. In one embodiment, the variant CDRL2 may have the sequence as shown in any one of SEQ ID Nos. 62 to 72.

In one embodiment, the antigen binding protein of the invention comprises a variant of CDRL3 (SEQ ID No: 32) which variant has 1, 2 or 3 amino acid substitutions as compared to SEQ ID No: 32. In one embodiment, the variant CDRL3 may have the sequence as shown in any one of SEQ ID Nos. 73 to 76.

In one embodiment, the invention relates to an antigen binding protein which specifically binds to TNF-alpha comprising one or more or all CDRs selected from: CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); wherein any of the CDRs could be a variant CDR which contains 1, 2, 3 or 4 amino acid substitutions, insertions or deletions as compared to CDRH1, CDRH2, CDRH3, CDRL1, CDRL2, or CDRL3. In one aspect, the antigen binding protein of the invention comprises CDRH1, CDRH3, CDRL1, CDRL2 and CDRL3 wherein any of the CDRs could be a variant CDR which contains 1, 2, 3 or 4 amino acid substitutions, insertions or deletions compared to CDRH1, CDRH3, CDRL1, CDRL2, or CDRL3. In one aspect, the antigen binding protein of the invention comprises CDRH1, CDRH2, CDRH3, CDRL1, CDRL2 and CDRL3 wherein any of the CDRs could be a variant CDR which contains 1, 2, 3 or 4 amino acid substitutions, insertions or deletions compared to CDRH1, CDRH2, CDRH3, CDRL1, CDRL2, or CDRL3

In one aspect, the invention relates to a method of treating a human patient with a disease, the method comprising administering an antigen binding protein according to the invention.

The invention also relates to an antigen binding protein as disclosed herein for the treatment of disease in a human.

The invention also relates to use of an antigen binding protein as disclosed herein in the manufacture of a medicament for the treatment of disease, and an antigen binding protein as disclosed herein for use in treatment of disease.

In one embodiment, the disease to be treated by the antigen binding protein of the invention is rheumatoid arthritis, polyarticular juvenile idiopathic arthritis, psoriatic arthritis, ankylosing spondylitis, Ulcerative colitis, spondyloarthropathy, Crohn's disease or Psoriasis.

In one embodiment, the antigen binding protein of the invention is to be administered with methotrexate. The methotrexate can be delivered before, after or at the same time, or substantially the same time, as the antigen binding protein. In a preferred embodiment the antigen binding protein of the invention is to be administered with methotrexate to a patient suffering from rheumatoid arthritis. In one embodiment, methotrexate is administered to patients receiving an antigen binding protein of the invention to reduce the immunogenic effect of the antigen binding protein. In one embodiment, the antigen binding protein of the invention is administered to patients already receiving methotrexate. Methotrexate may be substituted by another acceptable compound which reduced the immune response to the antigen binding protein, for example corticosteroids.

In one aspect, the invention relates to a method of treating a patient with a disease, the method comprising administering an antigen binding protein of the invention. In one embodiment, the method comprises administering an antigen binding protein to the patient as a single 20, 30, 35, 40, 45, 50, 55, 60, 65, 70, 75 or 80 mg dose no more than once every four weeks, preferably once every 5, 6, 7, or 8 weeks and most preferably once every 8 weeks. Preferably, the dose is 40 to 80 mg, for example 40mg.

The invention also provides a polynucleotide sequence encoding any amino acid sequence disclosed herein, including a heavy chain of any of the antigen binding constructs described herein, and a polynucleotide encoding a light chain of any of the antigen binding constructs described herein. Such polynucleotides represent the coding sequence which corresponds to the equivalent polypeptide sequences, however it will be understood that such polynucleotide sequences could be cloned into an expression vector along with a start codon, an appropriate signal sequence and a stop codon. The polynucleotide may be DNA or RNA.

The invention also provides a host cell, for example a recombinant, transformed or transfected cell, comprising one or more polynucleotides encoding a heavy chain and/or a light chain of any of the antigen binding constructs described herein.

The invention further provides a pharmaceutical composition comprising an antigen binding construct as described herein a pharmaceutically acceptable carrier.

The invention further provides a method for the production of any of the antigen binding constructs described herein which method comprises the step of culturing a host cell comprising a first and second vector, said first vector comprising a polynucleotide encoding a heavy chain of any of the antigen binding constructs described herein and said second vector comprising a polynucleotide encoding a light chain of any of the antigen binding constructs described herein, in a serum- free / chemically defined / animal derived component free culture media. Alternatively a method may comprise culturing a host cell comprising a vector comprising a polynucleotide encoding a heavy chain of any of the antigen binding constructs described herein and a polynucleotide encoding a light chain of any of the antigen binding constructs described herein, suitably in a serum- free / chemically defined / animal derived component free culture media.

In another embodiment, the invention includes a method of increasing the half-life of an antibody by modifying an Fc according to the modifications described herein.

In another embodiment, the invention includes an antigen binding protein as described herein with enhanced FcRn binding and having one or more additional substitutions, deletions or insertions that modulate another property of the effector function.

Once expressed by the desired method, the antigen binding protein of the invention is then examined for in vitro activity by use of an appropriate assay. Presently conventional ELISA and Biacore assay formats are employed to assess qualitative and quantitative binding of the antigen binding construct to its target. Additionally, other in vitro assays may also be used to verify neutralizing efficacy prior to subsequent human clinical studies performed to evaluate the persistence of the antigen binding protein in the body despite the usual clearance mechanisms.

The dose and duration of treatment relates to the relative duration of the molecules of the present invention in the human circulation, and can be adjusted by one of skill in the art depending upon the condition being treated and the general health of the patient based on the information provided herein. It is envisaged that repeated dosing (e.g. once every 4 weeks, 5 weeks, 6 weeks, 7 weeks or 8 weeks) over an extended time period (e.g. four to six months) maybe required to achieve maximal therapeutic efficacy.

The mode of administration of the therapeutic agent of the invention may be any suitable route which delivers the agent to the host. The antigen binding proteins, and pharmaceutical compositions of the invention are particularly useful for parenteral administration, i.e., subcutaneously (s.c.), intrathecally, intraperitoneally, intramuscularly (i.m.), intravenously (i.v.), or intranasally. In one embodiment the antigen binding proteins and pharmaceutical compositions of the invention are administered via a subcutaneous auto injector pen or a subcutaneous pre-filled syringe.

Antigen binding proteins of the invention may be prepared as pharmaceutical compositions containing an effective amount of the antigen binding protein of the invention as an active ingredient in a pharmaceutically acceptable carrier. In the prophylactic agent of the invention, an aqueous suspension or solution containing the antigen binding construct, preferably buffered at physiological pH, in a form ready for injection is preferred. The compositions for parenteral administration will commonly comprise a solution of the antigen binding construct of the invention or a cocktail thereof dissolved in a pharmaceutically acceptable carrier, preferably an aqueous carrier. A variety of aqueous carriers may be employed, e.g., 0.9% saline, 0.3% glycine, and the like. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen binding protein of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

It has been reported that adalimumab is difficult to formulate at high concentrations. WO2004016286 describes an adalimumab formulation comprising a citrate-phosphate buffer and other components including a polyol and a detergent. The oral presentation "Humira® - from Development to Commercial Scale Production" presented on 25 October 2005 at the PDA Conference reports formulations comprising (i) citrate-phosphate buffer; (ii) acetate-phosphate buffer; and (iii) phosphate buffer. The acetate-phosphate buffer tested displayed the worst stabilising effect upon adalimumab. Curtis et al. (2008) Current Medical Research and Opinion, Volume 27, p71-78, report the incidence of injection-site burning and stinging in patients with rheumatoid arthritis using injectable adalimumab. The burning and stinging has been partly attributed to citrate buffer-based formulations (Basic and Clinical Pharmacology & Toxicology, Volume 98, p218-221, 2006; and Journal of Pharmaceutical Sciences, Volume 97, p3051-3066, 2008). However, WO20100129469 describes a high adalimumab concentration formulation that still comprises a citrate-phosphate buffer and other components including a polyol with no sodium chloride. The more recent WO2012065072 describes an adalimumab formulation comprising a surfactant and a polyol with no buffer, thus potentially avoiding any citrate buffer effects upon injection.

In one embodiment there is provided a liquid formulation comprising a TNF-alpha antigen binding protein and an acetate buffer. In a further embodiment the TNF-alpha binding protein comprises a CDRH1 selected from SEQ ID NO:27 or SEQ ID NO:'s 33-38 and/or a CDRH2 of SEQ ID NO:28 and/or a CDRH3 selected from SEQ ID NO:29 or SEQ ID NO:'s 40-49 and/or a CDRL1 selected from SEQ ID NO:30 or SEQ ID NO:'s 50-61 and/or a CDRL2 selected from SEQ ID NO:31 or SEQ ID NO:'s 62-72 and/or a CDRL3 of SEQ ID NO:32 or SEQ ID NO:'s73-76. For example the TNF-alpha antigen binding protein comprises CDRH1 of SEQ ID NO:27 and CDRH2 of SEQ ID NO:28 and CDRH3 of SEQ ID NO:29 and CDRL1 of SEQ ID NO:30 and CDRL2 selected from SEQ ID NO:31 and a CDRL3 of SEQ ID NO:32 or variants thereof.

The TNF-alpha antigen binding protein may be adalimumab. The TNF-alpha antigen binding protein may be BPC1494. The TNF-alpha antigen binding protein may be BPC 1496.

The TNF-alpha antigen binding proteins described herein are formulated in an acetate buffer. The formulation may be in liquid form. The formulation may further comprise one or more, a combination, or all of: a surfactant; a chelator; a salt; and an amino acid. The TNF-alpha antigen binding proteins are formulated at high concentrations, for example at 50 mg/mL. In one embodiment, the formulation does not comprise a polyol. In another embodiment, the formulation does not comprise a further buffer component, for example citrate. Therefore, the formulations described herein solve the problem of providing TNF-alpha antigen binding proteins, in particular the TNF-alpha antigen binding proteins as described in Table A, at high concentrations in a stable formulation, and avoid the burning and stinging effects of citrate-based buffers.

In one embodiment, the acetate buffer formulation further comprises a surfactant and a chelator. In another embodiment, the acetate buffer formulation further comprises a surfactant and a salt. In another embodiment, the acetate buffer formulation further comprises a surfactant and an amino acid. In another embodiment, the acetate buffer formulation further comprises a chelator and a salt. In another embodiment, the acetate buffer formulation further comprises a chelator and an amino acid. In another embodiment, the acetate buffer formulation further comprises a salt and an amino acid.

In one embodiment, the acetate buffer formulation further comprises a surfactant, a chelator, and a salt. In another embodiment, the acetate buffer formulation further comprises a surfactant, a chelator, and an amino acid. In another embodiment, the acetate buffer formulation further comprises a surfactant, a salt, and an amino acid. In another embodiment, the acetate buffer formulation further comprises a chelator, a salt, and an amino acid.

In one embodiment, the buffer is sodium acetate trihydrate. This may be at a concentration of 10 to 100 mM sodium acetate trihydrate (1.361 to 13.61mg/mL). Sodium acetate trihydrate may be present in an amount of 20 to 80 mM, 30 to 70 mM, 40 to 60 mM, or about 40mM, about 45mM, about 50mM, about 55mM, or about 60mM. In one embodiment, sodium acetate trihydrate is at a concentration of about 50mM (6.80mg/mL).

The acetate buffer may be the sole buffer. In other words, the formulation may not comprise another buffer component, such as phosphate or citrate buffer. Citrate buffer may be detrimental to the formulation for a number of reasons: (i) it may not be a good buffer because the values of the three dissociation constants are too close to permit distinction of the three proton receptor phases; (ii) citrate may act as a metal chelator and thus influence metal ion balance: (iii) citrate is a metabolite of the citric acid cycle and has the potential to influence cellular metabolism.

Suitable surfactants (also known as detergents) may include, e.g., polysorbates (for example, polysorbate 20 or 80), polyoxyethylene alkyl ethers such as Brij 35.RTM., poloxamers (for example poloxamer 188, Poloxamer 407), Tween 20, Tween 80, Cremophor A25, Sympatens ALM/230, and Mirj. In one embodiment, the surfactant is polysorbate 80. The formulation may comprise a concentration of 0.01 to 0.1 % polysorbate 80 (0.1 to 1 mg/mL). Polysorbate 80 may be present in an amount of 0.01 to 0.05%, or 0.01 to 0.03%; or about 0.015%, about 0.02%, or about 0.025%. In one embodiment, polysorbate 80 is at a concentration of about 0.02% w/v (0.2mg/mL). A high concentration of polysorbate 80, for example more than 0.1%, may be detrimental to the formulation because this surfactant may contain high levels of oxidants which may increase levels of oxidation upon storage of the formulation and therefore reduce shelf life.

Suitable chelating agents may include EDTA and metal complexes (e.g. Zn-protein complexes). In one embodiment, the chelating agents is EDTA. The formulation may comprise a concentration of 0.02 to 0.2 mM EDTA (0.00748 to 0.0748mg/mL). EDTA may be present in an amount of 0.02 to 0.15 mM, 0.02 to 0.1 mM, 0.03 to 0.08 mM, or 0.04 to 0.06 mM; or about 0.03 mM, about 0.04 mM, about 0.05 mM, or about 0.06 mM. In one embodiment, EDTA is at a concentration of about 0.05mM (0.018mg/mL).

Suitable salts may include any salt-forming counterions, such as sodium. For example, sodium chloride may be used, or anionic acetate instead of chloride as a counterion in a sodium salt may be used. In one embodiment, the salt is sodium chloride. The formulation may comprise a concentration of 25 to 100 mM sodium chloride (1.461 to 5.84mg/mL). Sodium chloride may be present in an amount of 35 to 90 mM, 45 to 80 mM, 25 to 70 mM, or 45 to 60mM; or 45mM, 46mM, 47mM, 48mM, 49mM, 50mM, 51mM, 52mM, 53mM, 54mM, 55mM. In one embodiment, sodium chloride is at a concentration of about 51mM (2.98mg/mL).

Suitable amino acids may include arginine. The formulation may comprise a concentration of 0.5 to 5% arginine free base (5 to 50mg/mL). Arginine free base may be present in an amount of In other embodiments, the arginine free base may be between 0.5 to 4.0%, 0.5 to 3.5%, 0.5 to 3.0%, 0.5 to 2.5%, or about 0.5%, about 0.75%, about 1%, about 1.5%, about 2%, or about 3%. In one embodiment, arginine is at a concentration of about 1% (10mg/mL).

A polyol is a substance with multiple hydroxyl groups, and includes sugars (reducing and nonreducing sugars), sugar alcohols and sugar acids. Examples of polyols include fructose, mannose, maltose, lactose, arabinose, xylose, ribose, rhamnose, galactose, glucose, sucrose, trehalose, sorbose, melezitose, raffinose, mannitol, xylitol, erythritol, threitol, sorbitol, glycerol, L-gluconate and metallic salts thereof. In one embodiment, the formulation of the invention does not comprise a polyol.

In one embodiment, the acetate buffer formulation further comprises one or more, a combination, or all of: polysorbate 80, EDTA, sodium chloride, and arginine free base.

The pH of the formulation may be adjusted to pH 5.0 to 7.0. In one embodiment, acetic acid is present (about 100 mM acetic acid) to adjust the formulation to about pH 5.5. In other embodiments, the pH may be adjusted to pH 5.0, 5.5, 6.0, 6.5 or 7.0. In yet other embodiments of the invention, NaOH or HCl is used to adjust the pH to 5.0, 5.5, 6.0, 6.5 or 7.0.

The TNF-alpha antigen binding proteins described herein may be formulated in the concentration range of 20 to 300 mg/mL. For example, the antigen binding protein is present in a concentration of 20-200 mg/mL or 50-100 mg/mL; or about 40 mg/mL or about 45 mg/mL or about 50 mg/mL or about 55 mg/mL or about 60 mg/mL or about 70 mg/mL or about 80 mg/mL or about 90 mg/mL, or about 100mg/mL. In one embodiment, the TNF-alpha antigen binding protein is at a concentration of about 50 mg/mL.

The TNF-alpha antigen binding protein may be adalimumab. The TNF-alpha antigen binding protein may be BPC1494. The TNF-alpha antigen binding protein may be BPC 1496.

In one embodiment, the formulation is stable for at least 1 year, at least 18 months, or at least 2 years. For example, the formulation is stable at a temperature of about 5°C for at least 1 year, at least 18 months, or at least 2 years. In another embodiment, the formulation is stable at room temperature (about 25°C). For example, the formulation is stable at a temperature of about 25°C for at least 14 weeks, at least 2 weeks, at least 1 week, at least 6 days, at least 5 days, at least 4 days, at least 3 days, at least 2 days or at least 1 day. In another embodiment, the formulation is stable at a temperature of about 40°C. For example, the formulation is stable at a temperature of about 40°C for at least 9 weeks or at least 4 weeks.

As shown by Examples 25 and 26 below, the formulations are stable at room temperature (about 25°C). Therefore, there is minimal risk of aggregates or low molecular weight fragments forming in pre-filled devices for injection that may be left at room temperature for more than the recommended time. Aggregates are potentially immunogenic (see The AAPS Journal 2006; 8 (3) Article 59 Themed Issue: Proceedings of the 2005 AAPS Biotec Open Forum on Aggregation of Protein Therapeutics, Guest Editor - Steve Shire, Effects of Protein Aggregates: An Immunologic Perspective) and low molecular weight fragments may illicit pre-existing autoantibodies (see J Immunol 2008; 181:3183-3192; Human Anti-IgG1 Hinge Autoantibodies Reconstitute the Effector Functions of Proteolytically Inactivated IgGs1).

The stability of a TNF-alpha antigen binding protein in a liquid formulation may be assessed by any one or a combination of: appearance by visual observation, protein concentration (A280nm), size exclusion chromatography (SEC), Capillary Iso-Electric Focussing (c-IEF), and by a functional binding assay (ELISA). For example, the percentage of monomer, aggregate, or fragment, or combinations thereof, can be used to determine stability. In one embodiment, a stable liquid formulation is a formulation having less than about 10%, or less than about 5% of the TNF-alpha antigen binding protein being present as aggregate in the formulation. The formulation may have a monomer content of at least 95%, or at least 96%, or at least 97%, or at least 98%, or at least 99%. The formulation may have a monomer content of at least 95%, or at least 96%, or at least 97%, or at least 98%, or at least 99% at room temperature (about 25°C) after about 2 weeks. The formulation may have a monomer content of at least 95%, or at least 96%, or at least 97%, or at least 98%, or at least 99% at room temperature (about 25°C) after about 1 week. The formulation may have a monomer content of at least 95%, or at least 96%, or at least 97%, or at least 98%, or at least 99% at room temperature (about 25°C) after about 1 day.

Thus, a pharmaceutical composition of the invention for injection could be prepared to contain 1 mL sterile buffered water, and between about 1 mg to about 100 mg, e.g. about 30 mg to about 100 mg or more preferably, about 35 mg to about 80mg, such as 40, 50, 80 or 90 mg of an antigen binding construct of the invention. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antigen binding construct formulations of the invention see Lasmar U and Parkins D "The formulation of Biopharmaceutical products", Pharma. Sci.Tech.today, page 129-137, Vol.3 (3rd April 2000), Wang, W "Instability, stabilisation and formulation of liquid protein pharmaceuticals", Int. J. Pharm 185 (1999) 129-188, Stability of Protein Pharmaceuticals Part A and B ed Ahern T.J., Manning M.C., New York, NY: Plenum Press (1992), Akers,M.J. "Excipient-Drug interactions in Parenteral Formulations", J.Pharm Sci 91 (2002) 2283-2300, Imamura, K et al "Effects of types of sugar on stabilization of Protein in the dried state", J Pharm Sci 92 (2003) 266-274,Izutsu, Kkojima, S. "Excipient crystalinity and its protein-structure-stabilizing effect during freeze-drying", J Pharm. Pharmacol, 54 (2002) 1033-1039, Johnson, R, "Mannitol-sucrose mixtures-versatile formulations for protein lyophilization", J. Pharm. Sci, 91 (2002) 914-922.

Preferably, the antigen binding protein of the invention is provided or administered at a dose of about 40 mg. Preferably the antigen binding protein is suitable for subcutaneous delivery and is delivered subcutaneously. Other dosing or administration routes may also be used, as disclosed herein.

In one emboduiment the antigen binding proteins according to any aspect of the invention shows increased Mean Residence Time as compared to an IgG comprising the light chain sequence of SEQ ID No. 2 and heavy chain sequence of SEQ ID No.12.

The binding ability of modified IgGs and molecules comprising an IgG constant domain or FcRn binding portion thereof can be characterized by various *in vitro* assays. PCT publication WO 97/34631 by Ward discloses various methods in detail. For example, in order to compare the ability of the modified IgG or fragments thereof to bind to FcRn with that of the wild type IgG, the modified IgG or fragments thereof and the wild type IgG can be radio-labeled and reacted with FcRn-expressing cells *in vitro.* The radioactivity of the cell-bound fractions can be then counted and compared. The cells expressing FcRn to be used for this assay are may be endothelial cell lines including mouse pulmonary capillary endothelial cells (B10, D2.PCE) derived from lungs of B10.DBA/2 mice and SV40 transformed endothelial cells (SVEC) (Kim et al., J Immunol., 40: 457-465,1994) derived from C3H/HeJ mice. However, other types of cells which express sufficient number of FcRn, including mammalian cells which express recombinant FcRn of a species of choice, can be also used. Alternatively, after counting the radioactivity of the bound fraction of modified IgG or that of unmodified IgG, the bound molecules can be then extracted with the detergent, and the percent release per unit number of cells can be calculated and compared.

Affinity of antigen binding proteins of the inventions for FcRn can be measured by surface plasmon resonance (SPR) measurement using, for example, a BIAcore 2000 (BIAcore Inc.) as described previously (Popov et al., Mol. Immunol., 33: 493-502,1996; Karlsson et al., J Immunol. Methods, 145: 229-240,1991, both of which are incorporated by reference in their entireties). In this method, FcRn molecules are coupled to a BIAcore sensor chip (e. g., CM5 chip by Pharmacia) and the binding of modified IgG to the immobilized FcRn is measured at a certain flow rate to obtain sensorgrams using BIA evaluation 2.1 software, based on which on-and off-rates of the modified IgG, constant domains, or fragments thereof, to FcRn can be calculated. Relative affinities of antigen binding proteins of the invention and unmodified IgG for FcRn can be also measured by a simple competition binding assay. Furthermore, affinities of modified IgGs or fragments thereof, and the wild type IgG for FcRn can be also measured by a saturation study and the Scatchard analysis.

Transfer of modified IgG or fragments thereof across the cell by FcRn can be measured by in vitro transfer assay using radiolabeled IgG or fragments thereof and FcRn- expressing cells and comparing the radioactivity of the one side of the cell monolayer with that of the other side. Alternatively, such transfer can be measured *in vivo* by feeding 10-to 14-day old suckling mice with radiolabeled, modified IgG and periodically counting the radioactivity in blood samples which indicates the transfer of the IgG through the intestine to the circulation (or any other target tissue, e. g., the lungs). To test the dose-dependent inhibition of the IgG transfer through the gut, a mixture of radiolabeled and unlabeled IgG at certain ratio is given to the mice and the radioactivity of the plasma can be periodically measured (Kim et al., Eur. R Immunol., 24: 2429-2434,1994).

The half-life of antigen binding proteins can be measured by pharmacokinetic studies according to the method described by Kim et al. (Eur. J. of Immuno. 24: 542,1994), which is incorporated by reference herein in its entirety. According to this method, radiolabeled antigen binding protein is injected intravenously into mice and its plasma concentration is periodically measured as a function of time, for example, at 3 minutes to 72 hours after the injection. The clearance curve thus obtained should be biphasic. For the determination of the in vivo half-life of the modified IgGs or fragments thereof, the clearance rate in β-phase is calculated and compared with that of the unmodified IgG.

Antigen binding proteins of the invention may be assayed for the ability to immunospecifically bind to an antigen. Such an assay may be performed in solution (e. g., Houghten, BiolTechniques, 13: 412-421,1992), on beads (Lam, Nature, 354: 82-84,1991, on chips (Fodor, Nature, 364: 555-556,1993), on bacteria (U. S. Patent No. 5,223,409), on spores (U. S. Patent Nos. 5,571,698; 5,403,484; and 5,223,409), on plasmids (Cull et al., Proc. Natl. Acad. Sci. USA, 89: 1865-1869,1992) or on phage (Scott and Smith, Science, 249: 386-390,1990; Devlin, Science, 249: 404-406,1990; Cwirla et al., Proc. Natl. Acad. Sci. USA, 87: 6378-6382, 1990; and Felici, J : Mol. Biol., 222: 301-310, 1991) (each of these references is incorporated herein in its entirety by reference). Antibodies that have been identified to immunospecifically bind to an antigen or a fragment thereof can then be assayed for their specificity affinity for the antigen.

The antigen binding proteins of the invention may be assayed for immunospecific binding to an antigen and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay),"sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e. g., Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

In a preferred embodiment, BIAcore kinetic analysis is used to determine the binding on and off rates of antibodies to an antigen. BIAcore kinetic analysis comprises analyzing the binding and dissociation of an antigen from chips with immobilized antibodies on their surface.

Antigen binding protein: The term "antigen binding protein" as used herein includes reference to antibodies, antibody fragments and other protein constructs, which are capable of binding to TNF-alpha.

Antibody: The term "antibody" is used herein in the broadest sense and includes reference to molecules with an immunoglobulin-like domain and includes monoclonal, recombinant, polyclonal, chimeric, humanised, bispecific and heteroconjugate antibodies.

Human IgG1 heavy chain constant domain: refers to human amino acid sequence for the IgG1 heavy chain constant domain that is found in nature, including allelic variations.

"Half-life (t1/2)" refers to the time required for the concentration of the antigen binding polypeptide to reach half of its original value. The serum half-life of proteins can be measured by pharmacokinetic studies according to the method described by Kim et al. (Eur. J. of Immuno. 24: 542, 1994). According to this method, radiolabeled protein is injected intravenously into mice and its plasma concentration is periodically measured as a function of time, for example, at about 3 minutes to about 72 hours after the injection. Other methods for pharmacokinetic analysis and determination of the half-life of a molecule will be familiar to those skilled in the art. Details may be found in Kenneth, A et al: Chemical Stability of Pharmaceuticals: A Handbook for Pharmacists and in Peters et al, Pharmacokinetic analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC), and "Clinical Pharmacokinetics: Concepts and Applications", Rowland and Tozer, Third Edition (1995).

"Clearance (CL)" refers to the volume of plasma irreversibly cleared of a protein per unit time. Clearance is calculated as the Dose/AUC (AUC : is the Area Under Curve or Area under the plasma drug concentration time curve). Clearance can also be calculated by the rate of drug elimination divided by the plasma concentration of the drug (rate of elimination = CL*concentration)

"Mean Residence Time (MRT)" is the average time that the antigen binding polypeptides reside in the body before being irreversibly eliminated. Calculated as MRT= AUMC/AUC.

"Steady state concentration" (Css) is the concentration reached when the drug elimination rate becomes equal to drug administration rate as a result of continued drug administration. Css fluctuates between peak and trough levels and is measured in microgram/ml. "Mean steady-state trough concentration" refers to the mean of the trough level across the patient population at a given time.

"Comparable mean steady-state trough concentration" refers to mean steady-state trough concentration which is the same or within about 10% to 30% of the stated value. Comparable mean steady-state trough concentration for the antigen binding polypeptides of the invention may be considered to be those mean steady-state trough concentrations that are 0.8 to 1.25 times the mean steady-state trough concentration achieved with an IgG comprising the light chain sequence of SEQ ID No. 2 and the heavy chain sequence of SEQ ID No. 12.

Half lives and AUC can be determined from a curve of serum concentration of drug (for example the antigen binding polypeptide of the present invention) against time. Half life may be determined through compartmental or non-compartmental analysis. The WINNONLIN™ analysis package (available from Pharsight Corp., Mountain View, CA94040, USA) can be used, for example, to model the curve. In one embodiment, "half life" refers to the terminal half life.

Specifically binds: The term "specifically binds" as used throughout the present specification in relation to antigen binding proteins means that the antigen binding protein binds to TNF-alpha with no or insignificant binding to other unrelated proteins. The term however does not exclude the fact that the antigen binding proteins may also be cross-reactive with closely related molecules. The antigen binding proteins described herein may bind to TNF-alpha with at least 2, at least 5, at least 10, at least 50, at least 100, or at least 1000 fold greater affinity than they bind to closely related molecules. CDRs:

"CDRs" are defined as the complementarity determining region amino acid sequences of an antigen binding protein. These are the hypervariable regions of immunoglobulin heavy and light chains. There are three heavy chain and three light chain CDRs (or CDR regions) in the variable portion of an immunoglobulin. Thus, "CDRs" as used herein refers to all three heavy chain CDRs, all three light chain CDRs, all heavy and light chain CDRs, or at least two CDRs.

Throughout this specification, amino acid residues in variable domain sequences and full length antibody sequences are numbered according to the Kabat numbering convention. Similarly, the terms "CDR", "CDRL1", "CDRL2", "CDRL3", "CDRH1", "CDRH2", "CDRH3" used in the Examples follow the Kabat numbering convention. For further information, see Kabat et al., Sequences of Proteins of Immunological Interest, 4th Ed., U.S. Department of Health and Human Services, National Institutes of Health (1987).

% identity of variants : The term "identical" or "sequence identity" indicates the degree of identity between two nucleic acid or two amino acid sequences when optimally aligned and compared with appropriate insertions or deletions. The variants described herein may have 90, 91, 92, 93, 94, 95, 96, 97, 98, or 99% identity to the native CDR or variable domain sequences at the amino acid level.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In one aspect such open ended terms also comprise within their scope a restricted or closed definition, for example such as "consisting essentially of", or "consisting of".

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

All documents referred to herein are incorporated by reference to the fullest extent permissible.

Any element of a disclosure is explicitly contemplated in combination with any other element of a disclosure, unless otherwise apparent from the context of the application.

The present invention is further described by reference to the following examples, not limiting upon the present invention.

### Examples

### Example 1: Cloning of antibody expression vectors

The DNA expression constructs encoding the variable heavy (VH) and variable light (VL) domains of an anti-TNFα antibody were previously prepared de novo and included restriction sites for cloning into mammalian expression vectors. Both heavy and light chain variable domain sequences were sequence optimised for expression in mammalian cells (for methodology see WO2009024567 and Kotsopoulou et al, J Biotechnol (2010) 146: 186-193). Information describing the heavy and light chain variable region sequences can be found in US patent US6090382. To generate the constructs used in this study, the variable heavy domain (VH) sequences were amplified using PCR. The PCR primers contained *HindIII* and *Spel* restriction sites to frame the VH domain containing the signal sequence for cloning into a pTT mammalian expression vectors containing the human γ1 constant region. Similarly the VL domain sequence was amplified by PCR using primers containing *HindIII* and *BsiWI* restriction sites to facilitate cloning into a pTT mammalian expression vector containing the human kappa constant region. The heavy chain expression plasmid was given the code SJC322 and the light chain expression plasmid was given the plasmid code SJC321.

DNA expression constructs encoding alternative variable heavy and light chain regions of anti-TNFα antibodies with modifications in the CDR regions (as described in Rajpal et al. PNAS (2005) 102(24): pg 8466-8471) were prepared de novo by build up of overlapping oligonucleotides and similar molecular biology techniques to those described above. The resulting plasmids encoding the heavy and light chains of variants cb1-3, cb2-6 and cb2-44 are described in Table 1.

### Example 2: Engineering of the Fc region

Forward and reverse priming primers were used to introduce modifications (M252Y/S254T/T256E and T250Q/M428L) into the human γ1 constant region of the plasmid encoding the heavy chain of pascolizumab (anti-IL-4 antibody) using the Quikchange protocol (Promega).

As described in Example 1 above, a PCR fragment encoding the VH domain of an anti-TNFα antibody was generated using a previously constructed, codon optimised vector as a template. The resulting fragment was cloned using *HindIII* and *SpeI* into a pTT expression vector containing the modified human γ1 constant region described in the preceding paragraph. The plasmid encoding the heavy chain of the anti-TNFα antibody with the M252Y/S254T/T256E modification was designated SJC324. The plasmid encoding the heavy chain with the T250Q/M428L modification was designated SJC323.

Forward and reverse priming primers were used to introduce modifications into the human γ1 constant region of anti-TNFα heavy chain expression plasmid SJC322 using the Quikchange protocol (Promega). Plasmid SJC326 encodes the anti-TNFα heavy chain containing the M428L/N434S modification in the human γ1 constant region. Plasmid SJC328 encodes the anti-TNFα heavy chain containing the V308F modification in the human γ1 constant region.

### Example 3: Expression of antibodies in HEK2936E cells using pTT5 episomal vectors

Expression plasmids encoding the heavy and light chains described above were transiently co-transfected into HEK 293 6E cells. Expressed antibody was purified from the supernatant by affinity chromatography using a 1ml HiTrap Protein A column (GE Healthcare). Table 1 below shows the list of antibodies produced.

Some antibodies were also expressed in CHO cells using a different set of expression vectors. See Examples 13, 14 and 15 for a description of the molecular biology, expression and purification.

**Table 1: List of expressed antibodies**

| **BPC code** | **CDR variant** | **Fc modifications** | **Heavy chain expression vector** | **SEQ ID of heavy chain** | **Light chain expression vector** | **SEQ ID of light chain** |
|---|---|---|---|---|---|---|
| BPC1492 | None | Wild-type | SJC322 | 12 | SJC321 | 2 |
| BPC1494 | None | M252Y/S254T/T256 E | SJC324 | 5 | SJC321 | 2 |
| BPC1496 | None | M428L/N434S | SJC326 | 9 | SJC321 | 2 |
| BPC1493 | None | T250Q/M428L | SJC323 | 15 | SJC321 | 2 |
| BPC1498 | None | V308F | SJC328 | 18 | SJC321 | 2 |
| BPC1499 | cb1-3 | Wild-type | SJC336 | 150 | SJC339 | 147 |
| BPC1500 | cb2-44 | Wild-type | SJC337 | 151 | SJC340 | 148 |
| BPC1501 | cb2-6 | Wild-type | SJC336 | 150 | SJC338 | 149 |

### Example 4: Binding of antibodies to tumour necrosis factor alpha in a direct binding ELISA

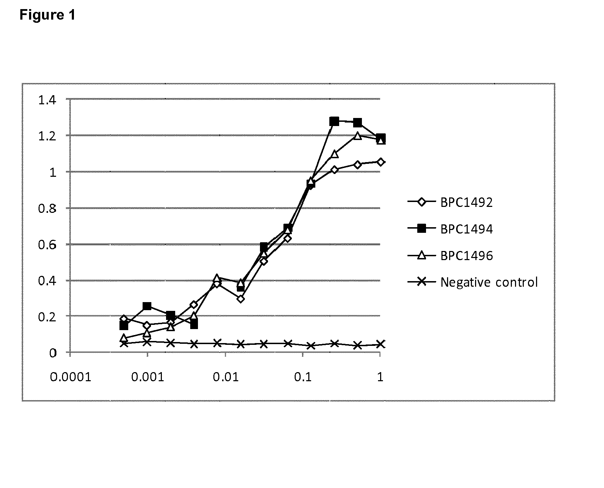

A binding ELISA was carried out to test the binding of the expressed antibodies purified using protein A to recombinant tumour necrosis factor alpha (TNFα). ELISA plates were coated with recombinant human TNFα at 0.1µg/ml and blocked with blocking solution (4% BSA. Various dilutions of the purified antibody were added (diluted in 4% BSA in T Tris-buffered saline at pH8.0 containing 0.05% Tween 20) and the plate was incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCl. Absorbance was measured at 490nm with a plate reader and the mean absorbance plotted against concentration. The results are shown in Figure 1 and confirm that all the antibodies have a similar profile.

### Example 5: Analysis of antibodies in an L929 in vitro neutralisation assay

This assay was used to test the neutralising ability **of** the antibodies to neutralise TNF-α and inhibit cell death. Briefly, L929 cells were seeded in a 96-well flat-bottomed plate at 10,000/well in 100µl RPMI 1640 (w/o phenol red) and incubated overnight at 37°C, 5% CO₂. Cells were sensitised with 1.25µg/ml actinomycin D for 1 hour. For the neutralising study, 0.001-60µg/ml (0.0067- 400 nM) anti-TNF-α mAb was pre-incubated with approx. 2ng/ml (approximately 0.05nM) TNF-α in a 1:1 ratio for 1 hour at room temperature. For control group, RPMI was used in place of the antibody. Following the 1 h pre-incubation with actinomycin D, 20 µl of antibody-antigen complex was added per well. 10µl media alone was added to wells as a negative control. Plates were incubated at 18 hour at 37°C, 5% CO₂. Following this treatment period, cell viability was determined by a cell titer-Glo Luminescent assay kit according to manufacturer's instructions (Promega, Madison USA). For L929 assay, the percentage cell viability of the unknowns was expressed as a percentage of the untreated group (taken as a 100%) and IC50 values were determined by Graphpad prism. Differences in IC50 values of antibodies was assessed by one-way ANOVA (Newman-Keuls post hoc test) and considered significant at P-values of less than 0.05. Data is represented as mean ± SEM, of n=4 experiments measured in duplicate. IC50 values for each antibody were determined and are listed in Table 2 below. The results show that the potency of all the antibodies tested are comparable.

**Table 2: IC₅₀ values for various anti-TNFa antibodies in an L929 neutralisation assay**

| **Antibody** | **IC₅₀ value (µg/ml)** |
|---|---|
| BPC1492 | 1.19 ± 0.10 |
| BPC1494 | 1.20 ± 0.13 |
| BPC1496 | 1.18 ± 0.10 |
| Adalimumab | 1.09 ± 0.07 |

Table 3 shows the IC50 values derived from the experiment. The results indicate that the improved anti-TNFα antibodies (BPC1499, BPC1500, BPC1501) show increased potency in this assay compared to BPC1492 and adalimumab.

**Table 3: IC₅₀ values for improved anti-TNFα antibodies in an L929 neutralisation assay**

| **Antibody** | **IC₅₀ value (µg/ml)** |
|---|---|
| BPC1492 | 1.19 ± 0.1 |
| BPC1499 | 0.21 ± 0.04 |
| BPC1500 | 0.13 ± 0.02 |
| BPC1501 | 0.21 ± 0.03 |
| Adalimumab | 1.09 ± 0.07 |

### Example 6: Effect of antibodies on in vitro IL-6 release

The neutralising ability of antibodies was determined by measuring their effect on inhibiting TNF-α mediated IL-6 release from whole blood cells. Briefly, 130 µL of whole blood was added to each well and plates were incubated at 37°C in a humidified 5% CO₂ incubator for 1 hour. For the neutralising study, 0.001-30µg/ml (0.0067- 200 nM) TNF-α mAb was pre-incubated with 10ng/ml (approx. 0.4 nM) TNF-alpha in a 1:1 ratio for 1 hour at 4°C. For control group, RPMI was used in place of the antibody. Following this pre-treatment, 20 µl of antigen-antibody complex or RPMI (negative control) was added per well and plates were incubated for 24 hour at 37°C, 5% CO₂. 100 µL PBS (w/o MgCl₂ or CaCl₂) added to each well and placed on plate shaker for 10 mins at 500 rpm. Plates were then spun at 2000 rpm for 5 mins. 120 µL supernatant was carefully removed and transferred to fresh 96-well round bottomed plate and IL-6 release was determined using an MSD based assay kit (Meso Scale Diagnostics, Maryland USA). For the whole blood assay, the MSD signal for each sample was read using a MSD SECTOR® Imager 2400 and IL-6 release from the cells was quantified using a standard data analysis package in PRISM 4.00 software (GraphPad. San Diego, USA). The percentage of IL-6 inhibition by each antibody was expressed as a percentage of the TNF-α alone treated group. Hence, dose response curves were obtained for each antibody and IC50 values were determined. Using the log of the IC50 values, the difference in potency of the antibodies was determined by one-way ANOVA (Newman-Keuls post hoc test) and considered significant at P-values of less than 0.05 for each donor (n=3). Data is represented as mean ± SEM of three donors, measured in duplicate. Table 4 below shows the IC50 values derived from these data. These results suggest that there is no significant difference in potency between the antibodies tested.

**Table 4: IC₅₀ values for various anti-TNF antibodies in a TNFα-induced IL-6 release assay**

| **Antibody** | **IC₅₀ value (nM)** |
|---|---|
| BPC1492 | 0.72 ± 0.32 |
| BPC1494 | 0.62 ± 0.11 |
| BPC1496 | 0.64 ± 0.13 |
| Adalimumab | 0.47 ± 0.09 |

The IC50 values are shown in Table 5. The results indicate that the improved anti-TNFα antibodies (BPC1499, BPC1500, BPC1501) show increased potency in this assay.

**Table 5: IC₅₀ values for various improved anti-TNF antibodies in a TNFα-induced IL-6 release assay**

| **Antibody** | **IC₅₀ value (nM)** |
|---|---|
| BPC1492 | 0.72 ± 0.32 |
| BPC1494 | 0.62 ± 0.12 |
| BPC1499 | 0.14 ± 0.02 |
| BPC1500 | 0.11 ± 0.05 |
| BPC1501 | 0.15 ± 0.03 |
| Adalimumab | 0.47 ± 0.09 |

### Example 7: Accelerated stressor studies

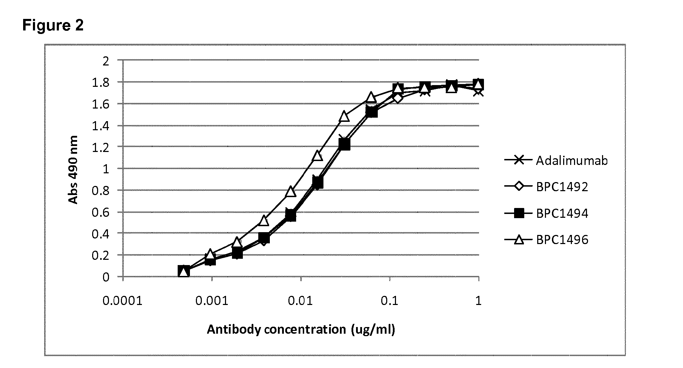

Prior to the study, antibodies to be tested were quantified on a spectrophotometer at OD280nm and diluted to 1.1mg ml in PBS (pH7.4). An aliquot was removed and 10%v/v of 500mM sodium acetate was added to give a final concentration of1mg/ml at pH5.5 and the sample inspected for precipitation. The remaining sample in PBS had 10% PBS v/v added to a final concentration of 1mg/ml at pH7.4 and an aliquot of this sample was removed to provide a baseline aggregation level (as monitored by size exclusion chromatography). The samples were then incubated at 37°C for two weeks in an incubator, after which the samples were re-quantified on a spectrophotometer at OD280nm and assessed (by size exclusion chromatography) for aggregation. The samples were tested for human TNFα binding in a direct binding ELISA. The results are shown in Figure 2 and confirm that the binding activity of all antibodies tested is comparable following the accelerated stressor study.

### Example 8: Stability study in 25% human serum

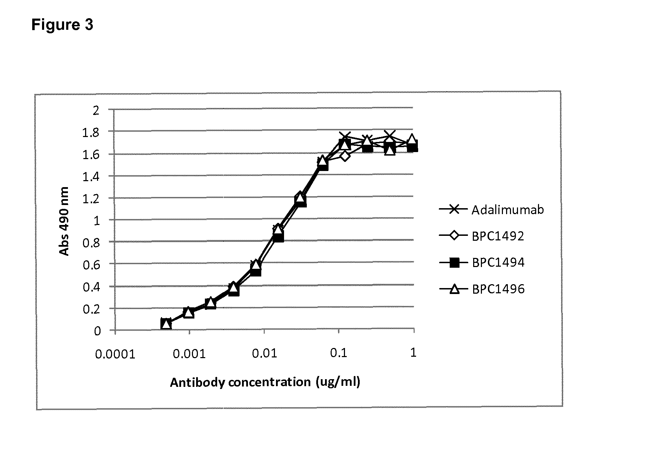

Prior to the study, antibodies to be tested were quantified on a spectrophotometer at OD280nm and diluted to 1.25 mg/ml in PBS (pH7.4). An aliquot was removed and 25%v/v of human serum was added to give a final concentration of 1mg/ml. The remaining sample in PBS had 25% PBS v/v added to a final concentration of 1mg/ml and an aliquot of this sample was removed to provide a baseline level. The samples were then incubated at 37°C for two weeks in an incubator, after which the samples were tested for human TNFα binding in a direct binding ELISA. The results are shown in Figure 3 and confirm that the binding activity of all antibodies tested is comparable following incubation in 25% human serum for two weeks.

### Example 9: Analysis of binding to human TNFα following freeze-thaw

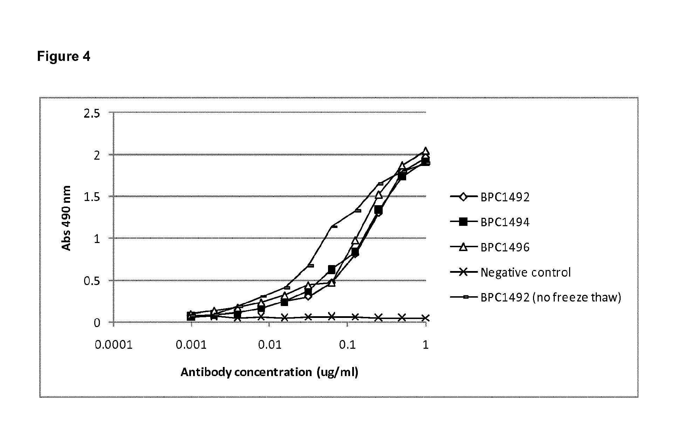

Antibody samples were diluted to 1mg/ml in a buffer containing 50mM Acetate and 150mM NaCl (pH6.0), snap-frozen in dry ice and then thawed at 4°C overnight. Binding of the antibodies to human TNFα was tested in comparison to an antibody which had not been snap-frozen.To assess the binding activity following freeze-thaw, ELISA plates were coated with recombinant human TNFα at 1µg/ml and blocked with blocking solution (4% BSA in Tris buffered saline). Various concentrations were added to the coated plates and incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCL. Absorbance was measured at 490nm with a plate reader and the mean absorbance plotted against concentration. The results are shown in Figure 4 and confirm that the binding activity of all antibodies tested is comparable following freeze-thaw.

### Example 10: Analysis of binding of anti-TNFα antibodies to FcγRIIIa

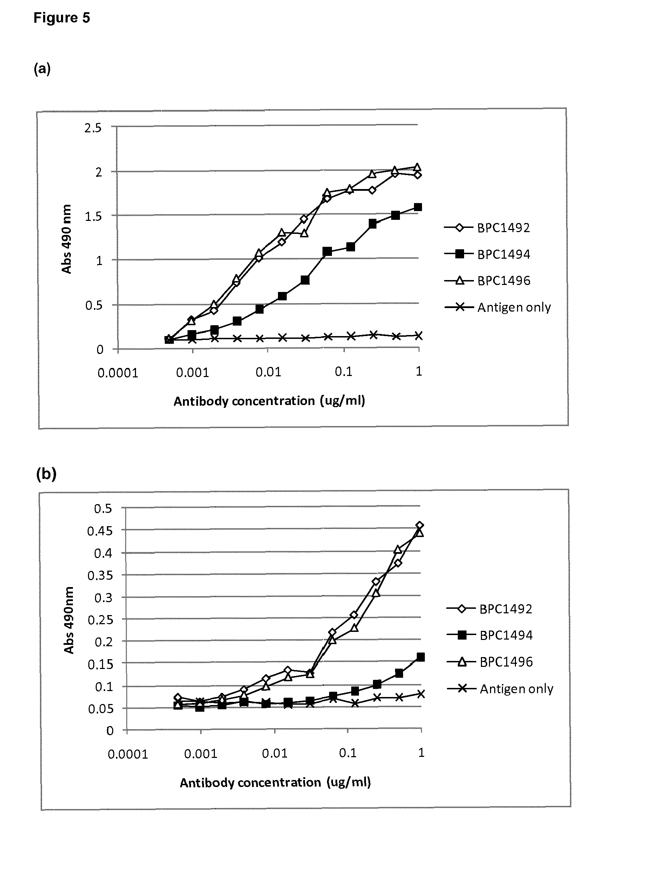

ELISA plates were coated with recombinant human FcγRIIIa (V158 and F158 variants) at 1µg/ml and blocked with blocking solution (4% BSA in Tris buffered saline). Various concentrations were added to the coated plates and incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCl. Absorbance was measured at 490nm with a plate reader and the mean absorbance plotted against concentration. The results are shown in Figure 5a and 5b and confirms that BPC1494 has reduced capacity to bind FcγRIIIa (V158 and F158 variants) compared to BPC1492 and BPC1496.

### Example 11: ProteOn Analysis: FcRn Binding

Antibodies for testing were immobilised to similar levels on a GLC biosensor chip (BioRad 176-5011) by primary amine coupling. Recombinant human and cynomolgus FcRn were used as analytes at 2048nM, 512nM, 128nM, 32nM, and 8nM, an injection of buffer alone (i.e. 0nM) was used to double reference the binding curves. Regeneration of the antibody surface following FcRn injection used HBS-N at pH9.0, the assay was run on the ProteOn XPR36 Protein Interaction Array System at 25°C and run in HBS-N pH7.4 and HBS-N pH6.0 with the FcRn diluted in appropriate buffer. Affinities were calculated using Equilibrium model, inherent to the ProteOn analysis software, using a "Global R-max" for binding at pH6.0 and the R-max from binding at pH6.0 for affinity calculation at pH7.4. Since the binding curves did not reach saturation at pH7.4, the values obtained are unlikely to be true affinities however they can be used to rank constructs. The results are shown in Table 6 and confirm that BPC1494 and BPC1496 have an improved affinity for human and cyno FcRn at pH6.0 when compared to BPC1492.

**Table 6 Affinities of Anti-TNF alpha constructs binding to Human and Cyno FcRn**

| **BPC number** | **Human pH6.0 KD(nM)** | **Human pH7.4 KD(nM)** | **Cyno pH6.0 KD(nM)** | **Cyno pH7.4 KD(nM)** |
|---|---|---|---|---|
| BPC1492 | 554 | 21200 | 579 | 29700 |
| BPC1494 | 204 | 2320 | 239 | 2640 |
| BPC1496 | 144 | 1910 | 154 | 2100 |
| BPC1497 | 428 | 15500 | 464 | 20800 |
| BPC1498 | 357 | 5910 | 402 | 6280 |
| BPC1493 | 264 | 4390 | 295 | 4690 |

### Example 12: PK studies in human FcRn transgenic mice.

In a single dose pharmacokinetic study BPC1494 and BPC1492, were administered intravenously (IV) at 1 mg/kg to two different strains of FcRn humanised mice and one strain deficient in FcRn (Petkova et al. Int. Immunol (2010) 18(12): 1759-1769). Plasma samples were analyzed for BPC1494 or BPC1492, as appropriate, using a validated Gyrolab fluorescent immunoassay.

The methods used biotinylated human TNF alpha as the capture antigen and an Alexa labelled anti-human IgG (Fc specific) antibody as the detection antibody. Using an aliquot of mouse plasma diluted 1:10 with assay buffer, the lower limit of quantification (LLQ) was 100 ng/mL and the higher limit of quantification (HLQ) was100,000 ng/mL. Plasma concentrations below the lowest standards were considered to be not quantifiable. QC samples prepared at three different concentrations and stored with the study samples, were analysed with each batch of samples against separately prepared calibration standards. For the analyses to be acceptable, at least one QC at each concentration must not deviate from nominal concentration by more than 20%. The QC results from this study met these acceptance criteria.

PK analysis was performed by non-compartmental pharmacokinetic analysis using WinNonLin, version 6.1. All computations utilised the nominal blood sampling times. The systemic exposure to BPC1494 and BPC1492 was determined by calculating the area under the plasma concentration time curve (AUC) from the start of dosing until the last quantifiable time point (AUC₀₋ₜ) using the linear log trapezoidal calculation method. Further PK parameters could not be derived from the data due discrepancies in sample labelling.

**Table 7 - Summary pharmacokinetic parameters for BPC1494 and BPC1492 following a single intravenous administration (bolus) at a target dose of 1 mg/kg to transgenic mice**

| Compound | Strain | Cmax (ug/mL) | AUC (hr*ug/mL) |
|---|---|---|---|
| BPC1494 | 1 | 13.8 | 2240 |
| BPC1492 | | 14.8 | 1730 |
| BPC1494 | 2 | 12.0 | 1320 |
| BPC1492 | | 13.2 | 1060 |
| BPC1494 | 3 | 13.6 | 214 |
| BPC1492 | | 12.2 | 250 |

| | | | |
|---|---|---|---|
| Strain 1 = mFcRn-/- hFcRn (32) Tg/Tg Strain 2 = mFcRn-/- hFcRn (276) Tg/Tg Rag1-/- Strain 3 = mFcRn -/-/Rag1-/- | | | |

Similar Cₘₐₓ concentrations were obtained for all groups. In both human FcRn knock-in mouse strains BPC1494 had a higher exposure (AUC₀₋ₜ) than BPC1492, although this difference was not notable (1.3 fold). In the absence of both human and mouse FcRn BPC1492 had a higher exposure than BPC1494.

### Example 13: Cloning of antibody expression vectors into pEF vectors

In some cases, the DNA encoding the expression cassettes for the heavy and light chains were excised from the vectors described in Example 3 using *HindIII* and *EcoRI* and cloned into pEF vectors, where expression occurs from the hEF1a promoter, using standard molecular biology techniques (for description of vectors see Kotsopoulou et al J. Biotechnol (2010) 146: 186-193).

**Table 8**

| BPC code | Fc modification | Heavy chain expression vectors | Light chain expression vector | Heavy chain SEQ ID No. | Light chain SED ID No. |
|---|---|---|---|---|---|
| BPC1492 | None | SJC330 | SJC329 | 12 | 2 |
| BPC1494 | M252Y/S254T/T256E | SJC331 | SJC329 | 5 | 2 |
| BPC1496 | M428L/N434S | SJC332 | SJC329 | 9 | 2 |

### Example 14: Expression of antibodies in CHO cells using pEF expression vectors

Expression plasmids encoding heavy and light chains were co-transfected into CHO DG44 cells and expressed at scale to produce antibody. For the generation of BPC1492 plasmids SJC329 and SJC330 were used. For the expression of BPC1494 plasmids SJC329 and SJC331 were used. For BPC1496 plasmids SJC329 and SJC332 were used.

Briefly, 30µg DNA (15µg heavy chain and 15µg light chain) was linearised overnight with *Not1* restriction enzyme. The resultant restricted DNA was then ethanol precipitated and re-dissolved in TE buffer. From culture, 6X10⁶ CHO DG44 cells were obtained and washed in 10ml of PBS. The cell pellet was then re-suspended in 300µl of Amaxa solution V. 100µl of the aforementioned cell suspension was then added into to each of three Amaxa cuvettes, which also contained 3µg of the linearised DNA. The cuvettes were inserted into an Amaxa nucleofector II device and electroporated with pre-set programme U-023. The contents of the three cuvettes (300µl) of electroporated cells were added to 10ml of warmed MR14 medium (including nucleosides and BSA) and incubated in a T75 flask for 48 hours. Following this period, the medium was changed to nucleoside-free-MR14 (MR14 containing only BSA)). Every 3-4 days, conditioned medium was removed and replaced with fresh selection medium. Once cells had undergone recovery, the medium was substituted to 2X MR14 and IgG expression was confirmed by nephlometry. 2L shake-flasks were seeded with 1L of the IgG-expressing cells at 0.6X10⁶/ml and grown for 7 days. Cells were separated from supernatant by centrifugation and the supernatant was used for protein purification.

1 litre cell culture supernatants were purified using a 2-step automated process on an AKTA Xpress system. The antibody was captured on a 5ml MabSelectSure column and then washed prior to elution. The eluted antibody was then loaded onto a 440ml Superdex 200 gel filtration column and 2ml fractions collected in a 96-well block. Fractions of purified antibody were pooled and 0.2µm filtered and then concentrated to ∼5mg/ml using Amicon spin concentrators. The final material was again 0.2µm filtered and then dispensed into sterile tubes for delivery. The final material was subject to analytical SEC to determine aggregation, an endotoxin assay, LC-MS for accurate mass determination (included PNGaseF and untreated material to determine glycosylation), SDS PAGE electrophoresis, PMF for sequence confirmation and A280 for concentration determination.

### Example 15: Alternative method for expression of antibodies in CHO cells using pEF expression vectors

DHFR-null CHO DG44 cells were obtained from Dr. Chasin of Columbia University. These cells were subsequently adapted to a chemically defined medium. These adapted host cells were designated DG44-c and are cultured in proprietary chemically defined medium supplemented with Glutamax and HT-supplement.

Generation of the polyclonal pool: For more details on protocols see WO2009024567 and Kotsopoulou et al, J. Biotechnol (2010) 164(4): 186-193. Briefly, DG44-c cells were transfected with plasmids encoding the heavy and light chains and DHFR and neoR respectively by electroporation (using the Amaxa nucleofector system). At 48 hours post transfection, selection was initiated by addition of G418 (at a final concentration of 400µg/ml) and removal of HT. When viability and cell counts increased sufficiently (in this case 2 months post transfection) methotrexate (MTX) was added at a final concentration of 5nM. Cells were scaled up and production curves were initiated 9-16 days after addition of MTX. For these production curves cells were seeded at 0.6-0.8x10⁶ cells/ml in chemically defined media and were fed on days 6, 9 or 10, 12 or 13 and/or 16. Supernatant was collected when viability dropped to approximately 50% and the cells were removed by centrifugation at 4000g for 30 mins followed by filtration through a sartobran capsule.

Antibodies were purified at room temperature using a two step chromatographic procedure: Initial capture was performed using a 50ml MabSelect SuRe column (GE Healthcare) followed by Size Exclusion Chromatography (SEC) with a 1.5L Superdex 200 pg SEC (GE Healthcare). The conditioned media was loaded onto a pre-equilibrated MabSelect SuRe column at a flow rate of 9cm/h. Following washing to base line with equilibration buffer (50mm Tris pH 8.0, 2M NaCl) the column was washed with a low salt buffer buffer (50mM NaCl Tris pH 8.0, 150mM NaCl) until conductivity was stable. The column was then eluted with elution buffer (25mM Citrate pH 2.5). Fractions corresponding to peak protein elution were immediately neutralized with 1/10 vol. 1.0M Tris pH 8.0 which were then pooled and filtered through a 0.2µm bottletop filter. The recovered sample was loaded at 21cm/h onto the SEC column pre-equilibrated with SEC buffer (50mM Na Acetate, 150mM NaCl). The fractions containing the main (monomeric) protein peak were pooled and filter sterilized.

Antibodies prepared by this method were used for analytical comparability studies summarised in the following example.

### Examples 16: Analytical comparability on stressed and control samples

Size exclusion chromatography was carried out to determine the aggregation levels of the protein. The optimised method involved injection of the sample onto a TOSOH TSK G3000SWXL column which had been equilibrated in 100 mM sodium phosphate, 400 mM NaCl, pH 6.8. Absorbance was measured at both 280nm and 214nm. Reverse-phase HPLC separates proteins and their isoforms based on hydrophobicity. Protein was injected onto a PLRP-S 1000 °A 8µm column and eluted using a gradient produced by 50%Formic acid, and 95% Acetonitrile. Absorbance was measured at 280nm. The purity of the molecule is reported as a percentage of the main peak area relative to the total peak area. Different isoforms of the mAb were separated on the basis of their pl values using capillary isoelectric focussing (clEF). IEF separation was performed on a 10cm, UV280 transparent cartridge capillary. The optimised method involved a solution containing 5% pH 3-10 ampholytes, 10mM NaOH, protein of interest and internal pl markers (7.05 and 9.5) which was loaded into the capillary by pressure injection.

The specific activity of antibodies (adalimumab, BPC1494, BPC1496) was determined using MSD. In brief, 96-well plates were coated with 50µL per well TNFα diluted to 1 µg/mL in PBS. The plate was incubated on the bench top at ambient temperature without shaking for 2 hours. The coating solution was removed and the plate was blocked with 50µL per well of 1% BSA in PBS, with 0.05% Polysorbate 20. The plate was incubated for 1 hour at 24°C with shaking at 400 rpm and then washed 4 times with wash buffer. The antibodies were diluted in 0.1 % BSA in PBS with 0.05% Polysorbate 20 and 30µl of each sample was added to the plate. The plate was incubated for 1 hour at 24°C with shaking at 400 rpm. The plate was then washed 4 times with wash buffer. Anti-human IgG sulfotag was diluted 1 in 5000 in assay buffer. 30µL was added to each well of the plate and then incubated for 1.5 hour at 24°C, with shaking at 400 rpm. The plate was then washed 4 times with wash buffer. The 4x MSD Read Buffer concentrate was diluted to 1x using deionised water. 100µL was then added per well of the plate. The plate was then read using the MSD Sector Imager instrument. From the signals obtained from the assay, specific activities of the molecules were calculated.

### Deamidation analysis

Deamidation is a common post-translational modification that can occur to asparagine and glutamine residues, but is most commonly observed with asparagine residues, particularly when adjacent to a glycine residue. In order to examine how susceptible these residues are and to determine the effects of deamidation on potency, adalimumab, BPC1494 and BPC1496 were exposed to a stress study. The stress was carried out by incubation in 1% ammonium bicarbonate at pH 9.0, for 48 hrs, conditions which have previously been shown to cause deamidation. The stressed samples were incubated alongside a control (in PBS) and were compared to this as well as an unstressed reference and analysed using c-IEF, SEC and Binding ELISA. Forced deamidation was also done on all samples in the presence and absence of EDTA. It has been shown previously that forced deamidation conditions cause fragmentation in addition to deamidation. EDTA prevents and or minimizes the fragmentation.

### Oxidation analysis

Oxidation of various residues can occur throughout the processing and storage of proteins; however the most commonly oxidised residue is methionine, which was the focus of this screen. Oxidation susceptibility of these residues was examined through exposure to stress conditions by incubation in 5mM and 50 mM H₂O₂ for 30minutes and evaluated using RP-HPLC, SEC and ELISA.

### Summary of results

Both BPC1494 and BPC1496 behave very favourably compared to adalimumab as shown by analytical comparability on both stressed and control samples. For all antibodies tested, no significant degradation was observed under forced oxidation conditions as shown by all analytical techniques employed. Significant deamidation as measured by c-IEF was observed at pH 9.0 as expected for all antibodies tested. In addition we saw significant fragmentation for all antibodies tested as shown by SEC at pH 9.0 in samples without EDTA, this is also as expected. There is a reduction in the pi value, (approximately 0.2) of BPC1494 when compared to adalimumab. This is attributed to the presence of an additional glutamic acid residue in the heavy chain sequence of the BPC1494 thus making it more acidic. Forced deamidation and oxidation had minimal impact on binding and this was observed for BPC1494, BPC1496 and adalimumab.

### Example 17: Analysis of binding of improved antibodies by ELISA

Antibodies BPC1499, 1500 and 1501 were assessed for binding activity by ELISA as described in Example 4. Using two different antigen coating concentrations (0.1 and 1.0 µg/ml), the antibodies did not show any difference in their binding profile when compared with BPC1492. Under the conditions tested, it appears that the ELISA does not discriminate between antibodies with different reported binding activities. The same antibodies were assessed using methodologies described in Examples 18, 5 and 6 which are considered more sensitive assays. In these assays, antibodies BPC1499, 1500 and 1501 show improved binding affinity and improved potency when compared with BPC1492.

### Example 18: Biacore Analysis of TNF alpha binding using a Capture surface

Protein A and anti-human IgG (GE Healthcare BR-1008-39) were coupled on separate flow cells on a CM3 biosensor chip. These surfaces were used to capture the antibodies for binding analysis. Recombinant human and cynomolgus TNF alpha were used as analytes at 64nM, 21.33nM, 7.11nM, 2.37nM, 0.79nM, an injection of buffer alone (i.e. 0nM) used to double reference the binding curves. Regeneration of the capture surface was carried out using 100mM phosphoric acid and 3M MgCl₂. The run was carried out on the Biacore T100 machine at 37°C using HBS-EP as running buffer. The constructs BPC1494 and BPC1496 showed reduced binding to Protein A and the anti-human IgG surface making these surfaces unsuitable for generating kinetics for those molecules.

**Table 9 Kinetic Analysis of Human and Cyno TNF alpha Binding to Captured Anti-TNF alpha Antibodies.**

| **Construct** | **Analyte** | **Capture Surface** | **ka(1/Ms)** | **kd(1/s)** | **KD(nM)** |
|---|---|---|---|---|---|
| BPC1492 | human TNFα | Protein A | 2.12E+06 | 1.10E-04 | 0.05196 |
| BPC1494 | human TNFα | Protein A | Data | not | Analysable |
| BPC1496 | human TNFα | Protein A | Data | not | Analysable |
| BPC1500 | human TNFα | Protein A | 2.68E+06 | 4.19E-05 | 0.01561 |
| BPC1492 | human TNFα | anti-human IgG | 6.78E+06 | 1.73E-04 | 0.02554 |
| BPC1494 | human TNFα | anti-human IgG | Data | not | Analysable |
| BPC1496 | human TNFα | anti-human IgG | Data | not | Analysable |
| BPC1500 | human TNFα | anti-human IgG | 4.51E+06 | 7.07E-05 | 0.01568 |
| BPC1492 | Cyno TNFα | Protein A | 1.10E+06 | 1.11E-04 | 0.101 |
| BPC1494 | Cyno TNFα | Protein A | Data | not | Analysable |
| BPC1496 | Cyno TNFα | Protein A | Data | not | Analysable |
| BPC1500 | Cyno TNFα | Protein A | 2.34E+06 | 3.51E-05 | 0.01503 |
| BPC1492 | Cyno TNFα | anti-human IgG | 1.96E+06 | 3.75E-04 | 0.1911 |
| BPC1494 | Cyno TNFα | anti-human IgG | Data | not | Analysable |
| BPC1496 | Cyno TNFα | anti-human IgG | Data | not | analysable |
| BPC1500 | Cyno TNFα | anti-human IgG | 4.48E+06 | 2.09E-04 | 0.04667 |

### Example 19: ProteOn Reverse Assay Binding Analysis

Biotinylated TNF alpha was mixed with biotinylated BSA at a 1:49 ratio, at a final total protein concentration of 20µg/ml (i.e. 0.4µg biotinylated TNF alpha and 19.6µg biotinylated BSA). This mixture was captured on a NLC biosensor chip (a single flowcell) (Biorad 176-5021). The chip surface was conditioned with 10mM glycine pH3.0 till a stable signal was achieved. The antibodies to be tested were used as analytes at 256nM, 64nM, 16nM, 4nM and 1nM and 0nM. The binding curves were referenced against a flowcell coated with biotinylated BSA alone. Regeneration was achieved using 10mM glycine pH3.0. Data was fitted to the 1:1 model inherent to the ProteOn analysis software.

**Table 10 Apparent Kinetics of Anti-TNF alpha antibodies binding to Neutravidin Captured TNF alpha**

| **BPC Number** | **ka (1/Ms)** | **kd (1/s)** | **KD (nM)** |
|---|---|---|---|
| BPC1499 | 2.27E+06 | 1.72E-05 | 0.008 |
| BPC1500 | 2.06E+06 | 3.00E-05 | 0.015 |
| BPC1501 | 1.17E+06 | 6.97E-05 | 0.06 |
| BPC1496 | 6.33E+05 | 4.04E-04 | 0.639 |
| BPC1494 | 7.23E+05 | 3.50E-04 | 0.484 |
| BPC1492 | 7.89E+05 | 3.21 E-04 | 0.407 |

This data is one set of two experiments which were carried out (second set not shown). The KD ranking of the data is representative of both data sets.

### Example 20: Construction of alternative antibodies which bind to human TNFα

The DNA expression constructs encoding additional variable heavy regions with modifications in the CDR regions (as described in Rajpal et al. PNAS (2005) 102(24): pg 8466-8471) were prepared de novo by build up of overlapping oligonucleotides and similar molecular biology techniques to those described in Example 1. Examples of DNA sequences encoding the variable heavy domains of these variant antibodies are given in SED IQ NO: 81, 83, 85, 87, 89, 91, 93 and 95. The DNA expression constructs encoding additional variable light domain regions with modifications in the CDR regions (as described in Rajpal et al. PNAS (2005) 102(24): pg 8466-8471) were prepared de novo by build up of overlapping oligonucleotides and similar molecular biology techniques to those described in Example 1. Examples of DNA sequences encoding the variable light domains of these variant antibodies are given in SED IQ NO: 97, 99, 101, 103, 105, 107, 109, 111, 113, 115, 117, 119, 121, 123, 125, 127, 129, 131, 133, 135 and 137. Once constructed, the expression plasmids encoding the heavy and light chains were transiently co-transfected into HEK 293 6E cells. Expressed antibody were purified from the supernatant and assessed for activity using the methods similar to those described in Example 6..

### Example 21: Construction of expression vectors for BPC2604 (Pascolizumab-YTE)

The pTT-based DNA expression constructs encoding the heavy chain of pascolizumab was engineered to include the following changes M252Y/S254T/T256E (EU index numbering) using the Quikchange protocol (Promega).

### Example 22: Expression/purification of Pasco and Pasco-YTE vectors

Expression plasmids encoding the heavy and light chains of BPC2604 were transiently co-transfected into HEK 293 6E cells. Expressed antibody was purified from the bulk supernatant using a two step purification carried out by affinity chromatography and SEC using a 5ml MabSelectSure column and Superdex 200 column on an AKTA Xpress.

### Example 23: BIAcore analysis of Pasco vs. Pasco YTE for FcRn binding

Antibodies were immobilised on a GLM chip (20µg/ml in acetate pH4.5) by primary amine coupling. Human, cynomolgus, rat and mouse FcRn receptors used at 2048, 512, 128, 32 and 8nM. 0nM used for double referencing. Assay were carried out in HBS-EP pH7.4 and HBS-EP pH6.0 (FcRn receptor diluted in appropriate running buffer for each pH. The surface was regenerated for FcRn binding with 200mM Tris pH9.0. Data was fitted to an equilibrium model, with R-max set to highest R-max obtained of any construct. The results are shown in Table 11 below and confirm that the YTE-modified pascolizumab (BPC2604) shows improved binding to FcRn at pH6.0 compared to pascolizumab.

**Table 11: Affinities of anti-IL-4 antibody constructs for Human and Cyno FcRn (n.a.b. is no analysable binding)**

| | | **KD (nM) at pH6.0: R-max = 1020** | | | | **KD (nM) at pH7.4: R-max = 1020** | | | |
|---|---|---|---|---|---|---|---|---|---|
| Antibody | Fc modification | Human FcRn | Cyno FcRn | Mouse FcRn | Rat FcRn | Human FcRn | Cyno FcRn | Mouse FcRn | Rat FcRn |
| BPC2604 | M252Y/S254T/T256E | 98 | 92.1 | 53.4 | 66.0 | 11600 | 11100 | 2160 | 4330 |
| Pascolizumab | None | 541 | 505 | 205 | 228 | n.a.b | n.a.b | n.a.b | n.a.b |

### Example 24: PK studies with Pasco vs. Pasco-YTE

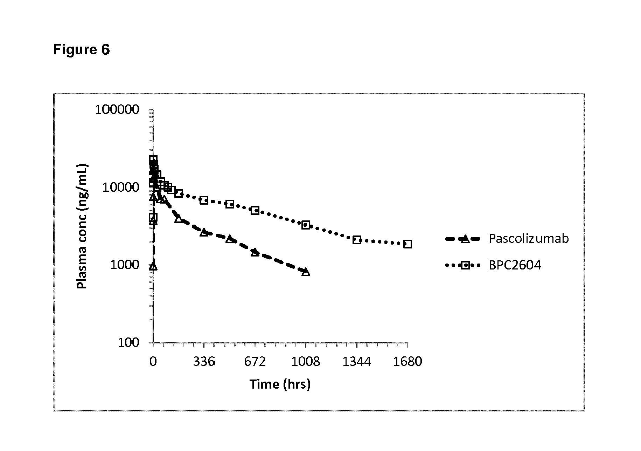

Figure 6 shows the average dose normalised plasma concentrations of pascolizumab-YTE (BPC2604)) in female cynomolgus monkeys and pascolizumab in male cynomolgus monkeys following a single intravenous (1 hr infusion) administration at a target dose of 1 mg/kg. The data for BPC2604 and pascolizumab were generated in separate studies. Plasma antibody concentrations for pascolizumab and BPC2604 were assessed by chemi-luminescence ELISA using IL-4 as the capture reagent and anti-human IgG (Fc specific)-HRP conjugate as the detection reagent. The validated range for the assay was 50-5000 ng/mL. The results are shown in Figure 6. Both compounds had similar Cmax but BPC2604 had a 3-fold lower plasma clearance resulting in 3-fold increase in AUC and 2-fold increase in half-life (T½).

### Example 25: Formulation studies at 5mg/ml

The stability of adalimumab and the TNF-alpha variant BPC1494 in two formulations was compared. Formulation 'A' (citrate-phosphate buffer) is the marketed adalimumab formulation made up of 6.16 mg/ml Sodium chloride + 0.30 mg/ml Sodium citrate monobasic + 1.30 mg/mL Citric acid monohydrate + 12 mg/ml Mannitol + 0.86mg/mL Monobasic sodium phosphate dihydrate + 1.53mg/mL Dibasic sodium phosphate dihydrate + 1.0 mg/ml PS80 at pH 5.2.

Formulation 'B' (acetate buffer) is composed of 6.81 mg/mL (50mM) Sodium Acetate trihydrate + 10mg/mL (1% w/v) Arginine + 0.0186mg/mL (0.05mM) EDTA + 2.98mg/mL (51mM) Sodium Chloride + 0.2mg/mL (0.02% w/v) Polysorbate 80, adjusted to pH 5.5 using HCl or NaOH.

The TNF-alpha variant BPC1494 material used in this study was made in a Chinese Hamster Ovary (CHO DG44) cell line and purified using a two step process involving mAb Select Sure followed by Superdex column 200pg. Adalimumab (Product code NDC 0074-3799-02, Lot number 91073LX40) manufactured by Abbott Laboratories was used.

Adalimumab was re-formulated into Formulation 'B' by overnight dialysis at 5°C using a 10KDa Slide - A - Lyzer cassette (Product Number 66830, Lot Number LJ150514); produced by Thermo Scientific (Rockford, IL ;USA). This experiment was carried out at a different time point to the other three formulations.Both Adalimumab in Formulations 'A' and 'B' were diluted to 5mg/mL using their respective formulation buffers. The TNF-alpha variant BPC1494 molecule was also formulated in Formulations A and B at ∼5mg/mL. A total of 4 samples were filtered through a MillexGV 0.22um filter under a clean laminar flow condition before being transferred into labelled pre-sterilized glass vials and incubated at 5°C, 25°C and 40°C for up to 14 weeks. Samples were taken at selected time points and analysed using SEC-HPLC (Table 12), cIEF (Table 13). Other assays as described below were also carried out to assess the stability of the antibodies.

### Appearance by visual observation

Samples were inspected for clarity under daylight conditions. Both antibodies in each formulation remained unchanged (clear colourless solution) after 14 weeks storage at 5°C, 25°C and 40°C.

### Protein concentration (A280nm) Measurement

Protein concentration was measured using a nanodrop spectrometer, which is indicative of protein stability. The extinction coefficient for adalimumab is 1.46 and for TNF-alpha variant BPC1494 is 1.48. There was no significant difference in the results after 14 weeks storage at 5°C, 25°C and 40°C.

### pH

pH was measured for all samples stored under different storage conditions to determine whether any significant pH drifts had occurred. All results remained within assay variability after 14 weeks storage at 5°C, 25°C and 40°C.

### Size exclusion chromatography (SEC)

This method separates soluble protein molecules in the solution based on size and not molecular weight. In theory, small molecules will penetrate every small pore of the stationary phase and hence will elute later. The chromatogram obtained enables the determination of percentage area of aggregates, monomer and low molecular weight (MW) fragments. The presence of aggregates and/or low molecular weight species is indicative of protein degradation. Increased stability corresponds to a high percentage of monomeric species (Mono) together with a low percentage of Total Aggregates (TA) and Total Low Molecular weight Fragments (TLMWF).

SEC-HPLC data (Table 12) shows that the TNF-alpha variant BPC1494 was relatively more stable in formulation 'B' compared to formulation A after storage at 25°C and 40°C for 14 weeks. Furthermore, TNF-alpha variant BPC1494 was relatively more stable, or at least as stable as adalimumab in formulation A. The results for adalimumab in formulation B are all within 5% TA and/or TLMWF. Therefore, formulation B has advantages over formulation A for both TNF-alpha variant BPC1494 and adalimumab.

For example, Table 12 shows that after storage at 25°C for 8 weeks, TNF-alpha variant BPC1494 in formulation A has 2.3% TLMWF while formulation B produced only 1.5%. Furthermore, TNF-alpha variant BPC1494 in formulation B was relatively more stable than adalimumab in formulation A (1.8% TLMWF). Similarly, at the 14 week time point at 25°C, 3.15% TLMWF was observed for TNF-alpha variant BPC1494 in formulation 'A' compared to 2.3% TLMWF in formulation 'B'. Furthermore, TNF-alpha variant BPC1494 in 'B' was relatively more stable than adalimumab in formulation A (3.4% TLMWF). A similar trend for TLMWF was observed for both molecules on incubation at 40°C for 4 weeks (adalimumab in 'A': 3.6%; TNF-alpha variant BPC1494 in 'A': 4.1%; TNF-alpha variant BPC1494 in `B': 2.6%).

Also, results for Total Aggregate (TA) show that at 14 weeks at 25°C, the TNF-alpha variant BPC1494 was relatively more stable in 'B' (0.3%) than in 'A' (0.5%); and relatively more stable than adalimumab in formulation A (0.4%).

### Capillary Iso-Electric Focusing (c-IEF)

This technique is used for determining the charge profile of molecules. A broad pl range reflects greater charge heterogeneity of the Product and in addition a broad pl range may be indicative of degradation. Typically the number of peaks will increase with increased degradation. The C-IEF data of Table 12 supports the SEC findings in Table 13.

The % area of main isoform (%AMI) was comparable between adalimumab in formulation A and TNF-alpha variant BPC1494 in formulation B at Weeks 8 and 14 at 25°C (56.0-57.7 and 53.2 respectively). At these time points and temperature, formulation B shows a slight advantage over 'A' for TNF-alpha variant BPC1494.

Similarly, adalimumab is relatively more stable in formulation 'B' than in formulation 'A' (see Week 4 data). For example, increased changes in charge heterogeneity (i.e. increase in number of peaks) were observed for adalimumab incubated for up to 4 weeks at 40°C in formulation 'A' compared to formulation 'B' (8 peaks and 6 peaks respectively). TNF-alpha variant BPC1494 showed a more consistent charge heterogeneity of 5 peaks at all timepoints and temperatures.

### Functional binding assay

The binding activity of adalimumab and TNF-alpha variant BPC1494 in the two formulations was assessed by Biacore. Over a 14 week period of storage at 5°C, 25°C and 40°C, the samples showed similar %binding within assay variability.

Hence, it can be concluded that formulation 'B' can serve as an alternative to formulation 'A' in a clinical setting without compromising the stability of the protein and potentially eliminating the pain associated with the marketed adalimumab formulation (A).

Importantly, this data shows that not only does the acetate formulation (B) improve the stability of the TNF-alpha variant BPC1494 compared to the citrate-phosphate formulation (A); but the acetate formulation is comparable or slightly better than the citrate-phosphate formulation when stabilising adalimumab.

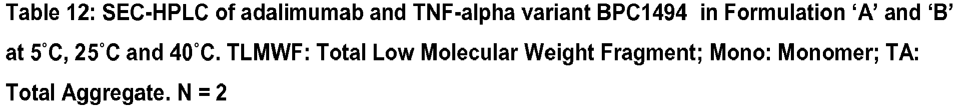

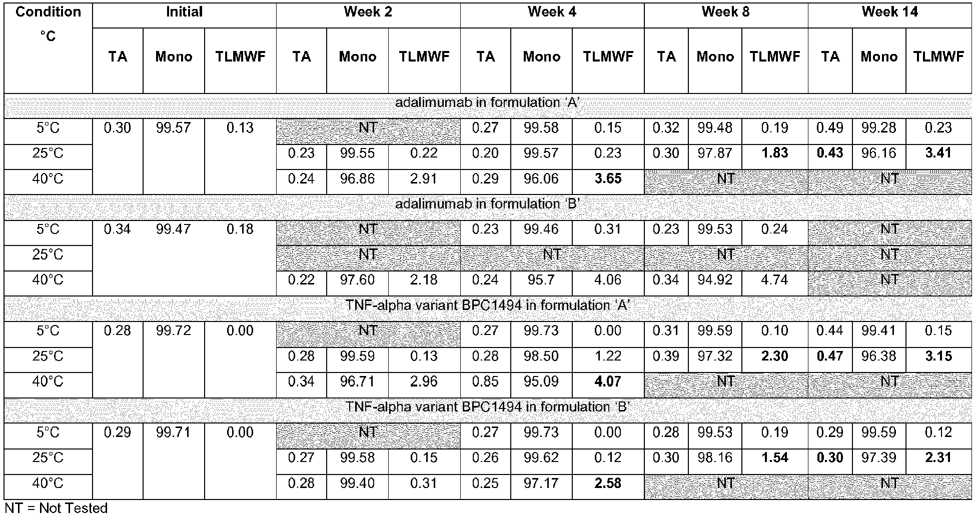

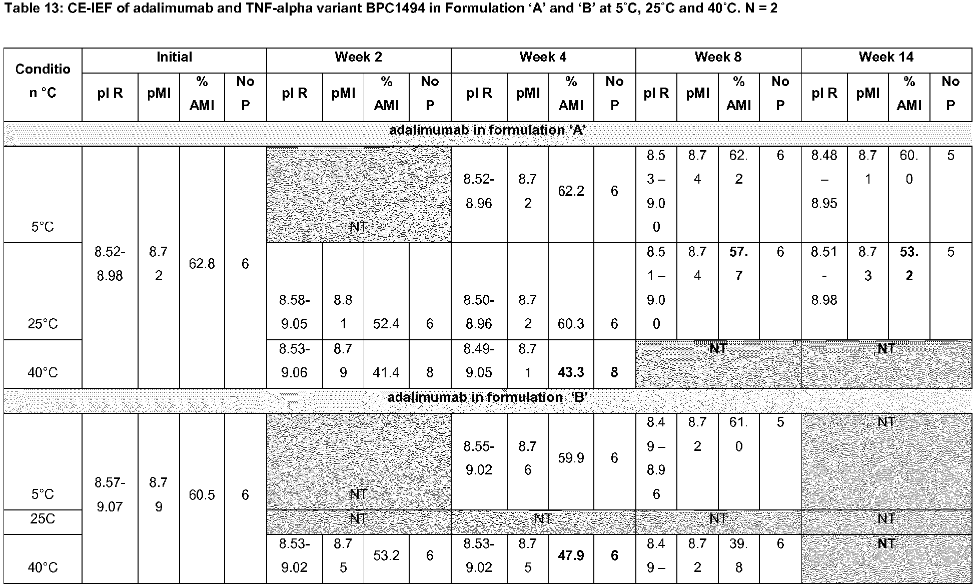

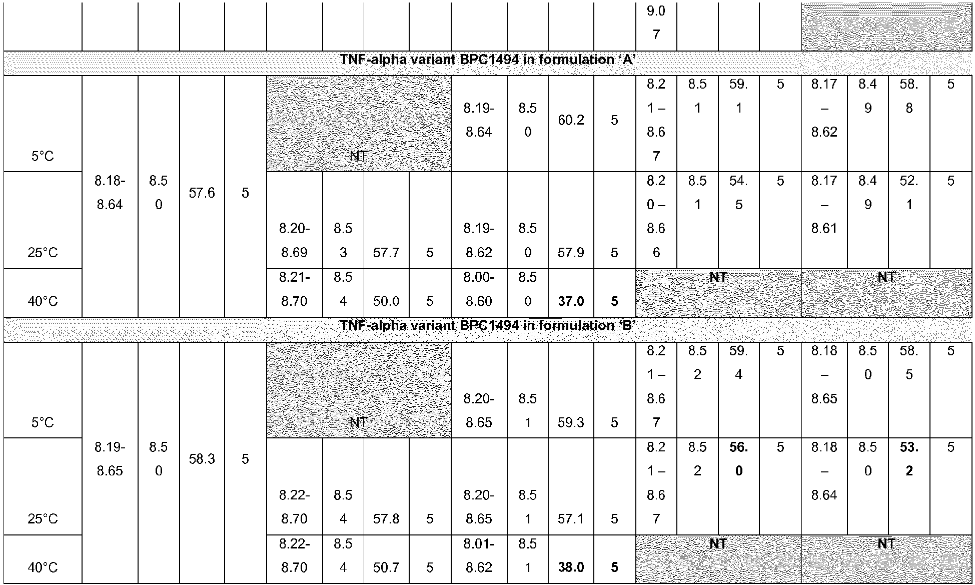

NT= Not Tested; pl R: Pi Range; pMl: pl of Main Isoform; % AMI: % Area Main Isoform; NoP: Number of Peaks.

### Example 26: Formulation studies at 50mg/ml

As shown in the previous example 25, adalimumab and TNF-alpha variant BPC1494 at 5mg/mL in formulation 'B' can serve as an alternative to formulation 'A'. This example is focused on comparing the stability of adalimumab in its marketed formulation 'A' compared to formulation 'B' and other TNF-alpha variants at 50mg/ml.

Two samples of TNF-alpha variant BPC1494 were analysed, one expressed in CHO DG44 cells and one expressed in CHOK1 cells. A second TNF-alpha variant BPC1496 was made in a CHO-DG44 cell line. All three samples were expressed and purified using mAb Select Sure. In contrast to Example 25, no Superdex column step was carried out. Adalimumab (Product code N 00515-01, Lot number 02136XH12) manufactured by Abbott Laboratories, as in Example 25. Adalimumab was formulated in formulations 'A' (as purchased) and 'B' (by buffer exchange) as described above in Example 25, and the TNF-alpha variants (BPC1494 and 1496) were formulated in 'B', all at ∼50mg/mL (total of 5 samples). The samples were filtered with MillexGV 0.22um filter under clean laminar flow conditions before being transferred into labelled pre-sterilized glass vials and incubated at 5°C and 40°C for up to 9 weeks. At selected time-points, samples were taken and analysed using SEC-HPLC (Table 14), cIEF (Table 15). Other assays as described below were also carried out.

### Appearance by visual observation.

Samples were observed for clarity under daylight conditions. Both antibodies in both formulations remained unchanged (clear colourless solution) after 9 weeks storage at 5°C and 40°C.

### Protein concentration (A280nm) Measurement

Protein concentration was measured using a nanodrop spectrometer, which is indicative of protein stability. There was no significant difference in the results after 9 weeks storage at 5°C and 40°C.

### Size exclusion chromatography (SEC)

SEC-HPLC data (Table 13) showed that adalimumab at 50mg/ml was relatively more stable in formulation 'B' compared to formulation 'A' after storage at 40°C for 9 weeks. Also, the TNF-alpha variants (BPC1494 and 1496) were relatively as stable or more stable in 'B' as adalimumab in 'B'. No comparison between the variants in 'A' and 'B' was carried out.

Note that the Initial TA levels for the TNF-alpha variants were relatively higher than for adalimumab. Therefore, the results include a % change column at the right hand side to compare the changes from Initial to Week 9 at 40°C. For example, table 13 shows that after 9 week storage, the percentage change in total low molecular weight fragment (TLMWF) in formulation 'B' was between 3.82-4.96% compared to 6.08% in formulation 'A'. Similarly, the monomer percentage change in formulation 'A' was greater for adalimumab than for 'B' (7.54 and 4.52% respectively). The TNF-alpha variants in 'B' were all relatively at least as stable or more stable as adalimumab in formulation 'A' (% change at Week 9). The results at week 4 for all samples are within the 5% TA and/or TLMWF allowance for a commercial product. Therefore, 'B' has advantages over 'A' for both TNF-alpha variants and adalimumab at 50 mg/ml.

In particular, the TNF-alpha variant BCP1496 showed a low TLMWF value of 3.86 at Week 9 at 40°C.

### Capillary Iso-Electric Focusing (c-IEF)

C-IEF data (Table 15) supports the findings in Table 14.

Formulation B shows a reduced % change of %AMI at week 9 for adalimumab as compared to Formulation A (23.53 and 27.57 respectively).

The TNF-alpha variants in 'B' are more stable in terms of charge heterogeneity (i.e. increase in number of peaks) than adalimumab (in both 'A' and 'B'). For example, at Week 9 there were 5 and 6 peaks for each of the variants; and 6 and 9 peaks for adalimumab, at 5°C and 40°C respectively.

In particular, the TNF-alpha variant BCP1496 and adalimumab, both in 'B', showed a low % change in %AMI at week 9 of 25.83 and 23.53 respectively. The relatively higher % change in %AMI at week 9 for the TNF-alpha variant BCP1496 (CHO DG44) of 38.13 may be due to the relatively high initial %AMI of 75.03.

### Functional binding assay (ELISA)

The biological activity of adalimumab and the TNF-alpha variants in the two formulations was assessed by Biacore. Over the 9 week period of storage at 5°C and 40°C, the samples showed the same %binding within assay variability.

Hence, it can be concluded that formulation 'B' can serve as an alternative to formulation 'A' in a clinical setting without compromising the stability of the antibody at 50mg/mL dosage strength.

**Table 14: SEC-HPLC of adalimumab and TNF-alpha variants BPC1494 and 1496 in Formulations 'A' and 'B' at 5°C, 25°C and 40°C. TLMWF - Total Low Molecular Weight Fragment; Mono - Monomer; TA- Total Aggregate. N = 2**

| Condition °C | Initial | | | Week 1 | | | Week 2 | | | Week 4 | | | Week 9 | | | % Change at Week 9 | | |
|---|---|---|---|---|---|---|---|---|---|---|---|---|---|---|---|---|---|---|
| | TA | Mono | TLMW F | TA | Mono | TLMW F | TA | Mono | TLMW F | TA | Mono | TLMW F | TA | Mono | TLMW F | TA | Mon o | TLMW F |
| **adalimumab in formulation 'A'** | | | | | | | | | | | | | | | | | | |
| 5°C | 0.30 | 99.62 | 0.08 | 0.30 | 99.54 | 0.16 | 0.32 | 99.53 | 0.15 | 0.29 | 99.51 | 0.21 | 0.34 | 99.49 | 0.17 | | | |
| 40°C | | | | 0.39 | 99.13 | 0.48 | 0.51 | 99.03 | 0.46 | 0.54 | 98.77 | 0.69 | 1.75 | 92.08 | 6.16 | 1.45 | 7.54 | 6.08 |

| **adalimumab in formulation 'B'** | | | | | | | | | | | | | | | | | | |
|---|---|---|---|---|---|---|---|---|---|---|---|---|---|---|---|---|---|---|
| 5°C | 0.42 | 99.45 | 0.13 | 0.34 | 99.49 | 0.17 | 0.38 | 99.45 | 0.16 | 0.35 | 99.42 | 0.23 | 0.38 | 99.44 | 0.18 | | | |
| 40°C | | | | 0.41 | 99.36 | 0.23 | 0.48 | 99.23 | 0.29 | 0.54 | 98.85 | 0.61 | 0.88 | 94.93 | 4.19 | 0.46 | 4.52 | 4.06 |

| TNF-alpha variant BPC1494 (CHO DG44) in formulation 'B' | | | | | | | | | | | | | | | | | | |
|---|---|---|---|---|---|---|---|---|---|---|---|---|---|---|---|---|---|---|
| 5°C | 2.76 | 97.13 | 0.11 | 2.65 | 97.25 | 0.09 | 3.29 | 96.45 | 0.25 | 2.66 | 97.18 | 0.16 | 2.83 | 97.05 | 0.12 | | | |
| 40°C | | | | 2.96 | 96.74 | 0.29 | 2.98 | 96.70 | 0.32 | 2.96 | 96.36 | 0.68 | 4.08 | 90.85 | 5.07 | 1.32 | 6.28 | 4.96 |

| **TNF-alpha variant BPC1494 (CHOK1) in formulation 'B'** | | | | | | | | | | | | | | | | | | |
|---|---|---|---|---|---|---|---|---|---|---|---|---|---|---|---|---|---|---|
| 5°C | 2.35 | 97.64 | 0.00 | 2.32 | 97.69 | 0.00 | 2.36 | 97.64 | 0.00 | 2.33 | 97.67 | 0.00 | 2.56 | 97.40 | 0.04 | | | |
| 40°C | | | | 2.67 | 97.09 | 0.24 | 2.79 | 96.99 | 0.22 | 3.17 | 96.20 | 0.63 | 4.91 | 90.22 | 4.88 | 2.56 | 7.42 | 4.88 |

| **TNF-alpha variant BPC1496 in formulation 'B'** | | | | | | | | | | | | | | | | | | |
|---|---|---|---|---|---|---|---|---|---|---|---|---|---|---|---|---|---|---|
| 5°C | 1.19 | 98.78 | 0.04 | 1.47 | 98.49 | 0.04 | 1.62 | 98.34 | 0.03 | 1.71 | 98.15 | 0.14 | 1.92 | 97.95 | 0.13 | | | |
| 40°C | | | | 1.75 | 97.99 | 0.26 | 1.46 | 98.27 | 0.27 | 1.40 | 98.05 | 0.54 | 2.64 | 93.50 | 3.86 | 1.45 | 5.28 | 3.82 |

**Table 15: CE-IEF of adalimumab and TNF-alpha variants BPC1494 and 1496 in Formulations 'A' and 'B' at 5°C, 25°C and 40°C. N = 2**

| **Condition °C** | **Initial** | | | | **Week 1** | | | | **Week 2** | | | | **Week 4** | | | | **Week 9** | | | | **% Change Week 9** |
|---|---|---|---|---|---|---|---|---|---|---|---|---|---|---|---|---|---|---|---|---|---|
| | **pl R** | **pMl** | **% AMI** | **NoP** | **pl R** | **pMl** | **% AMI** | **NoP** | **pl R** | **pMI** | **% AMI** | **NoP** | **pl R** | **pMI** | **% AMI** | **NoP** | **pl R** | **pMI** | **% AMI** | **NoP** | **% AMI** |
| **adalimumab in formulation 'A'** | | | | | | | | | | | | | | | | | | | | | |
| 5°C | 8.55-9.01 | 8.76 | 58.67 | 6 | 8.56-9.00 | 8.74 | 58.28 | 6 | 8.57-9.01 | 8.75 | 57.42 | 6 | 8.54-9.01 | 8.75 | 59.45 | 6 | 8.55-9.02 | 8.77 | 60.37 | 6 | |
| 40°C | | | | | 8.53-9.01 | 8.75 | 56.18 | 6 | 8.52-9.00 | 8.75 | 51.82 | 6 | 8.53-9.02 | 8.75 | 47.24 | 6 | 8.36-9.02 | 8.77 | 31.10 | 9 | 27.57 |
| **adalimumab in formulation 'B'** | | | | | | | | | | | | | | | | | | | | | |
| 5°C | 8.53-9.02 | 8.76 | 57.91 | 6 | 8.53-9.01 | 8.76 | 59.51 | 6 | 8.52-9.01 | 8.76 | 56.85 | 6 | 8.53-9.01 | 8.75 | 59.00 | 6 | 8.54-9.02 | 8.77 | 59.62 | 6 | |
| 40°C | | | | | 8.53-9.02 | 8.76 | 55.52 | 6 | 8.52-9.00 | 8.75 | 51.7 | 6 | 8.53-9.01 | 8.75 | 47.07 | 6 | 8.36-9.02 | 8.77 | 34.38 | 9 | 23.53 |
| **TNF-alpha variant BPC1494 (CHO DG44) in formulation 'B'** | | | | | | | | | | | | | | | | | | | | | |
| 5°C | 8.22-8.68 | 8.53 | 75.03 | 5 | 8.29-8.68 | 8.52 | 76.13 | 5 | 8.28-8.68 | 8.52 | 75.11 | 5 | 8.28-8.68 | 8.52 | 73.76 | 5 | 8.24-8.69 | 8.53 | 75.17 | 5 | |
| 40°C | | | | | 8.22-8.66 | 8.51 | 70.92 | 5 | 8.20-8.67 | 8.51 | 64.52 | 5 | 8.23-8.68 | 8.53 | 58.40 | 5 | 8.06-8.69 | 8.54 | 36.9 | 6 | 38.13 |
| **TNF-alpha variant BPC1494 (CHOK1) in formulation 'B'** | | | | | | | | | | | | | | | | | | | | | |
| 5°C | 8.23-8.68 | 8.53 | 63.75 | 5 | 8.22-8.68 | 8.53 | 63.69 | 5 | 8.22-8.67 | 8.52 | 63.37 | 5 | 8.22-8.68 | 8.52 | 62.32 | 5 | 8.24-8.70 | 8.53 | 63.88 | 5 | |
| 40°C | | | | | 8.21-8.66 | 8.51 | 60.57 | 5 | 8.21-8.65 | 8.51 | 56.21 | 5 | 8.22-8.67 | 8.52 | 50.64 | 5 | 8.06-8.68 | 8.54 | 30.66 | 6 | 33.09 |

| **TNF-alpha variant BPC1496 in formulation 'B'** | | | | | | | | | | | | | | | | | | | | | |
|---|---|---|---|---|---|---|---|---|---|---|---|---|---|---|---|---|---|---|---|---|---|
| 5°C | 8.53-8.89 | 8.75 | 65.48 | 5 | 8.52-8.88 | 8.74 | 64.01 | 5 | 8.51-8.88 | 8.74 | 67.57 | 5 | 8.52-8.88 | 8.75 | 67.06 | 5 | 8.53-8.89 | 8.76 | 68.75 | 5 | |
| 40°C | | | | | 8.51-8.87 | 8.73 | 60.52 | 5 | 8.51-8.87 | 8.74 | 57.82 | 5 | 8.51-8.87 | 8.74 | 51.37 | 5 | 8.36-8.88 | 8.76 | 39.65 | 6 | 25.83 |

| | | | | | | | | | | | | | | | | | | | | | |
|---|---|---|---|---|---|---|---|---|---|---|---|---|---|---|---|---|---|---|---|---|---|
| NT= Not Tested; pl R - Pi Range; pMl - pl of Main Isoform; % AMI - % Area Main Isoform; NoP - Number of Peaks. | | | | | | | | | | | | | | | | | | | | | |

### Example 27: Plasma concentrations of BPC1494 following subcutaneous administration in the male cynomolgus monkey

In a repeat dose pharmacokinetic study BPC1494 was administered sub-cutaneously weekly or biweekly for 4 weeks at 30 or 100 mg/kg to male cynomolgus monkeys. For group 2 (n=3), the animals were administered 2x 30mg/kg doses on day 1 (approximately 1 hour apart) followed by a single 30 mg/kg dose on days 8, 15 and 22. For group 3 (n=3), the animals were administered with 2x 30mg/kg doses on day 1 (approximately 1 hour apart) followed by a single 30 mg/kg dose on day 15. For group 4 (n=3), the animals were administered with 2x 100mg/kg doses on day 1 (approximately 1 hour apart) followed by a single 100 mg/kg dose on day 15. Plasma samples were taken at intervals throughout the dosing and recovery phases of the study.

Plasma samples were analyzed for BPC1494 using a qualified analytical method based on sample dilution followed by immunoassay analysis Plasma samples were analyzed for BPC1494 or BPC1492. The method used 10 µg/ml biotinylated recombinant human TNF-alpha as the capture antigen and a 1:100 dilution of AlexaFluor 647-labelled anti-human IgG (Fc specific) antibody as the detection antibody (G18-145). The lower limit of quantification (LLQ) for BPC1494 was 1 µg/mL using a 50 µL aliquot of 100-fold diluted monkey plasma with a higher limit of quantification (HLQ) of 100 µg/mL. The computer systems that were used on this study to acquire and quantify data included Gyrolab Workstation Version 5.2.0, Gyrolab Companion version 1.0 and SMS2000 version 2.3. PK analysis was performed by non-compartmental pharmacokinetic analysis using WinNonlin Enterprise Pheonix version 6.1.

Pharmacokinetic data is presented in Table 16 with parameters determined from last dose received on Week 4 to the time point (t) 840 hours post dosing for 30 mg/kg/week dose group (2) and last dose received on Week 3 to the time point (t)1008 hours post dosing for 30 & 100 mg/kg/biweekly dose groups (3 and 4).

**Table 16: Individual and Mean Pharmacokinetic Parameters for BPC1494 in the Male Cynomolgus Monkey Following Subcutaneous Dosing of BPC1494 at 30 mg/kg/week or 30 and 100 mg/kg/biweekly over a 4-Week Investigative Study**

| Dose (mg/kg/biweekly) | Animal Number | Pharmacokinetic Parameters b | | | | | | |
|---|---|---|---|---|---|---|---|---|
| | | AUC0-t (mg.h/mL) | Cmax (mg/mL) | Median Tmax (h) | t½ (h) | MRT (h) | Estimated c CL_F (mL/h/kg) | Estimated c Vz_F (mL/kg) |
| 30a | P12M-272 | 923 | 1.51 | 168 | 616 | 367 | 0.125 | 111 |
| | P12M-273 | 758 | 1.29 | 168 | 604 | 368 | 0.141 | 123 |
| | P12M-274d | 21.3 | 0.135 | 24 | 226 | 142 | 3.01 | 978 |
| | Mean | 841 (568) | 1.40 (0.977) | 168 | 610 (482) | 367 (292) | 0.133 (1.09) | 117 (404) |
| | | | | | | | | |
| 30 | P12M-275 | 743 | 1.08 | 24 | 420 | 419 | 0.115 | 69.5 |
| | P12M-276 | 538 | 2.31 | 48 | 197 | 307 | 0.141 | 40.2 |
| | P12M-277d | 239 | 1.09 | 24 | 123 | 189 | 0.217 | 38.6 |
| | Mean | 641 (507) | 1.70 (1.49) | 36 (24) | 309 (247) | 363 (305) | 0.128 (0.158) | 54.9 (49.4) |
| | | | | | | | | |
| 100 | P12M-278 | 2760 | 5.89 | 24 | 398 | 374 | 0.0998 | 57.3 |
| | P12M-279 | 2480 | 5.21 | 72 | 332 | 362 | 0.131 | 62.9 |
| | P12M-280 | 2080 | 4.10 | 72 | 331 | 364 | 0.123 | 58.8 |
| | Mean | 2440 | 5.07 | 72 | 354 | 367 | 0.118 | 59.7 |

| | | | | | | | | |
|---|---|---|---|---|---|---|---|---|
| a) Group 2 animals received 30 mg/kg weekly for 4 weeks b) Pharmacokinetic parameters determined from last dose received on Week 4 to the time point (t) 840 hours post dosing for 30 mg/kg/week and last dose received on Week 3 to the time point (t)1008 hours post dosing for 30 & 100 mg/kg/biweekly c) Cl_F and Vz_F are estimates due to elimination phase following multiple doses and steady state not yet achieved. Parameter estimates have been calculated from i) using AUC0-168 or 336, ii) extrapolation of data from week 1 based on half-life and iii) using total dose over the defined sampling with AUC0-inf d) Animal 274 and 277 excluded from mean pharmacokinetic calculations based on scientific judgment that these animals are likely to be exhibiting an anti-drug antibody response. | | | | | | | | |

Mean data shown in parentheses are inclusive of these animals.

### Example 28: SPR binding analysis of FcRn to Protein L captured anti-TNFα mAbs

The study was carried out using the ProteOnTM XPR36 (BioRadTM) biosensor machine, a surface plasmon based machine designed for label free kinetic/affinity measurements. Protein L was immobilised on a GLM chip (BioRad, Cat No: 176-5012) by primary amine coupling. This surface was then used to capture the humanised antibodies, human and cyno FcRn (both in-house materials) was then used as analytes at 2048nM, 512nM, 128nM, 32nM, and 8nM, an injection of buffer alone (i.e. 0nM) used to double reference the binding curves. Regeneration of the protein L surface was carried out using Glycine-HCl pH1.5. The assay was run at 25°C and run in HBS-EP pH7.4 and HBS-EP pH6.0 with human or cynomolgus FcRn diluted in appropriate buffer. Affinities were calculated using the Equilibrium model, inherent to the ProteOn analysis software, using a "Global R-max" for binding at pH6.0 and the R-max from binding at pH6.0 for affinity calculation at pH7.4. Since the binding curves did not reach saturation at pH7.4, the values obtained are unlikely to be true affinities however were used to rank the binding of the antibodies tested.

The binding affinity of different batches of BPC1492, BPC1494 and BPC1496 for human FcRn was compared using antibodies captures by Protein L. Table 17shows the results from a series of experiments using this format. The data confirms that BPC1494 and BPC1496 have an improved affinity for recombinant human FcRn compared to BPC1492 at both pH6.0 and pH7.4. The fold improvement in binding affinity of BPC1494 for FcRn compared to BPC1492 differs from experiment to experiment due to changes in the Protein L activity on the capture. However, in the experiments shown in Table 17, the fold improvement in binding affinity at pH6.0 ranges between 3.5-fold and 16.3-fold. It was not possible to determine the fold improvement in binding affinity at pH7.4 due to the weak binding activity of human IgG for FcRn at neutral pH.

The binding affinity of different batches of BPC1492, BPC1494 and BPC1496 for cynomolgus FcRn was also compared using antibodies captured with Protein L. Table 18 shows the results from the experiment using this format. The data confirms that BPC1494 has an improved affinity for recombinant cynomolgus FcRn compared to BPC1492 at both pH6.0 and pH7.4. The fold improvement in binding affinity of BPC1494 (range 41.8-46.8nM) for cynomolgus FcRn compared to BPC1492 (range 394-398nM) is approximately 9-fold at pH6. It was not possible to determine the fold improvement in binding affinity at pH7.4 due to the weak binding activity of BPC1492 for FcRn.

**Table 17 Recombinant human FcRn binding affinities using the Protein L capture method**

| | | Affinity KD (nM) | | | | | | | | |
|---|---|---|---|---|---|---|---|---|---|---|
| | | BPC1492 | | | BPC1494 | | | BPC1496 | | |
| | | Batch | | | Batch | | | Batch | | |
| Expt. | pH | HEK | HEK | CHO | HEK | HEK | GRITS | HEX | HEK | GRITS |

| | | 1406 | 1348 | clinical grade | 1407 | 1350 | 42954 | 1352 | 1408 | 42955 |
|---|---|---|---|---|---|---|---|---|---|---|
| 5 | 6 | 320. 0 | 325. 0 | 315.0 | 6.08* * | 24.9 | 26.2 | 14.3 | 16.9 | 15.4 |
| | 7.4 | NAB | NAB | NAB | 2020 ** | 1260 0 | 11700 | 8980 | 9830 | 9670 |
| 4 | 6 | 50.9 | 54.8 | 55.5 | 1.33 | 4.05 | 4.50 | 2.35 | 3.60 | 2.33 |
| | 7.4 | NAB | NAB | NAB | 303 | 5270 | 4740 | 6820 | 7550 | 7550 |
| 3 | 6 | 16.0 | 16.8 | 17.3 | 0.70 1 | 1.96 0 | 2.430 | 2.20 0 | 4.14 0 | 1.810 |
| | 7.4 | NAB | NAB | NAB | 1760 | 1050 0 | 10900 | 7830 | 8050 | 8460 |
| 2 | 6 | 13.1 | 12.9 | 13.9 | ## | 0.35 9 | 0.979 | 0.97 8 | 2.44 0 | 0.546 |
| | 7.4 | NAB | NAB | NAB | 2010 | 9190 | 9330 | 1090 0 | 9480 | 9550 |
| 1 | 6 | ND | 234 | ND | ND | 66 | ND | ND | 85 | ND |
| | 7.4 | ND | NAB | ND | ND | NAB | ND | ND | 2010 | ND |

| | | | | | | | | | | |
|---|---|---|---|---|---|---|---|---|---|---|
| ** - although data points have been reported, the values should be treated with caution because these data are not consistent with the data obtained for the other batches of the same molecule during this experiment NAB = no analysable binding ND = not tested in this experiment ## = high affinity binding - beyond the sensitivity of the machine | | | | | | | | | | |

**Table 18 Recombinant cynomolgus FcRn binding affinities using the Protein L capture method**

| Batch number | Construct | pH6 KD (nM) | pH7.4 KD (nM) |
|---|---|---|---|
| GRITS44463 | BPC1494 | 46.8 | 14800 |
| MCB16Marc2012 | BPC1494 | 41.8 | 13300 |
| GRITS42954 | BPC1494 | 43.2 | 13700 |
| Clinical grade | BPC1492 | 394 | No binding |
| GRITS44348 | BPC1492 | 398 | No binding |

**Table A**

| **Description** | **Sequence identifier (SEQ ID NO)** | |
|---|---|---|
| | **Polynucleotide** | **Amino acid** |
| Anti-TNF antibody light chain | 1 | 2 |
| Anti-TNF antibody variable domain (VL) | - | 3 |
| anti-TNF antibody heavy chain plus M252Y/S254T/T256E modification | 4 | 5 |
| Anti-TNF antibody heavy variable domain (VH) | - | 6 |
| IgG1 constant domain plus M252Y/S254T/T256E modification | - | 7 |
| Anti-TNF antibody heavy chain plus M428L/N434S modification | 8 | 9 |
| IgG1 constant domain plus M428L/N434S modification | - | 10 |
| Anti-TNF antibody heavy chain (wild-type IgG1) | 11 | 12 |
| IgG1 constant domain (wild-type) | - | 13 |
| Anti-TNF antibody heavy chain plus T250Q/M428L modification | 14 | 15 |
| IgG1 constant domain plus T250Q/M428L modification | - | 16 |
| Anti-TNF antibody heavy chain plus V308F modification | 17 | 18 |
| IgG1 constant domain plus V308F modification | - | 19 |
| Anti-TNF antibody heavy chain plus V259I modification | 20 | 21 |
| IgG1 constant domain plus V259I modification | - | 22 |
| Anti-TNF antibody heavy chain plus P257L and N434Y variant | 23 | 24 |
| IgG1 constant domain plus P257L and N434Y modification | - | 25 |
| Signal peptide sequence | - | 26 |
| Anti-TNF antibody CDRH1 | - | 27 |
| Anti-TNF antibody CDRH2 | - | 28 |
| Anti-TNF antibody CDRH3 | - | 29 |
| Anti-TNF antibody CDRL1 | - | 30 |
| Anti-TNF antibody CDRL2 | - | 31 |
| Anti-TNF antibody CDRL3 | - | 32 |
| Anti-TNF antibody CDRH1 variant | - | 33-38 |
| Cimzia (certolizumab) LC (VL + Ck | | 39 |
| Anti-TNF antibody CDRH3 variant | - | 40-49 |
| Anti-TNF antibody CDRL1 variant | - | 50-61 |
| Anti-TNF antibody CDRL2 variant | - | 62-72 |
| Anti-TNF antibody CDRL3 variant | - | 73-76 |
| cb1-3-VH | 77 | 78 |
| cb2-44-VH | 79 | 80 |
| cb1-39-VH | 81 | 82 |
| cb1-31-VH | 83 | 84 |
| cb2-11-VH | 85 | 86 |
| cb2-40-VH | 87 | 88 |
| cb2-35-VH | 89 | 90 |
| cb2-28-VH | 91 | 92 |
| cb2-38-VH | 93 | 94 |
| cb2-20-VH | 95 | 96 |
| cb1-8-VL | 97 | 98 |
| cb1-43-VL | 99 | 100 |
| cb1-45-VL | 101 | 102 |
| cb1-4-VL | 103 | 104 |
| cb1-41-VL | 105 | 106 |
| cb1-37-VL | 107 | 108 |
| cb1-39-VL | 109 | 110 |
| cb1-33-VL | 111 | 112 |
| cb1-35-VL | 113 | 114 |
| cb1-31-VL | 115 | 116 |
| cb1-29-VL | 117 | 118 |
| cb1-22-VL | 119 | 120 |
| cb1-23-VL | 121 | 122 |
| cb1-12-VL | 123 | 124 |
| cb1-10-VL | 125 | 126 |
| cb2-1-VL | 127 | 128 |
| cb2-11-VL | 129 | 130 |
| cb2-40-VL | 131 | 132 |
| cb2-35-VL | 133 | 134 |
| cb2-28-VL | 135 | 136 |
| cb2-20-VL | 137 | 138 |
| cb1-3-VL | 139 | 140 |
| cb2-6-VL | 141 | 142 |
| cb2-44-VL | 143 | 144 |
| Anti-TNF antibody heavy chain variant cb1-3-VH plus M252Y/S254T/T256E modification | - | 145 |
| | | |
| Anti-TNF antibody heavy chain variant cb2-44-VH plus M252Y/S254T/T256E modification | - | 146 |
| Anti-TNF antibody light chain variant cb1-3-VL | 147 | 148 |
| Anti-TNF antibody light chain variant cb2-6-VL | 149 | 150 |
| Anti-TNF antibody light chain variant cb2-44-VL | 151 | 152 |
| Anti-TNF antibody heavy chain variant cb1-3-VH | 153 | 154 |
| Anti-TNF antibody heavy chain variant cb2-44-VH | 155 | 156 |
| Pascolizumab heavy chain containing the M252Y/S254T/T256E modifications | 157 | 158 |
| Pascolizumab light chain | 159 | 160 |
| Pascolizumab heavy chain | - | 161 |
| Alternative anti-TNF antibody heavy chain plus M428L/N434S modification | | 162 |
| Alternative IgG1 constant domain plus M428L/N434S modification | | 163 |
| Anti-TNF antibody heavy chain plus H433K/N434F modification | | 164 |
| IgG1 constant domain plus H433K/N434F modification | | 165 |
| Alternative anti-TNF antibody heavy chain plus H433K/N434F modification | | 166 |
| Alternative IgG1 constant domain plus H433K/N434F modification | | 167 |
| Alternative anti-TNF antibody heavy chain plus M428L/N434S modification | | 168 |
| Alternative IgG1/2 constant domain plus M428L/N434S modification | | 169 |
| Golimumab_VH | | 170 |
| Golimumab_VL | | 171 |
| Golimumab HC | | 172 |
| Golimumab LC | | 173 |
| Remicade VH | | 174 |
| Remicade VL | | 175 |
| Remicade HC | | 176 |
| Remicade LC | | 177 |
| Cimzia (certolizumab) VH | | 178 |
| Cimzia (certolizumab) VL | | 179 |
| Cimzia (certolizumab) HC (VH+CH1) | | 180 |

### Sequence listing

SEQ ID NO: 1 Polynucleotide sequence of the anti-TNF antibody light chain 
GATATCCAGATGACCCAGAGCCCCAGCAGCCTGAGCGCCTCTGTGGGCGATAGAGTGACCA
TCACCTGCCGGGCCAGCCAGGGCATCAGAAACTACCTGGCCTGGTATCAGCAGAAGCCTG
GCAAGGCCCCTAAGCTGCTGATCTACGCCGCCAGCACCCTGCAGAGCGGCGTGCCCAGCA
GATTCAGCGGCAGCGGCTCCGGCACCGACTTCACCCTGACCATCAGCAGCCTGCAGCCCG
AGGACGTGGCCACCTACTACTGCCAGCGGTACAACAGAGCCCCTTACACCTTCGGCCAGGG
CACCAAGGTGGAGATCAAGCGTACGGTGGCCGCCCCCAGCGTGTTCATCTTCCCCCCCAGC
GATGAGCAGCTCAAGAGCGGCACCGCCAGCGTGGTGTGTCTGCTGAACAACTTCTACCCCC
GGGAGGCCAAAGTGCAGTGGAAAGTGGACAACGCCCTGCAGAGCGGCAACAGCCAGGAGA
GCGTGACCGAGCAGGACAGCAAGGACTCCACCTACAGCCTGAGCAGCACCCTGACCCTGA
GCAAGGCCGACTACGAGAAGCACAAAGTGTACGCCTGCGAAGTGACCCACCAGGGCCTGT
CCAGCCCCGTGACCAAGAGCTTCAACCGGGGCGAGTGC
SEQ ID NO: 2 Protein sequence of the anti-TNF antibody light chain 
DIQMTQSPSSLSASVGDRVTITCRASQGIRNYLAWYQQKPGKAPKLLIYAASTLQSGVPSRFSGS
GSGTDFTLTISSLQPEDVATYYCQRYNRAPYTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 3 Protein sequence of the anti-TNF antibody variable domain (VL) 
DIQMTQSPSSLSASVGDRVTITCRASQGIRNYLAWYQQKPGKAPKLLIYAASTLQSGVPSRFSGS
GSGTDFTLTISSLQPEDVATYYCQRYNRAPYTFGQGTKVEIKRT
SEQ ID NO: 4 Polynucleotide sequence of the anti-TNF antibody heavy chain plus M252Y/S254T/T256E modification 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGTACATCACCAGAGAGCC
CGAGGTGACCTGTGTGGTGGTGGATGTGAGCCACGAGGACCCTGAGGTGAAGTTCAACTG
 
GTACGTGGACGGCGTGGAGGTGCACAATGCCAAGACCAAGCCCAGGGAGGAGCAGTACAA
CAGCACCTACCGGGTGGTGTCCGTGCTGACCGTGCTGCACCAGGATTGGCTGAACGGCAA
GGAGTACAAGTGTAAGGTGTCCAACAAGGCCCTGCCTGCCCCTATCGAGAAAACCATCAGC
AAGGCCAAGGGCCAGCCCAGAGAGCCCCAGGTGTACACCCTGCCCCCTAGCAGAGATGAG
CTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGC
TGGACAGCGATGGCAGCTTCTTCCTGTACAGCAAGCTGACCGTGGACAAGAGCAGATGGCA
GCAGGGCAACGTGTTCAGCTGCTCCGTGATGCACGAGGCCCTGCACAATCACTACACCCAG
AAGAGCCTGAGCCTGTCCCCTGGCAAG
SEQ ID NO: 5 Protein sequence of the anti-TNF antibody heavy chain plus M252Y/S254T/T256E modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
YITREPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 6 Protein sequence of the anti-TNF antibody heavy variable domain (VH)
SEQ ID NO: 7 Protein sequence of the IgG1 constant domain plus M252Y/S254T/T256E modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLYITREPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQ
KSLSLSPGK
SEQ ID NO: 8 Polynucleotide sequence of the anti-TNF antibody heavy chain plus M428L/N434S modification 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
 
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTG
GTACGTGGACGGCGTGGAGGTGCACAATGCCAAGACCAAGCCCAGGGAGGAGCAGTACAA
CAGCACCTACCGGGTGGTGTCCGTGCTGACCGTGCTGCACCAGGATTGGCTGAACGGCAA
GGAGTACAAGTGTAAGGTGTCCAACAAGGCCCTGCCTGCCCCTATCGAGAAAACCATCAGC
AAGGCCAAGGGCCAGCCCAGAGAGCCCCAGGTGTACACCCTGCCCCCTAGCAGAGATGAG
CTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGC
TGGACAGCGATGGCAGCTTCTTCCTGTACAGCAAGCTGACCGTGGACAAGAGCAGATGGCA
GCAGGGCAACGTGTTCAGCTGCTCCGTGCTGCACGAGGCCCTGCACAGCCACTACACCCA
GAAGAGCCTGAGCCTGTCCCCTGGCAAG
SEQ ID NO: 9 Protein sequence of the anti-TNF antibody heavy chain plus M428L/N434S modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVLHEALHSHYTQKSLSL
SPGK
SEQ ID NO: 10 Protein sequence of the IgG1 constant domain plus M428L/N434S modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVLHEALHSHYTQ
KSLSLSPGK
SEQ ID NO: 11 Polynucleotide sequence of the anti-TNF antibody heavy chain (wild-type IgG1) 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
 
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTG
GTACGTGGACGGCGTGGAGGTGCACAATGCCAAGACCAAGCCCAGGGAGGAGCAGTACAA
CAGCACCTACCGGGTGGTGTCCGTGCTGACCGTGCTGCACCAGGATTGGCTGAACGGCAA
GGAGTACAAGTGTAAGGTGTCCAACAAGGCCCTGCCTGCCCCTATCGAGAAAACCATCAGC
AAGGCCAAGGGCCAGCCCAGAGAGCCCCAGGTGTACACCCTGCCCCCTAGCAGAGATGAG
CTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGC
TGGACAGCGATGGCAGCTTCTTCCTGTACAGCAAGCTGACCGTGGACAAGAGCAGATGGCA
GCAGGGCAACGTGTTCAGCTGCTCCGTGATGCACGAGGCCCTGCACAATCACTACACCCAG
AAGAGCCTGAGCCTGTCCCCTGGCAAG
SEQ ID NO: 12 Protein sequence of the anti-TNF antibody heavy chain (wild-type IgG1) 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 13 Protein sequence of the IgG1 constant domain (wild-type)
SEQ ID NO: 14 Polynucleotide sequence of the anti-TNF antibody heavy chain plus T250Q/M428L modification 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACcaaCTGATGATCAGCAGAACCCCC
GAGGTGACCTGTGTGGTGGTGGATGTGAGCCACGAGGACCCTGAGGTGAAGTTCAACTGGT
ACGTGGACGGCGTGGAGGTGCACAATGCCAAGACCAAGCCCAGGGAGGAGCAGTACAACA
GCACCTACCGGGTGGTGTCCGTGCTGACCGTGCTGCACCAGGATTGGCTGAACGGCAAGG
AGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCT
GACCAAGAACCAGGTGTCCCTGACCTGCCTGGTGAAGGGCTTCTACCCCAGCGACATCGCC
GTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGC
AGGGCAACGTGTTCAGCTGCTCCGTGtTGCACGAGGCCCTGCACAATCACTACACCCAGAA
GAGCCTGAGCCTGTCCCCTGGCAAG
SEQ ID NO: 15 Protein sequence of the anti-TNF antibody heavy chain plus T250Q/M428L modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDQL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVLHEALHNHYTQKSLSL
SPGK
SEQ ID NO: 16 Protein sequence of the IgG1 constant domain plus T250Q/M428L modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDQLMISRTPEVTCVVVDVSHEDPEVKFNWYVDGVEVHNAKTKPREEQYNSTYRVVSVLTVLH
 
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVLHEALHNHYTQ
KSLSLSPGK
SEQ ID NO: 17 Polynucleotide sequence of the anti-TNF antibody heavy chain plus V308F modification 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTG
GTACGTGGACGGCGTGGAGGTGCACAATGCCAAGACCAAGCCCAGGGAGGAGCAGTACAA
CAGCACCTACCGGGTGGTGTCCGTGCTGACCtTcCTGCACCAGGATTGGCTGAACGGCAAG
GAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGC
TGACCAAGAACCAGGTGTCCCTGACCTGCCTGGTGAAGGGCTTCTACCCCAGCGACATCGC
CGTGGAGTGGGAGAGCAACGGCCAGCCCGAGAACAACTACAAGACCACCCCCCCTGTGCT
GGACAGCGATGGCAGCTTCTTCCTGTACAGCAAGCTGACCGTGGACAAGAGCAGATGGCAG
CAGGGCAACGTGTTCAGCTGCTCCGTGATGCACGAGGCCCTGCACAATCACTACACCCAGA
AGAGCCTGAGCCTGTCCCCTGGCAAG
SEQ ID NO: 18 Protein sequence of the anti-TNF antibody heavy chain plus V308F modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTFLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 19 Protein sequence of the IgG1 constant domains plus V308F modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTFLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQ
KSLSLSPGK
SEQ ID NO: 20 Polynucleotide sequence of the anti-TNF antibody heavy chain plus V259I modification 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCC
CGAGATCACCTGTGTGGTGGTGGATGTGAGCCACGAGGACCCTGAAGTGAAGTTCAACTGG
TACGTGGACGGCGTGGAGGTGCACAATGCCAAGACCAAGCCCAGGGAGGAGCAGTACAAC
AGCACCTACCGGGTGGTGTCCGTGCTGACCGTGCTGCACCAGGATTGGCTGAACGGCAAG
GAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGC
TGACCAAGAACCAGGTGTCCCTGACCTGCCTGGTGAAGGGCTTCTACCCCAGCGACATCGC
CGTGGAGTGGGAGAGCAACGGCCAGCCCGAGAACAACTACAAGACCACCCCCCCTGTGCT
GGACAGCGATGGCAGCTTCTTCCTGTACAGCAAGCTGACCGTGGACAAGAGCAGATGGCAG
CAGGGCAACGTGTTCAGCTGCTCCGTGATGCACGAGGCCCTGCACAATCACTACACCCAGA
AGAGCCTGAGCCTGTCCCCTGGCAAG
SEQ ID NO: 21 Protein sequence of the anti-TNF antibody heavy chain plus V259I modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEITCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVE
 
WESNGQPENNYKTTPPVLDSDGSFFLYSKLTVDKSRWQQGNVFSCSVMHEALHNHYTQKSLSL
SPGK
SEQ ID NO: 22 Protein sequence of the IgG1 constant domains plus V259I modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEITCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQ
KSLSLSPGK
SEQ ID NO: 23 Polynucleotide sequence of the anti-TNF antibody heavy chain plus P257L and N434Y variant 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCT
GGAGGTGACCTGTGTGGTGGTGGATGTGAGCCACGAGGACCCTGAGGTGAAGTTCAACTG
GTACGTGGACGGCGTGGAGGTGCACAATGCCAAGACCAAGCCCAGGGAGGAGCAGTACAA
CAGCACCTACCGGGTGGTGTCCGTGCTGACCGTGCTGCACCAGGATTGGCTGAACGGCAA
GGAGTACAAGTGTAAGGTGTCCAACAAGGCCCTGCCTGCCCCTATCGAGAAAACCATCAGC
AAGGCCAAGGGCCAGCCCAGAGAGCCCCAGGTGTACACCCTGCCCCCTAGCAGAGATGAG
CTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGC
TGGACAGCGATGGCAGCTTCTTCCTGTACAGCAAGCTGACCGTGGACAAGAGCAGATGGCA
GCAGGGCAACGTGTTCAGCTGCTCCGTGATGCACGAGGCCCTGCACTATCACTACACCCAG
AAGAGCCTGAGCCTGTCCCCTGGCAAG
SEQ ID NO: 24 Protein sequence of the anti-TNF antibody heavy chain plus P257L and N434Y modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTLEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHYHYTQKSLSL
SPGK
SEQ ID NO: 25 Protein sequence of the IgG1 constant domains plus P257L and N434Y modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTLEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHYHYTQ
KSLSLSPGK
SEQ ID NO: 26 Signal peptide sequence
   MGWSCIILFLVATATGVHS
SEQ ID NO: 27 anti-TNF antibody CDRH1
   DYAMH
SEQ ID NO: 28 anti-TNF antibody CDRH2
   AITWNSGHIDYADSVEG
SEQ ID NO: 29 anti-TNF antibody CDRH3
   VSYLSTASSLDY
SEQ ID NO: 30 anti-TNF antibody CDRL1
   RASQGIRNYLA
SEQ ID NO: 31 anti-TNF antibody CDRL2
   AASTLQS
SEQ ID NO: 32 anti-TNF antibody CDRL3
   QRYNRAPYT
SEQ ID NO: 33 anti-TNF antibody CDRH1 variant
   QYAMH
SEQ ID NO: 34 anti-TNF antibody CDRH1 variant
   HYALH
SEQ ID NO: 35 anti-TNF antibody CDRH1 variant
   HYAMH
SEQ ID NO: 36 anti-TNF antibody CDRH1 variant
   QHALH
SEQ ID NO: 37 anti-TNF antibody CDRH1 variant
   QHAMH
SEQ ID NO: 38 anti-TNF antibody CDRH1 variant
   DHALH
SEQ ID NO: 39 Cimzia (certolizumab) LC (VL + Ck) 
DIQMTQSPSSLSASVGDRVTITCKASQNVGTNVAWYQQKPGKAPKALIYSASFLYSGVPYRFSG
SGSGTDFTLTISSLQPEDFATYYCQQYNIYPLTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 40 anti-TNF antibody CDRH3 variant
   VHYLSTASQLHH
SEQ ID NO: 41 anti-TNF antibody CDRH3 variant
   VQYLSTASSLQS
SEQ ID NO: 42 anti-TNF antibody CDRH3 variant
   VKYLSTASSLHY
SEQ ID NO: 43 anti-TNF antibody CDRH3 variant
   VKYLSTASNLES
SEQ ID NO: 44 anti-TNF antibody CDRH3 variant
   VHYLSTASSLDY
SEQ ID NO: 45 anti-TNF antibody CDRH3 variant
   VSYLSTASSLQS
SEQ ID NO: 46 anti-TNF antibody CDRH3 variant
   VRYLSTASNLQH
SEQ ID NO: 47 anti-TNF antibody CDRH3 variant
   VQYLSTASQLHS
SEQ ID NO: 48 anti-TNF antibody CDRH3 variant
   VRYLSTASQLDY
SEQ ID NO: 49 anti-TNF antibody CDRH3 variant
   VRYLSTASSLDY
SEQ ID NO: 50 anti-TNF antibody CDRL1 variant
   HASKKIRNYLA
SEQ ID NO: 51 anti-TNF antibody CDRL1 variant
   HASRKLRNYLA
SEQ ID NO: 52 anti-TNF antibody CDRL1 variant
   HASRRLRNYLA
SEQ ID NO: 53 anti-TNF antibody CDRL1 variant
   HASKRIRNYLA
SEQ ID NO: 54 anti-TNF antibody CDRL1 variant
   HASRKIRNYLA
SEQ ID NO: 55 anti-TNF antibody CDRL1 variant
   HASRRIRNYLA
SEQ ID NO: 56 anti-TNF antibody CDRL1 variant
   HASREIRNYLA
SEQ ID NO: 57 anti-TNF antibody CDRL1 variant
   HASQGIRNYLA
SEQ ID NO: 58 anti-TNF antibody CDRL1 variant
   HASQKIRNYLA
SEQ ID NO: 59 anti-TNF antibody CDRL1 variant
   RASRGLRNYLA
SEQ ID NO: 60 anti-TNF antibody CDRL1 variant
   HASQRIRNYLA
SEQ ID NO: 61 anti-TNF antibody CDRL1 variant
   RASRRIRNYLA
SEQ ID NO: 62 anti-TNF antibody CDRL2 variant
   AASSLLR
SEQ ID NO: 63 anti-TNF antibody CDRL2 variant
   AASSLLK
SEQ ID NO: 64 anti-TNF antibody CDRL2 variant
   AASSLLP
SEQ ID NO: 65 anti-TNF antibody CDRL2 variant
   AASSLQP
SEQ ID NO: 66 anti-TNF antibody CDRL2 variant
   AASSLLH
SEQ ID NO: 67 anti-TNF antibody CDRL2 variant
   AASSFLP
SEQ ID NO: 68 anti-TNF antibody CDRL2 variant
   AASSLLQ
SEQ ID NO: 69 anti-TNF antibody CDRL2 variant
   AASSLQQ
SEQ ID NO: 70 anti-TNF antibody CDRL2 variant
   AASTLLK
SEQ ID NO: 71 anti-TNF antibody CDRL2 variant
   AASSLQN
SEQ ID NO: 72 anti-TNF antibody CDRL2 variant
   AASSLQK
SEQ ID NO: 73 anti-TNF antibody CDRL3 variant
   QRYDRPPYT
SEQ ID NO: 74 anti-TNF antibody CDRL3 variant
   QRYDKPPYT
SEQ ID NO: 75 anti-TNF antibody CDRL3 variant
   QRYNRPPYT
SEQ ID NO: 76 anti-TNF antibody CDRL3 variant
   QRYNKPPYT
SEQ ID NO: 77 Polynucleotide sequence of anti-TNF antibody variable heavy domain variant cb1-3-VH (aka cb2-6-VH) 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGC
SEQ ID NO: 78 Protein sequence of anti-TNF antibody variable heavy domain variant cb1-3-VH (aka cb2-6-VH) 
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVSYLSTASSLDYWGQGTLVTVSS
SEQ ID NO: 79 Polynucleotide sequence of anti-TNF antibody variable heavy domain variant cb2-44-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACCACGCCCTGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGAG
GTACCTGAGCACCGCCAGCAGCCTGGACTACTGGGGCCAGGGCACACTAGTGACCGTGTC
CAGC
SEQ ID NO: 80 Protein sequence of anti-TNF antibody variable heavy domain variant cb2-44-VH 
EVQLVESGGGLVQPGRSLRLSCAASGFTFDDHALHWVRQAPGKGLEWVSAITWNSGHIDYADS
VEGRFTISRDNAKNSLYLQMNSLRAEDTAVYYCAKVRYLSTASSLDYWGQGTLVTVSS
SEQ ID NO: 81 Polynucleotide sequence of anti-TNF antibody variable heavy domain variant cb1-39-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCACTACGCCCTGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGC
SEQ ID NO: 82 Protein sequence of anti-TNF antibody variable heavy domain variant cb1-39-VH 
EVQLVESGGGLVQPGRSLRLSCAASGFTFDHYALHWVRQAPGKGLEWVSAITWNSGHIDYADS
VEGRFTISRDNAKNSLYLQMNSLRAEDTAVYYCAKVSYLSTASSLDYWGQGTLVTVSS
SEQ ID NO: 83 Polynucleotide sequence of anti-TNF antibody variable heavy domain variant cb1-31-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGCAC
TACCTGAGCACCGCCAGCCAACTGCACCACTGGGGCCAGGGCACACTAGTGACCGTGTCC
AGC
SEQ ID NO: 84 Protein sequence of anti-TNF antibody variable heavy domain variant cb1-31-VH 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVHYLSTASQLHHWGQGTLVTVSS
SEQ ID NO: 85 Polynucleotide sequence of anti-TNF antibody variable heavy domain variant cb2-11-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCACTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGCA
GTACCTGAGCACCGCCAGCAGCCTGCAGAGCTGGGGCCAGGGCACACTAGTGACCGTGTC
CAGC
SEQ ID NO: 86 Protein sequence of anti-TNF antibody variable heavy domain variant cb2-11-VH 
EVQLVESGGGLVQPGRSLRLSCAASGFTFDHYAMHWVRQAPGKGLEWVSAITWNSGHIDYAD
SVEGRFTISRDNAKNSLYLQMNSLRAEDTAVYYCAKVQYLSTASSLQSWGQGTLVTVSS
SEQ ID NO: 87 Polynucleotide sequence of anti-TNF antibody variable heavy domain variant cb2-40-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGAAG
TACCTGAGCACCGCCAGCAGCCTGCACTACTGGGGCCAGGGCACACTAGTGACCGTGTCC
AGC
SEQ ID NO: 88 Protein sequence of anti-TNF antibody variable heavy domain variant cb2-40-VH 
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVKYLSTASSLHYWGQGTLVTVSS
SEQ ID NO: 89 Polynucleotide sequence of anti-TNF antibody variable heavy domain variant cb2-35-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGCACGCCCTGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGCAC
TACCTGAGCACCGCCAGCAGCCTGGACTACTGGGGCCAGGGCACACTAGTGACCGTGTCC
AGC
SEQ ID NO: 90 Protein sequence of anti-TNF antibody variable heavy domain variant cb2-35-VH 
EVQLVESGGGLVQPGRSLRLSCAASGFTFDQHALHWVRQAPGKGLEWVSAITWNSGHIDYADS
VEGRFTISRDNAKNSLYLQMNSLRAEDTAVYYCAKVHYLSTASSLDYWGQGTLVTVSS
SEQ ID NO: 91 Polynucleotide sequence of anti-TNF antibody variable heavy domain variant cb2-28-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGCAC
TACCTGAGCACCGCCAGCCAGCTGCACCACTGGGGCCAGGGCACACTAGTGACCGTGTCC
AGC
SEQ ID NO: 92 Protein sequence of anti-TNF antibody variable heavy domain variant cb2-28-VH 
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVHYLSTASQLHHWGQGTLVTVSS
SEQ ID NO: 93 Polynucleotide sequence of anti-TNF antibody variable heavy domain variant cb2-38-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGCACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGC
SEQ ID NO: 94 Protein sequence of anti-TNF antibody variable heavy domain variant cb2-38-VH 
EVQLVESGGGLVQPGRSLRLSCAASGFTFDQHAMHWVRQAPGKGLEWVSAITWNSGHIDYAD
SVEGRFTISRDNAKNSLYLQMNSLRAEDTAVYYCAKVSYLSTASSLDYWGQGTLVTVSS
SEQ ID NO: 95 Polynucleotide sequence of anti-TNF antibody variable heavy domain variant cb2-20-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGAAG
TACCTGAGCACCGCCAGCAACCTGGAGAGCTGGGGCCAGGGCACACTAGTGACCGTGTCC
AGC
SEQ ID NO: 96 Protein sequence of anti-TNF antibody variable heavy domain variant cb2-20-VH 
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVKYLSTASNLESWGQGTLVTVSS
SEQ ID NO: 97 Polynucleotide sequence of anti-TNF antibody variable light domain variant cb1-8-VL 
GATATCCAGATGACCCAGAGCCCCAGCAGCCTGAGCGCCTCTGTGGGCGATAGAGTGACCA
TCACCTGCCACGCCAGCAAGAAGATCAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 98 Protein sequence of anti-TNF antibody variable light domain variant cb1-8-VL 
DIQMTQSPSSLSASVGDRVTITCHASKKIRNYLAWYQQKPGKAPKLLIYAASSLLRGVPSRFSGS
GSGTDFTLTISSLQPEDVATYYCQRYDRPPYTFGQGTKVEIKRT
SEQ ID NO: 99 Polynucleotide sequence of anti-TNF antibody variable light domain variant cb1-43-VL 
GATATCCAGATGACCCAGAGCCCCAGCAGCCTGAGCGCCTCTGTGGGCGATAGAGTGACCA
TCACCTGCCACGCCAGCAGGAAGCTGAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 100 Protein sequence of anti-TNF antibody variable light domain variant cb1-43-VL 
DIQMTQSPSSLSASVGDRVTITCHASRKLRNYLAWYQQKPGKAPKLLIYAASSLLKGVPSRFSGS
GSGTDFTLTISSLQPEDVATYYCQRYDRPPYTFGQGTKVEIKRT
SEQ ID NO: 101 Polynucleotide sequence of anti-TNF antibody variable light domain variant cb1-45-VL 
GATATCCAGATGACCCAGAGCCCCAGCAGCCTGAGCGCCTCTGTGGGCGATAGAGTGACCA
TCACCTGCCACGCCAGCAGGAGGCTGAGAAACTACCTGGCCTGGTATCAGCAGAAGCCTGG
CAAGGCCCCTAAGCTGCTGATCTACGCCGCCAGCAGCCTGCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 102 Protein sequence of anti-TNF antibody variable light domain variant cb1-45-VL 
DIQMTQSPSSLSASVGDRVTITCHASRRLRNYLAWYQQKPGKAPKLLIYAASSLLPGVPSRFSGS
GSGTDFTLTISSLQPEDVATYYCQRYDRPPYTFGQGTKVEIKRT
SEQ ID NO: 103 Polynucleotide sequence of anti-TNF antibody variable light domain variant cb1-4-VL 
GATATCCAGATGACCCAGAGCCCCAGCAGCCTGAGCGCCTCTGTGGGCGATAGAGTGACCA
TCACCTGCCACGCCAGCAGGAAGCTGAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 104 Protein sequence of anti-TNF antibody variable light domain variant cb1-4-VL 
DIQMTQSPSSLSASVGDRVTITCHASRKLRNYLAWYQQKPGKAPKLLIYAASSLLRGVPSRFSGS
GSGTDFTLTISSLQPEDVATYYCQRYDRPPYTFGQGTKVEIKRT
SEQ ID NO: 105 Polynucleotide sequence of anti-TNF antibody variable light domain variant cb1-41-VL 
GATATCCAGATGACCCAGAGCCCCAGCAGCCTGAGCGCCTCTGTGGGCGATAGAGTGACCA
TCACCTGCCACGCCAGCAAGAGGATCAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAAGCCCCCTTACACCTTCGGCCAGGG
CACCAAGGTGGAGATCAAGCGTACG
SEQ ID NO: 106 Protein sequence of anti-TNF antibody variable light domain variant cb1-41-VL 
DIQMTQSPSSLSASVGDRVTITCHASKRIRNYLAWYQQKPGKAPKLLIYAASSLLKGVPSRFSGS
GSGTDFTLTISSLQPEDVATYYCQRYDKPPYTFGQGTKVEIKRT
SEQ ID NO: 107 Polynucleotide sequence of anti-TNF antibody variable light domain variant cb1-37-VL 
GATATCCAGATGACCCAGAGCCCCAGCAGCCTGAGCGCCTCTGTGGGCGATAGAGTGACCA
TCACCTGCCACGCCAGCAGGAAGCTGAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACAACAGACCCCCTTACACCTTCGGCCAGGGC
ACCAAGGTGGAGATCAAGCGTACG
SEQ ID NO: 108 Protein sequence of anti-TNF antibody variable light domain variant cb1-37-VL 
DIQMTQSPSSLSASVGDRVTITCHASRKLRNYLAWYQQKPGKAPKLLIYAASSLLRGVPSRFSGS
GSGTDFTLTISSLQPEDVATYYCQRYNRPPYTFGQGTKVEIKRT
SEQ ID NO: 109 Polynucleotide sequence of anti-TNF antibody variable light domain variant cb1-39-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
CAAGGCCCCTAAGCTGCTGATCTACGCCGCCAGCAGCCTGCAGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 110 Protein sequence of anti-TNF antibody variable light domain variant cb1-39-VL 
DIQMTQSPSSLSASVGDRVTITCHASRKIRNYLAWYQQKPGKAPKLLIYAASSLQPGVPSRFSGS
GSGTDFTLTISSLQPEDVATYYCQRYDRPPYTFGQGTKVEIKRT
SEQ ID NO: 111 Polynucleotide sequence of anti-TNF antibody variable light domain variant cb1-33-VL 
GATATCCAGATGACCCAGAGCCCCAGCAGCCTGAGCGCCTCTGTGGGCGATAGAGTGACCA
TCACCTGCCACGCCAGCAGGAGGATCAGAAACTACCTGGCCTGGTATCAGCAGAAGCCTGG
CAAGGCCCCTAAGCTGCTGATCTACGCCGCCAGCAGCCTGCTGCACGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 112 Protein sequence of anti-TNF antibody variable light domain variant cb1-33-VL 
DIQMTQSPSSLSASVGDRVTITCHASRRIRNYLAWYQQKPGKAPKLLIYAASSLLHGVPSRFSGS
GSGTDFTLTISSLQPEDVATYYCQRYDRPPYTFGQGTKVEIKRT
SEQ ID NO: 113 Polynucleotide sequence of anti-TNF antibody variable light domain variant cb1-35-VL 
GATATCCAGATGACCCAGAGCCCCAGCAGCCTGAGCGCCTCTGTGGGCGATAGAGTGACCA
TCACCTGCCACGCCAGCAGGAGGCTGAGAAACTACCTGGCCTGGTATCAGCAGAAGCCTGG
CAAGGCCCCTAAGCTGCTGATCTACGCCGCCAGCAGCCTGCAGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 114 Protein sequence of anti-TNF antibody variable light domain variant cb1-35-VL 
DIQMTQSPSSLSASVGDRVTITCHASRRLRNYLAWYQQKPGKAPKLLIYAASSLQPGVPSRFSG
SGSGTDFTLTISSLQPEDVATYYCQRYDRPPYTFGQGTKVEIKRT
SEQ ID NO: 115 Polynucleotide sequence of anti-TNF antibody variable light domain variant cb1-31-VL 
GATATCCAGATGACCCAGAGCCCCAGCAGCCTGAGCGCCTCTGTGGGCGATAGAGTGACCA
TCACCTGCCACGCCAGCAGGAGGCTGAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACAACAAGCCCCCTTACACCTTCGGCCAGGGC
ACCAAGGTGGAGATCAAGCGTACG
SEQ ID NO: 116 Protein sequence of anti-TNF antibody variable light domain variant cb1-31-VL 
DIQMTQSPSSLSASVGDRVTITCHASRRLRNYLAWYQQKPGKAPKLLIYAASSLLKGVPSRFSGS
GSGTDFTLTISSLQPEDVATYYCQRYNKPPYTFGQGTKVEIKRT
SEQ ID NO: 117 Polynucleotide sequence of anti-TNF antibody variable light domain variant cb1-29-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
CAAGGCCCCTAAGCTGCTGATCTACGCCGCCAGCAGCTTCCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 118 Protein sequence of anti-TNF antibody variable light domain variant cb1-29-VL 
DIQMTQSPSSLSASVGDRVTITCHASRKIRNYLAWYQQKPGKAPKLLIYAASSFLPGVPSRFSGS
GSGTDFTLTISSLQPEDVATYYCQRYDRPPYTFGQGTKVEIKRT
SEQ ID NO: 119 Polynucleotide sequence of anti-TNF antibody variable light domain variant cb1-22-VL 
GATATCCAGATGACCCAGAGCCCCAGCAGCCTGAGCGCCTCTGTGGGCGATAGAGTGACCA
TCACCTGCCACGCCAGCAAGAAGATCAGAAACTACCTGGCCTGGTATCAGCAGAAGCCTGG
CAAGGCCCCTAAGCTGCTGATCTACGCCGCCAGCAGCCTGCAGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 120 Protein sequence of anti-TNF antibody variable light domain variant cb1-22-VL 
DIQMTQSPSSLSASVGDRVTITCHASKKIRNYLAWYQQKPGKAPKLLIYAASSLQPGVPSRFSGS
GSGTDFTLTISSLQPEDVATYYCQRYDRPPYTFGQGTKVEIKRT
SEQ ID NO: 121 Polynucleotide sequence of anti-TNF antibody variable light domain variant cb1-23-VL 
GATATCCAGATGACCCAGAGCCCCAGCAGCCTGAGCGCCTCTGTGGGCGATAGAGTGACCA
TCACCTGCCACGCCAGCAGGAGGATCAGAAACTACCTGGCCTGGTATCAGCAGAAGCCTGG
CAAGGCCCCTAAGCTGCTGATCTACGCCGCCAGCAGCCTGCTGCAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 122 Protein sequence of anti-TNF antibody variable light domain variant cb1-23-VL 
DIQMTQSPSSLSASVGDRVTITCHASRRIRNYLAWYQQKPGKAPKLLIYAASSLLQGVPSRFSGS
GSGTDFTLTISSLQPEDVATYYCQRYDRPPYTFGQGTKVEIKRT
SEQ ID NO: 123 Polynucleotide sequence of anti-TNF antibody variable light domain variant cb1-12-VL 
GATATCCAGATGACCCAGAGCCCCAGCAGCCTGAGCGCCTCTGTGGGCGATAGAGTGACCA
TCACCTGCCACGCCAGCAGGAAGCTGAGAAACTACCTGGCCTGGTATCAGCAGAAGCCTGG
 
CAAGGCCCCTAAGCTGCTGATCTACGCCGCCAGCAGCCTGCAGCAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 124 Protein sequence of anti-TNF antibody variable light domain variant cb1-12-VL 
DIQMTQSPSSLSASVGDRVTITCHASRKLRNYLAWYQQKPGKAPKLLIYAASSLQQGVPSRFSG
SGSGTDFTLTISSLQPEDVATYYCQRYDRPPYTFGQGTKVEIKRT
SEQ ID NO: 125 Polynucleotide sequence of anti-TNF antibody variable light domain variant cb1-10-VL 
GATATCCAGATGACCCAGAGCCCCAGCAGCCTGAGCGCCTCTGTGGGCGATAGAGTGACCA
TCACCTGCCACGCCAGCAGGAAGCTGAGAAACTACCTGGCCTGGTATCAGCAGAAGCCTGG
CAAGGCCCCTAAGCTGCTGATCTACGCCGCCAGCAGCCTGCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 126 Protein sequence of anti-TNF antibody variable light domain variant cb1-10-VL 
DIQMTQSPSSLSASVGDRVTITCHASRKLRNYLAWYQQKPGKAPKLLIYAASSLLPGVPSRFSGS
GSGTDFTLTISSLQPEDVATYYCQRYDRPPYTFGQGTKVEIKRT
SEQ ID NO: 127 Polynucleotide sequence of anti-TNF antibody variable light domain variant cb2-1-VL 
GATATCCAGATGACCCAGAGCCCCAGCAGCCTGAGCGCCTCTGTGGGCGATAGAGTGACCA
TCACCTGCCACGCCAGCAGGGAGATCAGAAACTACCTGGCCTGGTATCAGCAGAAGCCTGG
CAAGGCCCCTAAGCTGCTGATCTACGCCGCCAGCAGCCTGCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 128 Protein sequence of anti-TNF antibody variable light domain variant cb2-1-VL 
DIQMTQSPSSLSASVGDRVTITCHASREIRNYLAWYQQKPGKAPKLLIYAASSLLPGVPSRFSGS
GSGTDFTLTISSLQPEDVATYYCQRYDRPPYTFGQGTKVEIKRT
SEQ ID NO: 129 Polynucleotide sequence of anti-TNF antibody variable light domain variant cb2-11-VL 
GATATCCAGATGACCCAGAGCCCCAGCAGCCTGAGCGCCTCTGTGGGCGATAGAGTGACCA
TCACCTGCCACGCCAGCCAGGGCATCAGAAACTACCTGGCCTGGTATCAGCAGAAGCCTGG
CAAGGCCCCTAAGCTGCTGATCTACGCCGCCAGCACCCTGCTGAAGGGCGTGCCCAGCAG
 
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 130 Protein sequence of anti-TNF antibody variable light domain variant cb2-11-VL 
DIQMTQSPSSLSASVGDRVTITCHASQGIRNYLAWYQQKPGKAPKLLIYAASTLLKGVPSRFSGS
GSGTDFTLTISSLQPEDVATYYCQRYDRPPYTFGQGTKVEIKRT
SEQ ID NO: 131 Polynucleotide sequence of anti-TNF antibody variable light domain variant cb2-40-VL 
GATATCCAGATGACCCAGAGCCCCAGCAGCCTGAGCGCCTCTGTGGGCGATAGAGTGACCA
TCACCTGCCACGCCAGCCAGAAGATCAGAAACTACCTGGCCTGGTATCAGCAGAAGCCTGG
CAAGGCCCCTAAGCTGCTGATCTACGCCGCCAGCAGCCTGCAGCAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 132 Protein sequence of anti-TNF antibody variable light domain variant cb2-40-VL 
DIQMTQSPSSLSASVGDRVTITCHASQKIRNYLAWYQQKPGKAPKLLIYAASSLQQGVPSRFSGS
GSGTDFTLTISSLQPEDVATYYCQRYDRPPYTFGQGTKVEIKRT
SEQ ID NO: 133 Polynucleotide sequence of anti-TNF antibody variable light domain variant cb2-35-VL 
GATATCCAGATGACCCAGAGCCCCAGCAGCCTGAGCGCCTCTGTGGGCGATAGAGTGACCA
TCACCTGCCACGCCAGCAGGAGGCTGAGAAACTACCTGGCCTGGTATCAGCAGAAGCCTGG
CAAGGCCCCTAAGCTGCTGATCTACGCCGCCAGCAGCCTGCTGCACGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 134 Protein sequence of anti-TNF antibody variable light domain variant cb2-35-VL 
DIQMTQSPSSLSASVGDRVTITCHASRRLRNYLAWYQQKPGKAPKLLIYAASSLLHGVPSRFSGS
GSGTDFTLTISSLQPEDVATYYCQRYDRPPYTFGQGTKVEIKRT
SEQ ID NO: 135 Polynucleotide sequence of anti-TNF antibody variable light domain variant cb2-28-VL 
GATATCCAGATGACCCAGAGCCCCAGCAGCCTGAGCGCCTCTGTGGGCGATAGAGTGACCA
TCACCTGCCACGCCAGCAGGAGGCTGAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
 
GGACGTGGCCACCTACTACTGCCAGCGGTACGACAGACCCCCTTACACCTTCGGCCAGGG
CACCAAGGTGGAGATCAAGCGTACG
SEQ ID NO: 136 Protein sequence of anti-TNF antibody variable light domain variant cb2-28-VL 
DIQMTQSPSSLSASVGDRVTITCHASRRLRNYLAWYQQKPGKAPKLLIYAASSLLKGVPSRFSGS
GSGTDFTLTISSLQPEDVATYYCQRYDRPPYTFGQGTKVEIKRT
SEQ ID NO: 137 Polynucleotide sequence of anti-TNF antibody variable light domain variant cb2-20-VL 
GATATCCAGATGACCCAGAGCCCCAGCAGCCTGAGCGCCTCTGTGGGCGATAGAGTGACCA
TCACCTGCCACGCCAGCAAGAGGATCAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACAACAAGCCCCCTTACACCTTCGGCCAGGGC
ACCAAGGTGGAGATCAAGCGTACG
SEQ ID NO: 138 Protein sequence of anti-TNF antibody variable light domain variant cb2-20-VL 
DIQMTQSPSSLSASVGDRVTITCHASKRIRNYLAWYQQKPGKAPKLLIYAASSLLRGVPSRFSGS
GSGTDFTLTISSLQPEDVATYYCQRYNKPPYTFGQGTKVEIKRT
SEQ ID NO: 139 Polynucleotide sequence of anti-TNF antibody variable light domain variant cb1-3-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAAGCCCCCTTACACCTTCGGCCAGGG
CACCAAGGTGGAGATCAAGCGTACG
SEQ ID NO: 140 Protein sequence of anti-TNF antibody variable light domain variant cb1-3-VL 
DIQMTQSPSSLSASVGDRVTITCHASRKIRNYLAWYQQKPGKAPKLLIYAASSLLRGVPSRFSGS
GSGTDFTLTISSLQPEDVATYYCQRYDKPPYTFGQGTKVEIKRT
SEQ ID NO: 141 Polynucleotide sequence of anti-TNF antibody variable light domain variant cb2-6-VL 
GATATCCAGATGACCCAGAGCCCCAGCAGCCTGAGCGCCTCTGTGGGCGATAGAGTGACCA
TCACCTGCCACGCCAGCAAGAGGATCAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACAACAAGCCCCCTTACACCTTCGGCCAGGGC
ACCAAGGTGGAGATCAAGCGTACG
SEQ ID NO: 142 Protein sequence of anti-TNF antibody variable light domain variant cb2-6-VL 
DIQMTQSPSSLSASVGDRVTITCHASKRIRNYLAWYQQKPGKAPKLLIYAASSLLKGVPSRFSGS
GSGTDFTLTISSLQPEDVATYYCQRYNKPPYTFGQGTKVEIKRT
SEQ ID NO: 143 Polynucleotide sequence of anti-TNF antibody variable light domain variant cb2-44-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
CAAGGCCCCTAAGCTGCTGATCTACGCCGCCAGCAGCCTGCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACG
SEQ ID NO: 144 Protein sequence of anti-TNF antibody variable light domain variant cb2-44-VL 
DIQMTQSPSSLSASVGDRVTITCHASRKIRNYLAWYQQKPGKAPKLLIYAASSLLPGVPSRFSGS
GSGTDFTLTISSLQPEDVATYYCQRYDRPPYTFGQGTKVEIKRT
SEQ ID NO: 145 Protein sequence of anti-TNF antibody heavy chain variant cb1-3-VH plus M252Y/S254T/T256E modification 
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
YITREPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 146 Protein sequence of anti-TNF antibody heavy chain variant cb2-44-VH plus M252Y/S254T/T256E modification 
EVQLVESGGGLVQPGRSLRLSCAASGFTFDDHALHWVRQAPGKGLEWVSAITWNSGHIDYADS
VEGRFTISRDNAKNSLYLQMNSLRAEDTAVYYCAKVRYLSTASSLDYWGQGTLVTVSSASTKGP
SVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTLY
ITREPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 147 Polynucleotide sequence of anti-TNF antibody light chain variant cb1-3-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
CAAGGCCCCTAAGCTGCTGATCTACGCCGCCAGCAGCCTGCTGAGGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAAGCCCCCTTACACCTTCGGCCAGGG
CACCAAGGTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCC
GGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGA
GCAAGGCCGACTACGAGAAGCACAAGGTGTACGCCTGTGAGGTGACCCACCAGGGCCTGT
CCAGCCCCGTGACCAAGAGCTTCAACCGGGGCGAGTGC
SEQ ID NO: 148 Protein sequence of anti-TNF antibody light chain variant cb1-3-VL 
DIQMTQSPSSLSASVGDRVTITCHASRKIRNYLAWYQQKPGKAPKLLIYAASSLLRGVPSRFSGS
GSGTDFTLTISSLQPEDVATYYCQRYDKPPYTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 149 Polynucleotide sequence of anti-TNF antibody light chain variant cb2-6-VL 
GATATCCAGATGACCCAGAGCCCCAGCAGCCTGAGCGCCTCTGTGGGCGATAGAGTGACCA
TCACCTGCCACGCCAGCAAGAGGATCAGAAACTACCTGGCCTGGTATCAGCAGAAGCCTGG
CAAGGCCCCTAAGCTGCTGATCTACGCCGCCAGCAGCCTGCTGAAGGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACAACAAGCCCCCTTACACCTTCGGCCAGGGC
ACCAAGGTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCC
GGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGA
GCAAGGCCGACTACGAGAAGCACAAGGTGTACGCCTGTGAGGTGACCCACCAGGGCCTGT
CCAGCCCCGTGACCAAGAGCTTCAACCGGGGCGAGTGC
SEQ ID NO: 150 Protein sequence of anti-TNF antibody light chain variant cb2-6-VL 
DIQMTQSPSSLSASVGDRVTITCHASKRIRNYLAWYQQKPGKAPKLLIYAASSLLKGVPSRFSGS
GSGTDFTLTISSLQPEDVATYYCQRYNKPPYTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 151 Polynucleotide sequence of anti-TNF antibody light chain variant cb2-44-VL 
GATATCCAGATGACCCAGAGCCCCAGCAGCCTGAGCGCCTCTGTGGGCGATAGAGTGACCA
TCACCTGCCACGCCAGCAGGAAGATCAGAAACTACCTGGCCTGGTATCAGCAGAAGCCTGG
 
CAAGGCCCCTAAGCTGCTGATCTACGCCGCCAGCAGCCTGCTGCCCGGCGTGCCCAGCAG
ATTCAGCGGCAGCGGCTCCGGCACCGACTTCACCCTGACCATCAGCAGCCTGCAGCCCGA
GGACGTGGCCACCTACTACTGCCAGCGGTACGACAGACCCCCTTACACCTTCGGCCAGGG
CACCAAGGTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCC
GGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGA
GCAAGGCCGACTACGAGAAGCACAAGGTGTACGCCTGTGAGGTGACCCACCAGGGCCTGT
CCAGCCCCGTGACCAAGAGCTTCAACCGGGGCGAGTGC
SEQ ID NO: 152 Protein sequence of anti-TNF antibody light chain variant cb2-44-VL 
DIQMTQSPSSLSASVGDRVTITCHASRKIRNYLAWYQQKPGKAPKLLIYAASSLLPGVPSRFSGS
GSGTDFTLTISSLQPEDVATYYCQRYDRPPYTFGQGTKVEIKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 153 Polynucleotide sequence of anti-TNF antibody heavy chain variant cb1-3-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACCAGTACGCCATGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGTCC
TACCTGAGCACCGCCAGCAGCCTGGACTACTGGGGCCAGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAGC
CCAAGAGCTGTGACAAGACCCACACCTGCCCCCCCTGCCCTGCCCCCGAGCTGCTGGGAG
GCCCCAGCGTGTTCCTGTTCCCCCCCAAGCCTAAGGACACCCTGATGATCAGCAGAACCCC
CGAGGTGACCTGTGTGGTGGTGGATGTGAGCCACGAGGACCCTGAGGTGAAGTTCAACTG
GTACGTGGACGGCGTGGAGGTGCACAATGCCAAGACCAAGCCCAGGGAGGAGCAGTACAA
CAGCACCTACCGGGTGGTGTCCGTGCTGACCGTGCTGCACCAGGATTGGCTGAACGGCAA
GGAGTACAAGTGTAAGGTGTCCAACAAGGCCCTGCCTGCCCCTATCGAGAAAACCATCAGC
AAGGCCAAGGGCCAGCCCAGAGAGCCCCAGGTGTACACCCTGCCCCCTAGCAGAGATGAG
CTGACCAAGAACCAGGTGTCCCTGACCTGCCTGGTGAAGGGCTTCTACCCCAGCGACATCG
CCGTGGAGTGGGAGAGCAACGGCCAGCCCGAGAACAACTACAAGACCACCCCCCCTGTGC
TGGACAGCGATGGCAGCTTCTTCCTGTACAGCAAGCTGACCGTGGACAAGAGCAGATGGCA
GCAGGGCAACGTGTTCAGCTGCTCCGTGATGCACGAGGCCCTGCACAATCACTACACCCAG
AAGAGCCTGAGCCTGTCCCCTGGCAAG
 
SEQ ID NO: 154 Protein sequence of anti-TNF antibody heavy chain variant cb1-3-VH
EVQLVESGGGLVQPGRSLRLSCAASGFTFDQYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 155 Polynucleotide sequence of anti-TNF antibody heavy chain variant cb2-44-VH 
GAGGTGCAGCTGGTGGAGTCTGGCGGCGGACTGGTGCAGCCCGGCAGAAGCCTGAGACT
GAGCTGTGCCGCCAGCGGCTTCACCTTCGACGACCACGCCCTGCACTGGGTGAGGCAGGC
CCCTGGCAAGGGCCTGGAGTGGGTGTCCGCCATCACCTGGAATAGCGGCCACATCGACTA
CGCCGACAGCGTGGAGGGCAGATTCACCATCAGCCGGGACAACGCCAAGAACAGCCTGTA
CCTGCAGATGAACAGCCTGAGAGCCGAGGACACCGCCGTGTACTACTGTGCCAAGGTGAG
GTACCTGAGCACCGCCAGCAGCCTGGACTACTGGGGCCAGGGCACACTAGTGACCGTGTC
CAGCGCCAGCACCAAGGGCCCCAGCGTGTTCCCCCTGGCCCCCAGCAGCAAGAGCACCAG
CGGCGGCACAGCCGCCCTGGGCTGCCTGGTGAAGGACTACTTCCCCGAACCGGTGACCGT
GTCCTGGAACAGCGGAGCCCTGACCAGCGGCGTGCACACCTTCCCCGCCGTGCTGCAGAG
CAGCGGCCTGTACAGCCTGAGCAGCGTGGTGACCGTGCCCAGCAGCAGCCTGGGCACCCA
GACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACCC
CCGAGGTGACCTGTGTGGTGGTGGATGTGAGCCACGAGGACCCTGAGGTGAAGTTCAACT
GGTACGTGGACGGCGTGGAGGTGCACAATGCCAAGACCAAGCCCAGGGAGGAGCAGTACA
ACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAG
CAAGGCCAAGGGCCAGCCCAGAGAGCCCCAGGTGTACACCCTGCCCCCTAGCAGAGATGA
GCTGACCAAGAACCAGGTGTCCCTGACCTGCCTGGTGAAGGGCTTCTACCCCAGCGACATC
GCCGTGGAGTGGGAGAGCAACGGCCAGCCCGAGAACAACTACAAGACCACCCCCCCTGTG
CTGGACAGCGATGGCAGCTTCTTCCTGTACAGCAAGCTGACCGTGGACAAGAGCAGATGGC
AGCAGGGCAACGTGTTCAGCTGCTCCGTGATGCACGAGGCCCTGCACAATCACTACACCCA
GAAGAGCCTGAGCCTGTCCCCTGGCAAG
SEQ ID NO: 156 Protein sequence of anti-TNF antibody heavy chain variant cb2-44-VH 
EVQLVESGGGLVQPGRSLRLSCAASGFTFDDHALHWVRQAPGKGLEWVSAITWNSGHIDYADS
VEGRFTISRDNAKNSLYLQMNSLRAEDTAVYYCAKVRYLSTASSLDYWGQGTLVTVSSASTKGP
SVFPLAPSSKSTSGGTAALGCLVKDYFPEPVTVSWNSGALTSGVHTFPAVLQSSGLYSLSSVVT
 
VPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 157 Polynucleotide sequence of pascolizumab heavy chain containing the M252Y/S254T/T256E modifications 
CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGGC
AGCCACCCGGCAAAGGCCTGGAGTGGCTGGCCCACATCTACTGGGACGACGACAAGAGGT
ACAACCCCAGCCTGAAGAGCCGGCTGACCATCAGCAAGGATACCAGCAGGAACCAGGTGG
TGCTGACCATGACCAACATGGACCCCGTGGACACCGCTACCTACTACTGCGCCAGGAGGGA
GACCGTCTTCTACTGGTACTTCGACGTGTGGGGAAGGGGCACACTAGTGACCGTGTCCAGC
GCCAGCACCAAGGGCCCCAGCGTGTTCCCCCTGGCCCCCAGCAGCAAGAGCACCAGCGGC
GGCACAGCCGCCCTGGGCTGCCTGGTGAAGGACTACTTCCCCGAACCGGTGACCGTGTCC
TGGAACAGCGGAGCCCTGACCAGCGGCGTGCACACCTTCCCCGCCGTGCTGCAGAGCAGC
GGCCTGTACAGCCTGAGCAGCGTGGTGACCGTGCCCAGCAGCAGCCTGGGCACCCAGACC
TACATCTGTAACGTGAACCACAAGCCCAGCAACACCAAGGTGGACAAGAAGGTGGAGCCCA
AGAGCTGTGACAAGACCCACACCTGCCCCCCCTGCCCTGCCCCCGAGCTGCTGGGAGGCC
CCAGCGTGTTCCTGTTCCCCCCCAAGCCTAAGGACACCCTGtacATCacCAGAgagCCCGAGG
TGACCTGTGTGGTGGTGGATGTGAGCCACGAGGACCCTGAGGTGAAGTTCAACTGGTACGT
GGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTA
CAAGTGTAAGGTGTCCAACAAGGCCCTGCCTGCCCCTATCGAGAAAACCATCAGCAAGGCC
AAGGGCCAGCCCAGAGAGCCCCAGGTGTACACCCTGCCCCCTAGCAGAGATGAGCTGACC
AAGAACCAGGTGTCCCTGACCTGCCTGGTGAAGGGCTTCTACCCCAGCGACATCGCCGTGG
AGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGG
GCAACGTGTTCAGCTGCTCCGTGATGCACGAGGCCCTGCACAATCACTACACCCAGAAGAG
CCTGAGCCTGTCCCCTGGCAAG
SEQ ID NO: 158 Protein sequence of pascolizumab heavy chain containing the M252Y/S254T/T256E modifications 
QVTLRESGPALVKPTQTLTLTCTFSGFSLSTSGMGVSWIRQPPGKGLEWLAHIYWDDDKRYNPS
LKSRLTISKDTSRNQVVLTMTNMDPVDTATYYCARRETVFYWYFDVWGRGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDTLYI
TREPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVEW
 
ESNGQPENNYKTTPPVLDSDGSFFLYSKLTVDKSRWQQGNVFSCSVMHEALHNHYTQKSLSLS
PGK
SEQ ID NO: 159 Polynucleotide sequence of pascolizumab light chain 
GACATCGTGCTGACCCAGAGCCCCTCTTCCCTGAGCGCAAGCGTGGGCGATAGGGTGACC
ATCACCTGCAAGGCCAGCCAGAGCGTGGACTACGACGGCGACAGCTACATGAACTGGTACC
AGCAGAAGCCCGGCAAGGCCCCCAAACTGCTGATCTACGCCGCCAGCAACCTCGAGTCAG
GCATTCCCAGCAGGTTTAGCGGCAGCGGCAGCGGCACCGACTTCACCTTCACAATCAGCAG
CCTGCAGCCCGAGGACATCGCCACCTACTACTGCCAGCAGAGCAACGAGGACCCTCCCAC
CTTCGGACAGGGCACCAAGGTCGAGATCAAGCGTACGGTGGCCGCCCCCAGCGTGTTCAT
CTTCCCCCCCAGCGATGAGCAGCTGAAGAGCGGCACCGCCAGCGTGGTGTGTCTGCTGAA
CAACTTCTACCCCCGGGAGGCCAAGGTGCAGTGGAAGGTGGACAATGCCCTGCAGAGCGG
CAACAGCCAGGAGAGCGTGACCGAGCAGGACAGCAAGGACTCCACCTACAGCCTGAGCAG
CACCCTGACCCTGAGCAAGGCCGACTACGAGAAGCACAAGGTGTACGCCTGTGAGGTGAC
CCACCAGGGCCTGTCCAGCCCCGTGACCAAGAGCTTCAACCGGGGCGAGTGC
SEQ ID NO: 160 Protein sequence of pascolizumab light chain 
DIVLTQSPSSLSASVGDRVTITCKASQSVDYDGDSYMNWYQQKPGKAPKLLIYAASNLESGIPSR
FSGSGSGTDFTFTISSLQPEDIATYYCQQSNEDPPTFGQGTKVEIKRTVAAPSVFIFPPSDEQLKS
GTASVVCLLNNFYPREAKVQWKVDNALQSGNSQESVTEQDSKDSTYSLSSTLTLSKADYEKHK
VYACEVTHQGLSSPVTKSFNRGEC
SEQ ID NO: 161 Protein sequence of pascolizumab heavy chain 
QVTLRESGPALVKPTQTLTLTCTFSGFSLSTSGMGVSWIRQPPGKGLEWLAHIYWDDDKRYNPS
LKSRLTISKDTSRNQVVLTMTNMDPVDTATYYCARRETVFYWYFDVWGRGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALHNHYTQKSLSLS
PGK
SEQ ID NO: 162 Alternative protein sequence of the anti-TNF antibody heavy chain plus M428L/N434S modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVKGFYPSDIAV
 
EWESNGQPENNYKTTPPVLDSDGSFFLYSKLTVDKSRWQQGNVFSCSVLHEALHSHYTQKSLS
LSPGK
SEQ ID NO: 163 Alternative protein sequence of the IgG1 constant domain plus M428L/N434S modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVLHEALHSHYT
QKSLSLSPGK
SEQ ID NO: 164 Protein sequence of the anti-TNF antibody heavy chain plus H433K/N434F modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALKFHYTQKSLSL
SPGK
SEQ ID NO: 165 Protein sequence of the IgG1 constant domain plus H433K/N434F modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALKFHYTQ
KSLSLSPGK
SEQ ID NO: 166 Alternative protein sequence of the anti-TNF antibody heavy chain plus H433K/N434F modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCLVKGFYPSDIAV
 
EWESNGQPENNYKTTPPVLDSDGSFFLYSKLTVDKSRWQQGNVFSCSVMHEALKFHYTQKSLS
LSPGK
SEQ ID NO: 167 Alternative protein sequence of the IgG1 constant domain plus H433K/N434F modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHEALKFHYT
QKSLSLSPGK
SEQ ID NO: 168 Alternative protein sequence of the anti-TNF antibody heavy chain plus M428L/N434S modification 
EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSASTKG
PSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPPVAGPSVFLFPPKPKDTL
MISRTPEVTCVVVDVSHEDPEVQFNWYVDGVEVHNAKTKPREEQFNSTFRVVSVLTVVHQDWL
NGKEYKCKVSNKGLPAPIEKTISKTKGQPREPQVYTLPPSREEMTKNQVSLTCLVKGFYPSDIAV
EWESNGQPENNYKTTPPMLDSDGSFFLYSKLTVDKSRWQQGNVFSCSVLHEALHSHYTQKSLS
LSPGK
SEQ ID NO: 169 Alternative protein sequence of the IgG1/2 constant domain plus M428L/N434S modification 
ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPPVAGPSVFLFPPKP
KDTLMISRTPEVTCVVVDVSHEDPEVQFNWYVDGVEVHNAKTKPREEQFNSTFRVVSVLTVVHQ
DWLNGKEYKCKVSNKGLPAPIEKTISKTKGQPREPQVYTLPPSREEMTKNQVSLTCLVKGFYPS
DIAVEWESNGQPENNYKTTPPMLDSDGSFFLYSKLTVDKSRWQQGNVFSCSVLHEALHSHYTQ
KSLSLSPGK
SEQ ID NO: 170 Golimumab_VH 
QVQLVESGGGVVQPGRSLRLSCAASGFIFSSYAMHWVRQAPGNGLEWVAFMSYDGSNKKYAD
SVKGRFTISRDNSKNTLYLQMNSLRAEDTAVYYCARDRGIAAGGNYYYYGMDVWGQGTTVTVS
S
SEQ ID NO: 171 Golimumab_VL 
EIVLTQSPATLSLSPGERATLSCRASQSVYSYLAWYQQKPGQAPRLLIYDASNRATGIPARFSGS
GSGTDFTLTISSLEPEDFAVYYCQQRSNWPPFTFGPGTKVDIKRT
SEQ ID NO: 172 Golimumab_HC 
QVQLVESGGGVVQPGRSLRLSCAASGFIFSSYAMHWVRQAPGNGLEWVAFMSYDGSNKKYAD
SVKGRFTISRDNSKNTLYLQMNSLRAEDTAVYYCARDRGIAAGGNYYYYGMDVWGQGTTVTVS
SASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLFPP
KPKDTLYITREPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHY
TQKSLSLSPGK
SEQ ID NO: 173 Golimumab_LC 
EIVLTQSPATLSLSPGERATLSCRASQSVYSYLAWYQQKPGQAPRLLIYDASNRATGIPARFSGS
GSGTDFTLTISSLEPEDFAVYYCQQRSNWPPFTFGPGTKVDIKRTVAAPSVFIFPPSDEQLKSGT
ASVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 174 Remicade_VH 
EVKLEESGGGLVQPGGSMKLSCVASGFIFSNHWMNWVRQSPEKGLEWVAEIRSKSINSATHYA
ESVKGRFTISRDDSKSAVYLQMTDLRTEDTGVYYCSRNYYGSTYDYWGQGTTLTVSS
SEQ ID NO: 175 Remicade_VL 
DILLTQSPAILSVSPGERVSFSCRASQFVGSSIHWYQQRTNGSPRLLIKYASESMSGIPSRFSGS
GSGTDFTLSINTVESEDIADYYCQQSHSWPFTFGSGTNLEVKRT
SEQ ID NO: 176 Remicade_HC 
EVKLEESGGGLVQPGGSMKLSCVASGFIFSNHWMNWVRQSPEKGLEWVAEIRSKSINSATHYA
ESVKGRFTISRDDSKSAVYLQMTDLRTEDTGVYYCSRNYYGSTYDYWGQGTTLTVSSASTKGP
SVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTLY
ITREPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSL
SPGK
SEQ ID NO: 177 Remicade_LC 
DILLTQSPAILSVSPGERVSFSCRASQFVGSSIHWYQQRTNGSPRLLIKYASESMSGIPSRFSGS
GSGTDFTLSINTVESEDIADYYCQQSHSWPFTFGSGTNLEVKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC
SEQ ID NO: 178 Cimzia (certolizumab) VH 
EVQLVESGGGLVQPGGSLRLSCAASGYVFTDYGMNWVRQAPGKGLEWMGWINTYIGEPIYAD
SVKGRFTFSLDTSKSTAYLQMNSLRAEDTAVYYCARGYRSYAMDYWGQGTLVTVSS
SEQ ID NO: 179 Cimzia (certolizumab) VL 
DIQMTQSPSSLSASVGDRVTITCKASQNVGTNVAWYQQKPGKAPKALIYSASFLYSGVPYRFSG
SGSGTDFTLTISSLQPEDFATYYCQQYNIYPLTFGQGTKVEIKRT
SEQ ID NO: 180 Cimzia (certolizumab) HC (VH+CH1)

EVQLVESGGGLVQPGGSLRLSCAASGYVFTDYGMNWVRQAPGKGLEWMGWINTYIGEPIYAD
SVKGRFTFSLDTSKSTAYLQMNSLRAEDTAVYYCARGYRSYAMDYWGQGTLVTVSSASTKGPS
VFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCAA

A. An antigen binding protein which specifically binds to TNF-alpha comprising:
   (i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); or a CDR variant thereof which contains 1, 2, 3 or 4 amino acid substitutions, insertions or deletions as compared to CDRH1, CDRH2, CDRH3, CDRL1, CDRL2, or CDRL3; and
   (ii) a neonatal Fc receptor (FcRn) binding portion of a human IgG1 constant domain comprising one of more amino acid substitutions relative to the human IgG1 constant domain;
   wherein the antigen binding protein has an increased FcRn binding affinity at pH 6 and/ or increased half-life as compared to an IgG comprising the light chain sequence of SEQ ID No. 2 and the heavy chain sequence of SEQ ID No.12.
B. An antigen binding protein which specifically binds to binds to TNF-alpha comprising:
   (i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); or a CDR variant thereof which contains 1, 2, 3 or 4 amino acid substitutions, insertions or deletions as compared to CDRH1, CDRH2, CDRH3, CDRL1, CDRL2, or CDRL3; and
   (ii) a neonatal Fc receptor (FcRn) binding portion of a human IgG1 constant domain comprising one of more amino acid substitutions relative to the human IgG1 constant domain;
      wherein the antigen binding protein has an increased half life as compared to an IgG comprising the light chain sequence of SEQ ID No. 2 and the heavy chain sequence of SEQ ID No.12 and the antigen binding protein can be administered no more than once every four weeks to achieve comparable mean steady-state trough concentration as that achieved by the same dose of the IgG comprising light chain sequence of SEQ ID No. 2 and heavy chain sequence of SEQ ID No. 12 administered once every two weeks.
C. An antigen binding protein which specifically binds to TNF-alpha comprising:
   (i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); or a CDR variant thereof which contains 1, 2, 3 or 4 amino acid substitutions, insertions or deletions as compared to CDRH1, CDRH2, CDRH3, CDRL1, CDRL2, or CDRL3; and
   (ii) a neonatal Fc receptor (FcRn) binding portion of a human IgG1 constant domain comprising one of more amino acid substitutions relative to the human IgG1 constant domain;
   wherein the antigen binding protein has an affinity for FcRn of 4 fold greater than an anti-TNF antigen binding protein with the same CDR's without such modifications at pH 6 as assessed by ProteOn XPR36 protein interaction array system at 25°C, the array system having antigen binding proteins immobilised on the chip.
D. An antigen binding protein which is a variant of an IgG comprising the light chain sequence of SEQ ID No. 2 and the heavy chain sequence of SEQ ID No.12, wherein the antigen binding protein variant comprises one or more substitutions in the neonatal Fc receptor (FcRn) binding portion of the IgG constant domain to increase the half-life of the antigen binding protein variant compared with the IgG, wherein when the variant is administered to patients at a single dose of 40 mg at a four to eight weekly interval, the mean steady-state trough antibody concentration in the patient population does not fall below 5 µg/ml, preferably below 6 µg/ml, between dosing intervals.
E. An antigen binding protein as claimed in any of paragraphs A-E comprising amino acid substitutions relative to the human IgG1constant domain at one or more of positions 250, 252, 254, 256, 257, 259, 308, 428 or 434 numbered according to EU index of Kabat.
F. An antigen binding protein as claimed in any of paragraphs A-E wherein the human IgG1 constant domain has the sequence of SEQ ID No. 13 before amino acid substitutions are introduced.
G. An antigen binding protein as claimed in any one of paragraphs A to F for use as a medicament for treating disease wherein the antigen binding protein can be administered to patients no more than once every four weeks to achieve comparable mean steady-state trough concentration as that achieved by the same dose of an IgG comprising the light chain sequence of SEQ ID No. 2 and the heavy chain sequence of SEQ ID No.12 administered once every two weeks.
H. An antigen binding protein as claimed in claim in any of paragraphs A to G for treatment of a disease wherein the antigen binding protein is administered to patients at a single dose between about 35 to about 45 mg at a four to eight weekly interval.
I. An antigen binding protein as described in paragraph H wherein the antigen binding protein is administered to patients subcutaneously as a single 40 mg dose no more than once every four weeks.
J. An antigen binding protein as described in paragraphs H or I wherein the antigen binding protein is administered to patients subcutaneously as a single 40 mg dose no more than once every eight weeks.
K. An antigen binding protein as as described in paragraphs A to J wherein the half-life of the antigen binding protein is increased 2 fold, 3 fold, 4 fold or 5 fold as compared to the native IgG.
L. An antigen binding protein as as described in any of paragraphs A to K wherein administration of the antigen binding protein no more than once every four weeks in patients achieves the mean steady-state trough concentration in the patient population of between about 4 µg/ml to about 7 µg/ml.
M. An antigen binding protein as described in paragraph L wherein the mean steady-state trough concentration is between about 5 µg/ml to about 6 µg/ml.
N. An antigen binding protein as described in any one of paragraphs A to M wherein the clearance of the antigen binding protein is about 2.ml/ hr to about 4ml/hr
O. An antigen binding protein as described in paragraphs A to N wherein the one or more amino acid substitution is at amino acid residues 252, 254 and 256 numbered according to EU index of Kabat and the substitution at residue 252 is a substitution with tyr, phe, trp or thr; the substitution at residue 254 is a substitution with thr; and the substitution at residue 256 is a substitution with ser, arg, glu, asp or thr.
P. An antigen binding protein as described in paragraph O wherein the substitution at residue 252 is a substitution of met with tyr; the substitution at residue 254 is a substitution of ser with thr and the substitution at residue 256 is a substitution of thr with glu.
Q. An antigen binding protein as described in any of paragraphs A to P comprising a constant domain as shown in SEQ ID No: 7
R. An antigen binding protein as described in paragraph A to N wherein the one or more amino acid substitution is at amino acid residues 250 and 428 numbered according to EU index of Kabat and the substitution at residue 250 is a substitution with glu or gin; the substitution at residue 428 is a substitution with leu or phe.
S. An antigen binding protein as described in paragraph R wherein the substitution at residue 250 is a substitution of thr with glu and the substitution at residue 428 is a substitution of met with leu.
T. An antigen binding protein as described in paragraph S comprising a constant domain as shown in SEQ ID No: 16
U. An antigen binding protein as described in any of paragraphs A to N wherein the one or more amino acid substitution is at amino acid residues 428 and 434 numbered according to EU index of Kabat and the aa substitution at residue 428 is a substitution of met with leu and the aa substitution at residue 434 is a substitution of asn with ser.
V. An antigen binding protein as described in paragraph U comprising a constant domain as shown in SEQ ID No: 10
W. An antigen binding protein as described in any of paragraphs A to V wherein the antigen binding protein is an antibody.
X. An antigen binding protein as described in any of paragraphs A to W wherein the antigen binding protein is to be administered with methotrexate, preferably wherein the antigen binding protein is administered for the treatment of rheumatoid arthritis.
Y. An antigen binding protein as described in any of paragraphs A to X comprising a variable domain of SEQ ID NO: 6 and/or SEQ ID NO: 3 or a variant thereof which contains 1, 2, 3, 4, 5, 6, 7, 8, 9 or 10 amino acid substitutions, insertions or deletions or shares at least 90% identity across the length of SEQ ID NO: 6 or SEQ ID NO: 3.
Z. An antigen binding protein as described in any one of paragraphs A to X comprising the heavy chain sequence as as shown in SEQ ID No 5, 9 or 15 optionally with a light chain sequence as shown in SEQ ID No: 2.
AA. An antigen binding protein as described in any of paragraphs A to X comprising a variable heavy domain sequence as shown in SEQ ID NO: 78 or 80.
AB. An antigen binding protein as described in any of paragraphs A to X comprising a heavy chain sequence as shown in SEQ ID NO: 145 or SEQ ID NO: 146.
AC. A method of treating a patient with a disease, the method comprising administering an antigen binding protein as described in any of paragraphs A to AB.
AD. A method of treating a patient with a disease, the method comprising administering an antigen binding protein as described in any of paragraphs A to AB to the patient subcutaneously as a single of dose about 35 to about 45 mg at a four to eight weekly interval.
AE. An antigen binding protein as described in any of paragraphs G to J or a method as as described in any one of paragraphs AC to AD wherein the disease is rheumatoid arthritis, polyarticular juvenile idiopathic arthritis, psoriatic arthritis, ankylosing spondylitis, Crohn's disease or Psoriasis.
AF. Use of an antigen binding protein as described in any one paragraphs A to AB for use in the manufacture of a medicament for the treatment of rheumatoid arthritis, polyarticular juvenile idiopathic arthritis, psoriatic arthritis, ankylosing spondylitis, Crohn's disease or Psoriasis.
AG. A nucleic acid comprising a nucleotide sequences encoding the antigen binding protein as described in any one of paragraphs A to AB.
AH. A host cell comprising the nucleic acid as described in paragraph AG.
AI. A kit comprising an antigen binding protein as described in any of paragraphs A to AB, and optionally comprising methotrexate for concomitant delivery of the antigen binding protein and methotrexate.
AJ. A liquid formulation comprising a TNF-alpha antigen binding protein and an acetate buffer. AK. The formulation of paragraph AJ, wherein the formulation does not comprise a polyol.
AL. The formulation as described in any one of paragraphs AJ to AK, wherein the buffer further comprises one or more, a combination, or all of: a surfactant; a chelator; a salt; and an amino acid.
AM. The formulation as described in any one of paragraphs AJ to AL, wherein the TNF-alpha antigen binding protein is at a concentration of 20 to 300 mg/mL.
AN. The formulation as described in any one of paragraphs AJ to AM, wherein the formulation comprises:
   (a) 10 to 100 mM sodium acetate trihydrate; and/or
   (b) 25 to 100 mM sodium chloride; and/or
   (c) 0.5 to 5% arginine free base; and/or
   (d) 0.02 to 0.2 mM EDTA; and/or
   (e) 0.01 to 0.1 % polysorbate 80,
   and is adjusted to pH 5.0 to 7.0.
AO. The formulation as described in any one of paragraphs AJ to AN, wherein:
   (a) sodium acetate trihydrate is at a concentration of about 50mM; and/or
   (b) sodium chloride is at a concentration of about 51 mM; and/or
   (c) arginine free base is at a concentration of about 1%; and/or
   (d) EDTA is at a concentration of about 0.05 mM; and/or
   (e) polysorbate 80 is at a concentration of about 0.02%.
AP. The formulation as described in any one of paragraphs AJ to AO, wherein the formulation is pH adjusted to about pH 5.5.
AQ. The formulation as described in any one of paragraphs AJ to AP, wherein the TNF-alpha antigen binding protein is at a concentration of 50 mg/mL.
AR. The formulation as described in any one of paragraphs AJ to AQ wherein the formulation has a monomer content of at least 95%, or at least 96%, or at least 97%, or at least 98%, or at least 99% at room temperature (about 25°C) after about 1 week.

## Claims

1. A liquid formulation comprising a TNF-alpha antigen binding protein and an acetate buffer wherein the TNF-alpha antigen binding protein comprises the light chain sequence of SEQ ID No. 2 and the heavy chain sequence of SEQ ID No.12.

2. The formulation of claim 1, wherein the formulation does not comprise a polyol.

3. The formulation of any one of claims 1 or 2, wherein the buffer further comprises one or more, a combination, or all of: a surfactant; a chelator; a salt; and an amino acid.

4. The formulation of any one of claims 1 to 3, wherein the TNF-alpha antigen binding protein is at a concentration of 20 to 300 mg/mL.

5. The formulation of any one of claims 1 to 4, wherein the formulation comprises:
(a) 10 to 100 mM sodium acetate trihydrate; and/or
(b) 25 to 100 mM sodium chloride; and/or
(c) 0.5 to 5% arginine free base; and/or
(d) 0.02 to 0.2 mM EDTA; and/or
(e) 0.01 to 0.1 % polysorbate 80,
and is adjusted to pH 5.0 to 7.0.

6. The formulation of claim 5, wherein:
(a) sodium acetate trihydrate is at a concentration of about 50mM; and/or
(b) sodium chloride is at a concentration of about 51 mM; and/or
(c) arginine free base is at a concentration of about 1%; and/or
(d) EDTA is at a concentration of about 0.05 mM; and/or
(e) polysorbate 80 is at a concentration of about 0.02%.

7. The formulation of any one of claims 1 to 6, wherein the formulation is pH adjusted to about pH 5.5.

8. The formulation of any one of claims 1 to 7, wherein the TNF-alpha antigen binding protein is at a concentration of 50 mg/mL.

9. The formulation of any one of claims 1 to 8, wherein the formulation has a monomer content of at least 95%, or at least 96%, or at least 97%, or at least 98%, or at least 99% at room temperature (about 25°C) after about 1 week.
